# EUROPEAN PATENT SPECIFICATION

(11) **EP 3 344 780 B1**
(45) Date of publication and mention of the grant of the patent: **23.06.2021**
(21) Application number: 16766420.0
(22) Date of filing: 30.08.2016
(51) Int. Cl.: C12Q 1/6886

(54) **SPLICE VARIANTS ASSOCIATED WITH NEOMORPHIC SF3B1 MUTANTS**
SPLEISSVARIANTEN IN ZUSAMMENHANG MIT NEOMORPHEN SF3B1-MUTANTEN
VARIANTS D'ÉPISSAGE ASSOCIÉS À DES MUTANTS DE SF3B1 NÉOMORPHES

(30) Priority: 01.09.2015 US 201562212876 P
(43) Date of publication of application: 11.07.2018
(60) Divisional application: 21174658.1
(73) Proprietor: Eisai R&D Management Co., Ltd., Tokyo 112-8088 (JP)
(72) Inventor: YU, Lihua, Acton, MA 01720 (US); LIM, Kian, Huat, Burlington, Massachusetts 01803 (US); FEALA, Jacob, D., Franklin, MA 02038 (US); BUONAMICI, Silvia, Boston, MA 02118 (US); MIZUI, Yoshiharu, Tokyo 112-8088 (JP); SMITH, Peter, G., Arlington, MA 02474 (US); ZHU, Ping, Boxborough, MA 01719 (US); PARK, Eunice, Sun, Arlington, MA 02474 (US); SEILER, Michael, W., Belmont, MA 02478 (US); FEKKES, Marco, Peter, Waltham, MA 02451 (US)
(74) Representative: Finnegan Europe LLP
(86) International application number: PCT/US2016/049490
(87) International publication number: WO 2017/040526

(56) References cited:
- WO-A1-2013/070521
- WO-A1-2014/165753
- WO-A2-01/75067
- YOUZHONG WAN ET AL: "SF3B1 Mutation Alters The Selection Of 3' RNA Splice Sites In Chronic Lymphocytic Leukemia", BLOOD, vol. 122, no. 21, 5 December 2013 (2013-12-05), page 117, XP055315235,
- S. MAGUIRE ET AL: "575 SF3B1 mutations are associated with alternative splicing in ER-positive breast cancer", EUROPEAN JOURNAL OF CANCER, vol. 50, 1 November 2014 (2014-11-01), page 186, XP055315153, AMSTERDAM, NL ISSN: 0959-8049, DOI: 10.1016/S0959-8049(14)70701-4
- LILI WANG ET AL: "SF3B1 and Other Novel Cancer Genes in Chronic Lymphocytic Leukemia", NEW ENGLAND JOURNAL OF MEDICINE, vol. 365, no. 26, 29 December 2011 (2011-12-29), pages 2497-2506, XP055065481, ISSN: 0028-4793, DOI: 10.1056/NEJMoa1109016
- S. J. FURNEY ET AL: "SF3B1 Mutations Are Associated with Alternative Splicing in Uveal Melanoma", CANCER DISCOVERY, vol. 3, no. 10, 1 October 2013 (2013-10-01), pages 1122-1129, XP055315109, US ISSN: 2159-8274, DOI: 10.1158/2159-8290.CD-13-0330
- D GENTIEN ET AL: "A common alternative splicing signature is associated with SF3B1 mutations in malignancies from different cell lineages", LEUKEMIA., vol. 28, no. 6, 1 June 2014 (2014-06-01), pages 1355-1357, XP055315159, US ISSN: 0887-6924, DOI: 10.1038/leu.2014.28
- YOUZHONG WAN ET AL: "Review Article SF3B1 mutations in chronic lymphocytic leukemia", BLOOD, vol. 121, no. 23, 8 April 2013 (2013-04-08), pages 4627-4634, XP055233923, DOI: 10.1182/blood-2013-02-
- P. CONVERTINI ET AL: "Sudemycin E influences alternative splicing and changes chromatin modifications", NUCLEIC ACIDS RESEARCH, vol. 42, no. 8, 1 April 2014 (2014-04-01), pages 4947-4961, XP055315163, GB ISSN: 0305-1048, DOI: 10.1093/nar/gku151
- DATABASE EMBL [Online] 23 January 2009 (2009-01-23), "Homo sapiens cDNA FLJ59337 complete cds, highly similar to DCC-interacting protein 13 beta.", XP002767728, retrieved from EBI accession no. EM_STD:AK297100 Database accession no. AK297100
- DATABASE EMBL [Online] 21 April 2007 (2007-04-21), "Homo sapiens cDNA, clone LYMPB2001721, 5' end, mRNA sequence.", XP002767729, retrieved from EBI accession no. EM_EST:DC428363 Database accession no. DC428363
- DATABASE Geneseq [Online] 13 February 2002 (2002-02-13), "DNA encoding novel human diagnostic protein #543.", XP002767730, retrieved from EBI accession no. GSN:AAS64739 Database accession no. AAS64739
- DATABASE EMBL [Online] 21 October 2005 (2005-10-21), "Homo sapiens cDNA clone SKMUS2007303, 5' end, mRNA sequence.", XP002767731, retrieved from EBI accession no. EM_EST:DA898882 Database accession no. DA898882
- KIMURA KOUICHI ET AL: "Diversification of transcriptional modulation: large-scale identification and characterization of putative alternative promoters of human genes", GENOME RESEARCH, COLD SPRING HARBOR LABORATORY PRESS, US, vol. 16, no. 1, 1 January 2006 (2006-01-01), pages 55-65, XP002446089, ISSN: 1088-9051, DOI: 10.1101/GR.4039406

## Description

The instant application contains a Sequence Listing which has been submitted electronically in ASCII format. The ASCII copy, created on May 16, 2014, is named 12636.6-304_SL.txt and is 183 kilobytes in size.

RNA splicing, a highly regulated molecular event orchestrated by the spliceosome, results in the removal of intronic sequences from pre-mRNA to generate mature mRNA. Dysregulation of RNA splicing has been identified as a causative defect in several diseases. In addition, dysregulated splicing has been proposed to play an important role in tumorigenesis and resistance to therapy; however, the molecular causes of dysregulated splicing in cancer have remained elusive.

SF3B1 is a protein involved in RNA splicing. It forms part of the U2 snRNP complex which binds to the pre-mRNA at a region containing the branchpoint site and is involved in early recognition and stabilization of the spliceosome at the 3' splice site (3'ss). A thorough and systematic analysis of the effects of SF3B1 mutations is needed to define their effects on RNA splicing in cells and may lead to novel therapeutic approaches for SF3B1 mutant cancers.

Youzhong Wan et al. discloses that the SF3B1 mutation alters the selection of 3' RNA splice sites in chronic lymphocytic leukemia (see Blood, 2013, 122(21); 117)

The description provided herein demonstrates that certain SF3B1 mutations result in neomorphic activity with the production of known and novel splicing alterations. In addition, lineage-specific splicing aberrations were identified in chronic lymphocytic leukemia (CLL), melanoma, and breast cancer. Furthermore, treatment of SF3B1-mutant cancer cell lines, xenografts, and CLL patient samples with modulators of SF3B1 reduced aberrant splicing and induced tumor regression.

### SUMMARY

The methods described herein involve detecting or quantifying the expression of one or more splice variants in a cell containing a neomorphic mutant SF3B1 protein. Various embodiments of the invention include detecting or quantifying splice variants to determine whether a patient has a cancer with one or more neomorphic SF3B1 mutations. Additional embodiments include measuring the amount of a splice variant to evaluate the effects of a compound on a mutant SF3B1 protein. Further embodiments include SF3B1-modulating compounds for use in treating a patient who has cancer cells with a neomorphic mutant SF3B1 protein. These embodiments are described in more detail in the claims.

Various embodiments encompass a method of detecting one or more splice variants selected from Table 1 in a biological sample, comprising:
a) providing a biological sample suspected of containing one or more splice variants;
b) contacting the biological sample with one or more nucleic acid probes capable of specifically hybridizing to the one or more splice variants, and
c) detecting the binding of the one or more probes to the one or more splice variants.

In some embodiments, the one or more nucleic acid probes capable of specifically hybridizing to the one or more splice variants each comprise a label. In some embodiments, the method of detecting one or more splice variants selected from Table 1 in a biological sample further comprises contacting the biological sample with one or more additional nucleic acid probes, wherein the additional probes are each labeled with a molecular barcode.

Also disclosed are methods of modulating the activity of a neomorphic mutant SF3B1 protein in a target cell, comprising applying an SF3B1-modulating compound to the target cell, wherein the target cell has been determined to express one or more aberrant splice variants selected from Table 1 at a level that is increased or decreased relative to the level in a cell not having the neomorphic mutant SF3B1 protein.

Also disclosed are methods for evaluating the ability of a compound to modulate the activity of a neomorphic mutant SF3B1 protein in a target cell, comprising the steps of:
a) providing a target cell having a mutant SF3B1 protein;
b) applying the compound to the target cell; and
c) measuring the expression level of one or more splice variants selected from Table 1.

Also disclosed are methods for evaluating the ability of a compound to modulate the activity of a neomorphic mutant SF3B1 protein in a target cell further comprises the step of measuring the expression level of one or more splice variants selected from Table 1 before step (b).

In some embodiments, the neomorphic mutant SF3B1 protein is selected from K700E, K666N, R625C, G742D, R625H, E622D, H662Q, K666T, K666E, K666R, G740E, Y623C, T663I, K741N, N626Y, T663P, H662R, G740V, D781E, or R625L. In some embodiments, the neomorphic mutant SF3B1 protein is selected from E622D, E622K, E622Q, E622V, Y623C, Y623H, Y623S, R625C, R625G, R625H, R625L, R625P, R625S, N626D, N626H, N626I, N626S, N626Y, H662D, H662L, H662Q, H662R, H662Y, T663I, T663P, K666E, K666M, K666N, K666Q, K666R, K666S, K666T, K700E, V701A, V701F, V701I, I704F, I704N, I704S, I704V, G740E, G740K, G740R, G740V, K741N, K741Q, K741T, G742D, D781E, D781G, or D781N.

In some embodiments, the step of measuring the expression level of one or more splice variants comprises using an assay to quantify nucleic acid selected from nucleic acid barcoding (e.g. NanoString®), RT-PCR, microarray, nucleic acid sequencing, nanoparticle probes (e.g. SmartFlare™), and in situ hybridization (e.g. RNAscope®).

In some embodiments, the step of measuring the expression level of one or more splice variants comprises measuring the number of copies of the one or more splice variant RNAs in the target cell.

In further embodiments, the compound is selected from a small molecule, an antibody, an antisense molecule, an aptamer, an RNA molecule, and a peptide. In further embodiments, the small molecule is selected from pladienolide and a pladienolide analog. In additional embodiments, the pladienolide analog is selected from pladienolide B, pladienolide D, E7107, a compound of formula 1: 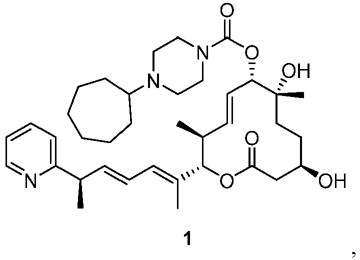 a compound of formula 2: 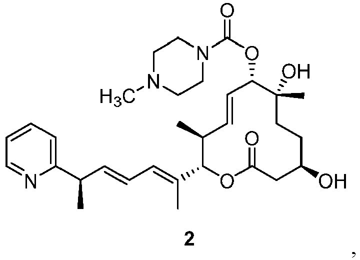 a compound of formula 3: 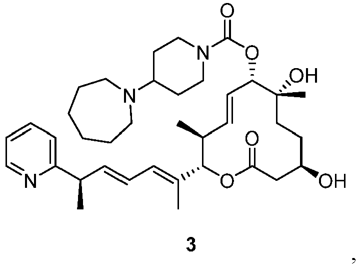 or a compound of formula 4:

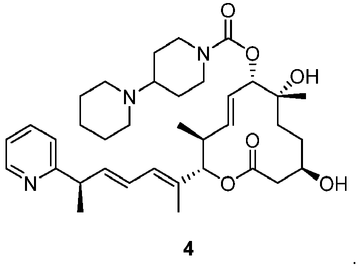

The target cell may be obtained from a patient suspected of having myelodysplastic syndrome, chronic lymphocytic leukemia, chronic myelomonocytic leukemia, or acute myeloid leukemia. In some embodiments, the target cell is obtained from a sample selected from blood or a blood fraction or is a cultured cell derived from a cell obtained from a sample chosen from blood or a blood fraction. In some embodiments, the target cell is a lymphocyte.

In further embodiments, the target cell is obtained from a solid tumor. In some embodiments, the target cell is a breast tissue cell, pancreatic cell, lung cell, or skin cell.

Embodiments further encompass an SF3B1-modulating compound for use in a method for treating a patient with a neoplastic disorder, the method comprising administering a therapeutically effective amount of an SF3B1-modulating compound to the patient, wherein a cell from the patient has been determined to:
a) contain a neomorphic mutant SF3B1 protein; and
b) express one or more aberrant splice variants selected from Table 1 at a level that is increased or decreased relative to the level in a cell not having the neomorphic mutant SF3B1 protein.

Additional embodiments are set forth in the description which follows.

It is to be understood that both the foregoing general description and the following detailed description are exemplary and explanatory only and are not restrictive of the invention, as claimed.

### BRIEF DESCRIPTION OF THE DRAWINGS

Fig. 1 is a schematic diagram depicting modes of alternative splicing.
Fig. 2 is a graph depicting levels of gene expression for abnormally spliced genes across different cancers in patient samples.
Fig. 3 is a schematic diagram showing the locations of certain neomorphic mutations in the SF3B1 protein and corresponding coding regions of the SF3B1 gene.
Fig. 4 is a graph depicting levels of aberrant splice variants detected in RNA isolated from pancreatic, lung cancer, and Nalm-6 isogenic cell lines using a NanoString® assay. Data are represented as the mean of three replicates.
Fig. 5 is a set of western blot images that confirm overexpression of SF3B1 proteins in 293FT cells.
Fig. 6 is a graph depicting levels of aberrant splice variants in RNA isolated from 293FT cells expressing wild type SF3B1 (SF3B1^{WT}) or mutant SF3B1 proteins, as measured in a NanoString® assay. Data are represented as the mean of three replicates.
Fig. 7 is a set of western blot images that confirm overexpression of SF3B1 proteins in 293FT cells.
Fig. 8 is a graph depicting levels of aberrant splice variants in RNA isolated from 293FT cells expressing SF3B1^{WT} or mutant SF3B1 proteins, as measured in a NanoString® assay.
Fig. 9A depicts a set of western blot images showing expression of SF3B1 alleles before and after shRNA-knockdown in Panc 05.04 cells. Fig. 9B depicts a graph showing levels of SF3B1 RNA detected by qPCR in Panc 05.04 cells before and after shRNA-knockdown of all SF3B1 alleles ("SF3B1^{PAN}) or SF3B1^{WT} or mutant SF3B1 (SF3B1^{MUT}) alleles. qPCR data are represented as fold change relative to pLKO non-treated with doxycycline (mean ± SD, n=3). Solid black, outlined, and gray bars indicate SF3B1^{PAN}, SF3B1^{WT}, and SF3B1^{MUT} allele-specific qPCR data, respectively.
Fig. 10A depicts a set of western blot images showing expression of SF3B1 alleles before and after shRNA-knockdown in Panc 10.05 cells. Fig. 10B depicts a graph showing levels of SF3B1 RNA detected by qPCR in Panc 10.05 cells before and after shRNA- knockdown of SF3B1 alleles. qPCR data are represented as fold change relative to pLKO non-treated with doxycycline (mean ± SD, n=3). Solid black, outlined, and gray bars indicate SF3B1^{PAN}, SF3B1^{WT}, and SF3B1^{MUT} allele-specific qPCR data, respectively.
Figs. 11A and 11B are a set of graphs depicting levels of splice variants in Panc 05.04 (Fig. 11A) and Panc 10.05 cells (Fig. 11B) before and after shRNA-knockdown of SF3B1 alleles, as measured in a NanoString® assay. Data are represented as mean of three biological replicates.
Fig. 12 is a set of graphs depicting growth curves of Panc 05.04 cells before (circles) and after (squares) shRNA-knockdown of SF3B1 alleles.
Fig. 13 is a set of graphs depicting growth curves of Panc 10.05 cells before (circles) and after (squares) shRNA-knockdown of SF3B1 alleles.
Figs. 14A and 14B are a set of images of culture plates showing colony formation of Panc 05.04 cells (Fig. 14A) and Panc 10.05 (Fig. 14B) cells before and after shRNA-knockdown of SF3B1 alleles.
Figs 15A and 15B are a set of graphs showing the level of splicing of pre-mRNA Ad2 substrate in nuclear extracts from (Fig. 15A) 293F cells expressing Flag-tag SF3B1^{WT} or SF3B1^{K700E} (left and right panels [circles and triangles], respectively) and (Fig. 15B) Nalm-6 (SF3B1^{WT}) and Nalm-6 SF3B1^{K700E} cells (left and right panels [circles and triangles], respectively) treated with varying concentrations of E7101. Data are represented as mean ± SD, n=2.

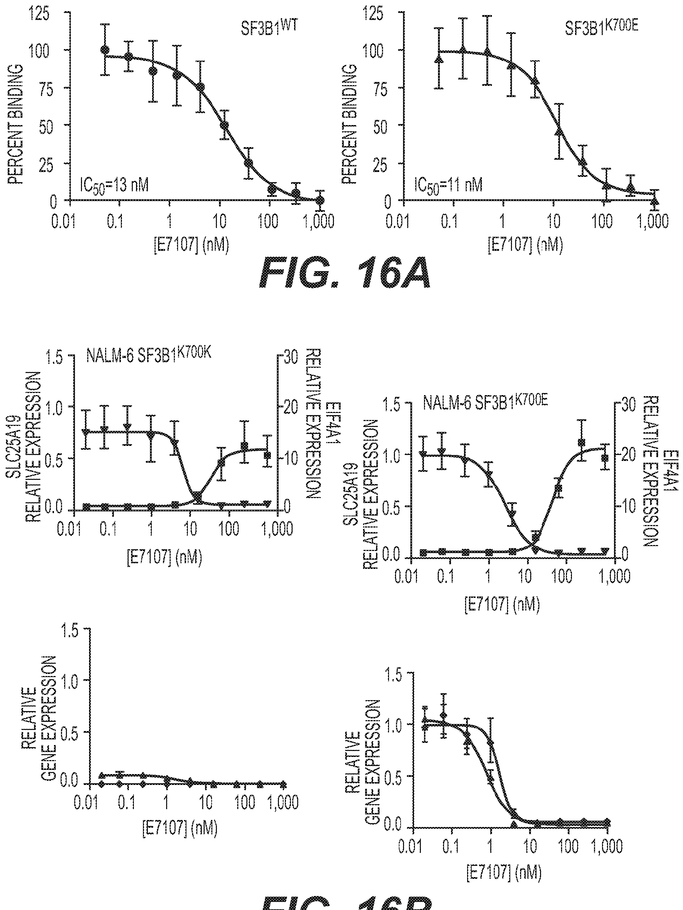

Figs. 16A depicts a pair of graphs showing the binding of a radiolabeled E7107 analog to either SF3B1^{WT} (circles, left panel) or SF3B1^{K700E} (triangles, right panel) after incubation of the proteins with varying concentrations of E7107. Fig. 16B depicts upper panels, a pair of graphs showing the levels of EIF4A1 pre-mRNA (squares) and SLC25A19 mature RNA (inverted triangles) in Nalm-6 SF3B1^{K700K} cells (left panel) and Nalm-6 SF3B1^{K700E} cells (right panel) treated with varying concentrations of E7107, as measured by qPCR. Fig. 16B depicts lower panels, a pair of graphs showing the levels of abnormally spliced isoforms of abnormally spliced genes COASY (triangles) and ZDHHC16 (diamonds) in Nalm-6 SF3B1^{K700K} cells (left panel) and Nalm-6 SF3B1^{K700E} cells (right panel) treated with varying concentrations of E7107, as measured by qPCR. qPCR data in (Fig. 16B) are represented as mean ± SD (n=3).
Fig. 17 is a set of graphs depicting levels of splice variants in Nalm-6 SF3B1^{K700K} and Nalm-6 SF3B1^{K700E} cells after treatment of cells with E7107 for two or six hours, as measured in a NanoString® assay. Data are expressed as fold change from DMSO-only treatment
Fig. 18 is a set of graphs depicting levels of splice variants in Nalm-6 SF3B1^{K700K} and Nalm-6 SF3B1^{K700E} cells after treatment of cells with E7107 for six hours, as measured by RNA-Seq analysis.
Fig. 19 is a set of graphs depicting levels of splice variants in Nalm-6 SF3B1^{K700K} and Nalm-6 SF3B1^{K700E} cells after treatment of cells with the numbered compounds indicated above the graphs, as measured by RNA-Seq analysis.

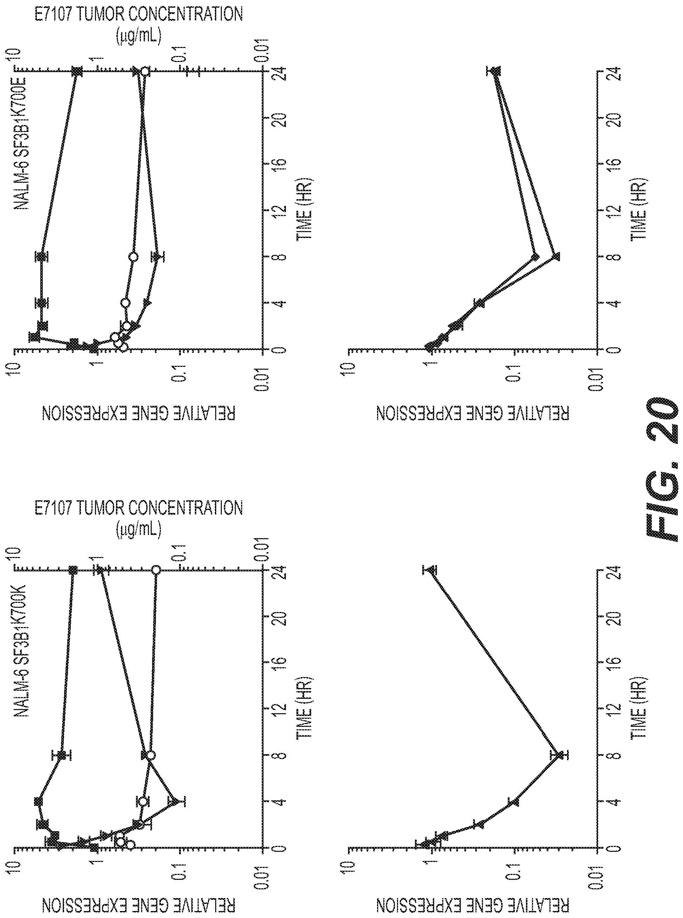

Fig. 20 is set of graphs depicting levels of splice variants in Nalm-6 SF3B1^{K700K} and Nalm-6 SF3B1^{K700E} cells at varying times following treatment of cells with E7107, as measured by qPCR of RNA. Data are represented as mean ± SD (n=3). The upper panels of Fig. 20 depict the levels of EIF4A1 pre-mRNA (squares) and SLC25A19 mature RNA (inverted triangles) in Nalm-6 SF3B1^{K700K} cells (left panel) and Nalm-6 SF3B1^{K700E} cells (right panel) detected at certain times after treatment with E7107. The lower panels of Fig. 20 depict the levels of abnormally spliced isoforms of abnormally spliced genes COASY (triangles) and ZDHHC16 (diamonds) in Nalm-6 SF3B1^{K700K} cells (left panel) and Nalm-6 SF3B1^{K700E} cells (right panel) detected at certain times after treatment with E7107. Open circles show the concentration of E7107 (in µg/ml [right vertical axis]) as determined by mass spectrometry of tumor samples.
Fig. 21 is a set of graphs depicting levels of canonical and aberrant splice variants in Nalm-6 SF3B1^{K700K}- and Nalm-6 SF3B1^{K700E}-xenograft tumors (left and right sets of panels, respectively) at certain timepoints after treatment of xenograft mice with E7107, as measured in a NanoString® assay. Data are represented as mean of three replicates.
Fig. 22 is a set of graphs depicting levels of canonical and aberrant splice variants in Panc 05.04-xenograft tumors at certain timepoints after treatment of xenograft mice with E7107 at various concentrations, as measured in a NanoString® assay (n=4 mice for each group).
Fig. 23 is a graph depicting tumor volume (shown as mean ± SEM) in Nalm-6 SF3B1^{K700E}-xenograft mice following treatment with E7107, with control mice treated with vehicle shown by open circles (n=10 animals for each group). For E7107-treated animals, inverted triangles = 1.25 mg/kg, triangles = 2.5 mg/kg, and squares = 5 mg/kg.
Fig. 24 is a graph depicting survival rates in 10-animal cohorts of Nalm-6 SF3B1^{K700E}-xenograft mice following treatment with E7107, with an untreated cohort shown by the solid black line. For E7107-treated animals, dashed line = 1.25 mg/kg, gray line = 2.5 mg/kg, and dotted line = 5 mg/kg.
Fig. 25 is set of graphs depicting levels of splice variants in SF3B1^{WT} and neomorphic SF3B1 mutant CLL cell samples following treatment with 10nM E7107 for 6 hours, as measured by analysis. Data are represented as mean values (n=3).

### DESCRIPTION

In certain aspects, the methods of the invention provide assays for measuring the amount of a splice variant in a cell, thereby determining whether a patient has a cancer with a neomorphic SF3B1 mutation. In some embodiments, at least one of the measured splice variants is an aberrant splice variant associated with a neomorphic mutation in an SF3B1 protein. In additional aspects, the measurement of a splice variant in a cell may be used to evaluate the ability of a compound to modulate a mutant neomorphic SF3B1 protein in a cell.

To assist in understanding the present invention, certain terms are first defined. Additional definitions are provided throughout the application.

As used herein, the term "mutant SF3B1 protein" includes SF3B1 proteins that differ in amino acid sequence from the human wild type SF3B1 protein set forth in SEQ ID NO:1200 (GenBank Accession Number NP_036565, Version NP_036565.2) (S. Bonnal, L. Vigevani, and J. Valcárcel, "The spliceosome as a target of novel antitumour drugs," Nat. Rev. Drug Discov. 11:847-59 [2012]). Certain mutant SF3B1 proteins are "neomorphic" mutants, which refers to mutant SF3B1 proteins that are associated with differential expression of aberrant splice variants. In certain embodiments, neomorphic SF3B1 mutants include K700E, K666N, R625C, G742D, R625H, E622D, H662Q, K666T, K666E, K666R, G740E, Y623C, T663I, K741N, N626Y, T663P, H662R, G740V, D781E, or R625L. In other embodiments, neomophic SF3B1 mutants include E622D, E622K, E622Q, E622V, Y623C, Y623H, Y623S, R625C, R625G, R625H, R625L, R625P, R625S, N626D, N626H, N626I, N626S, N626Y, H662D, H662L, H662Q, H662R, H662Y, T663I, T663P, K666E, K666M, K666N, K666Q, K666R, K666S, K666T, K700E, V701A, V701F, V701I, I704F, I704N, I704S, I704V, G740E, G740K, G740R, G740V, K741N, K741Q, K741T, G742D, D781E, D781G, or D781N. Certain SF3B1 mutations are not associated with expression of aberrant splice variants, including K700R.

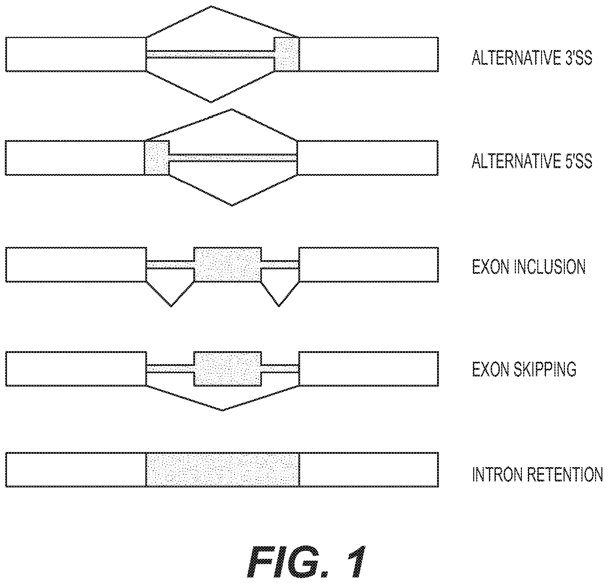

The term "splice variant" as used herein includes nucleic acid sequences that span a junction either between two exon sequences or across an intron-exon boundary in a gene, where the junction can be alternatively spliced. Alternative splicing includes alternate 3' splice site selection ("3'ss"), alternate 5' splice site selection ("5'ss"), differential exon inclusion, exon skipping, and intron retention (Fig. 1). Certain splice variants associated with a given genomic location may be referred to as wild type, or "canonical," variants. These splice variants are most abundantly expressed in cells that do not contain a neomorphic SF3B1 mutant protein. Additional splice variants may be referred to as "aberrant" splice variants, which differ from the canonical splice variant and are primarily associated with the presence of a neomorphic SF3B1 mutant protein in a cell. Aberrant splice variants may alternatively be referred to as "abnormal" or "noncanonical" splice variants. In certain circumstances, cells with a wild type or non-neomorphic SF3B1 protein have low or undetected amounts of an aberrant splice variant, while cells with a neomorphic SF3B1 protein have levels of an aberrant splice variant that are elevated relative to the low or undetected levels in the wild type SF3B1 cells. In some cases, an aberrant splice variant is a splice variant that is present in a wild type SF3B1 cell but is differentially expressed in a cell that has a neomorphic SF3B1 mutant, whereby the latter cell has a level of the aberrant splice variant that is elevated or reduced relative to the level in the wild type SF3B1 cell. Different types of cells containing a neomorphic SF3B1 mutant, such as different types of cancer cells, may have differing levels of expression of certain aberrant splice variants. In addition, certain aberrant splice variants present in one type of cell containing a neomorphic SF3B1 mutant may not be present in other types of cells containing a neomorphic SF3B1 mutant. In some cases, patients with a neomorphic SF3B1 mutant protein may not express an aberrant splice variant or may express an aberrant splice variant at lower levels, due to low allelic frequency of the neomorphic SF3B1 allele. The identity and relative expression levels of aberrant splice variants associated with various types of cells containing neomorphic SF3B1 mutants, such as certain cancer cells, will be apparent from the description and examples provided herein.

The term "evaluating" includes determining the ability of a compound to treat a disease associated with a neomorphic SF3B1 mutation. In some instances, "evaluating" includes determining whether or to what degree a compound modulates aberrant splicing events associated with a neomorphic SF3B1 protein. Modulation of the activity of an SF3B1 protein may encompass up-regulation or down-regulation of aberrant splice variant expression associated with a neomorphic SF3B1 protein. Additionally, "evaluating" includes distinguishing patients that may be successfully treated with a compound that modulates the expression of splice variants associated with a neomorphic SF3B1 protein.

The use of the word "a", "an" or "the" when used in conjunction with the term "comprising" in the claims or the specification may mean "one," but it is also consistent with the meaning of "one or more," "at least one," and "one or more than one." "Or" is to be read inclusively to mean "and/or" unless explicitly indicated to refer to alternatives only, such as where alternatives are mutually exclusive.

### Splice Variants

Splice variants of the invention are listed in Table 1. Table 1 provides the genomic location of each canonical ("WT") and aberrant ("Ab.") splice junction, as well as the sequence. Each sequence listed in the table contains 20 nucleotides from each of the 3' and 5' sides of a splice junction (i.e., the splice junction is at the midpoint of the listed nucleotide sequence). The "Avg WT %" and "Avg Ab. %" columns provide the average percentage count that the canonical (WT) or aberrant splice variant, respectively, represented out of the total counts of all splice variants that utilize a shared splice site, where the counts were determined as set forth in Example 1. The "Log₂ Fold Change" column provides the log₂ of the fold change observed between percentage counts of canonical and aberrant cohorts (see Example 1). The "FDR Q-Value" column provides, as a measure of statistical significance, q-values calculated using the Benjamini-Hochberg procedure from p-values, which in turn were determined using the *moderated t-test* defined in the Bioconductor's *limma* package (available at http://www.bioconductor.org) (see Example 1). The "Event" column indicates the nature of the aberrant splice variant, where "3'ss" indicates alternate 3' splice site selection, "5'ss" indicates alternate 5' splice site selection, "exon incl." indicates differential exon inclusion, and "exon skip" indicates exon skipping. The "Type" column refers to the cancer type of the sample in which the aberrant splice variant was identified, where "Br." indicates breast cancer, "CLL" indicates chronic lymphocytic leukemia, and "Mel." indicates melanoma.

**Table 1.**

| | Aberrant junction | WT junction | Aberrant sequence (SEQ ID NO) | WT sequence (SEQ ID NO) | Avg WT % | Avg Ab. % | Log₂ Fold Diff. | FDR Q-Value | Event | Type |
|---|---|---|---|---|---|---|---|---|---|---|
| 21 | chr12: 105601825-105601935 | chr12: 105601807-105601935 | | | 0 | 23 | 4.58 | 8.14E-08 | 3'ss | Br. |
| 31 | chrl4: 74358911-74360478 | chr14: 74358911-74360499 | | | 0 | 18 | 4.25 | 1.65E-10 | 3'ss | Br. |
| 51 | chrl5: 59209219-59224554 | chr15: 59209198-59224554 | | | 1 | 24 | 3.64 | 4.30E-09 | 3'ss | Br. |
| 81 | chr19: 9728842-9730107 | chr19: 9728855-9730107 | | | 3 | 31 | 3.00 | 7.42E-06 | 3'ss | Br. |
| 118 | chrl5: 25213229-25219533 | chr15: 25213229-25219457 | | | 1 | 10 | 2.46 | 1.54E-06 | 3'ss | Br. |
| 279 | chrl4: 74358911-74360478 | chr14: 74358911-74360499 | | | 0 | 26 | 4.75 | 9.14E-07 | 3'ss | CLL |
| 372 | chr12: 105601825-105601935 | chr12: 105601807-105601935 | | | 3 | 34 | 3.13 | 1.20E-04 | 3'ss | CLL |
| 401 | chrl5: 25213229-25219533 | chr15: 25213229-25219457 | | | 2 | 19 | 2.74 | 3.63E-05 | 3'ss | CLL |
| 426 | chrl5: 59209219-59224554 | chr15: 59209198-59224554 | | | 3 | 21 | 2.46 | 9.18E-03 | 3'ss | CLL |
| 443 | chr19: 9728842-9730107 | chr19: 9728855-9730107 | | | 11 | 53 | 2.17 | 2.14E-08 | 3'ss | CLL |
| 528 | chrl4: 74358911-74360478 | chr14: 74358911-74360499 | | | 0 | 41 | 5.39 | 4.30E-08 | 3'ss | Mel. |
| 543 | chr19: 9728842-9730107 | chr19: 9728855-9730107 | | | 1 | 54 | 4.78 | 1.18E-09 | 3'ss | Mel. |
| 545 | chr12: 105601825-105601935 | chr12: 105601807-105601935 | | | 1 | 49 | 4.64 | 6.42E-06 | 3'ss | Mel. |
| 548 | chrl5: 59209219-59224554 | chr15: 59209198-59224554 | | | 0 | 23 | 4.58 | 2.75E-04 | 3'ss | Mel. |
| 566 | chrl5: 25213229-25219533 | chr15: 25213229-25219457 | | | 1 | 34 | 4.13 | 1.03E-06 | 3'ss | Mel. |

Certain splice variants are associated with more than one disease, and thus appear in Table 1 more than one time. In certain instances, splice variants associated with more than one cancer type may have different expression levels in each disease, so there may be more than one set of expression data for a given splice variant. Variants differentially expressed across all tested cancer types can be used to evaluate cells having SF3B1 neomorphic mutations in additional cancer types. Such variants are shown in the following rows of Table 1 (triplicates represent the same splice junction, measured in different cancer types): [21, 372, 545], [31, 279, 528], [81, 543, 443], [51, 426, 548] and [118, 401, 566]. In certain embodiments, variants that are nonspecific to a particular cancer type can be chosen from the following rows of Table 1: [21, 372, 545], [31, 279, 528], [81, 543, 443], or [51, 426, 548].

Certain embodiments of the invention provide splice variants as markers for cancer. In certain circumstances, cancer cells with a neomorphic SF3B1 protein demonstrate differential expression of certain splice variants compared to cells without a neomorphic SF3B1 protein. The differential expression of one or more splice variants therefore may be used to determine whether a patient has cancer with a neomorphic SF3B1 mutation. In certain embodiments, the patient is also determined to have a cancer cell having a mutant SF3B1 protein. In these methods, one or more of the splice variants listed in Table 1 can be measured to determine whether a patient has cancer with a neomorphic SF3B1 mutation. In certain embodiments, one or more aberrant splice variants from Table 1 are measured. In other embodiments, one or more canonical splice variants are measured. Sometimes, both aberrant and canonical variants are measured.

In some embodiments, one or more aberrant splice variants selected from rows 279, 372 or 443 of Table 1 can be measured in a patient suspected of having CLL. In additional embodiments, a patient suspected of having CLL can be identified by measuring the amounts of one or more of the following aberrant splice variants listed in Table 1: row 443. In additional embodiments, a patient suspected of having CLL can be identified by measuring the amounts of one or more of the following aberrant splice variants listed in Table 1: row 443. In still further embodiments, a patient suspected of having CLL can be identified by measuring the amount of one or more of the following aberrant splice variants listed in Table 1: row 433.

In other embodiments, one or more aberrant splice variants selected from rows 21, 31 or 81 of Table 1 can be measured in a patient suspected of having breast cancer.

In further embodiments, one or more aberrant splice variants selected from rows 528, 543 or 545 of Table 1 can be measured in a patient suspected of having melanoma.

The one or more of the aberrant variants are selected from rows 21, 31, 51, 81, 118, 279, 372, 401, 426, 443, 528, 543, 545, 548 or 566 of Table 1. A patient suspected of having cancer can be identified by measuring the amount of one or more of the aberrant variants selected from 21, 31, 51, 81, 118, 279, 372, 401, 426, 443, 528, 543, 545, 548 or 566. In various embodiments the cancer may be CLL, breast cancer, or melanoma, for example.

Additional methods include predicting or monitoring the efficacy of a treatment for cancer by measuring the level of one or more aberrant splice variants in samples obtained from patients before or during the treatment. For example, a decrease in the levels of one or more aberrant splice variants over the course of treatment may indicate that the treatment is effective. In other cases, the absence of a decrease or an increase in the levels of one or more aberrant splice variants over the course of treatment may indicate that the treatment is not effective and should be adjusted, supplemented, or terminated. In some embodiments, the splice variants are used to track and adjust individual patient treatment effectiveness.

Also disclosed are methods of stratifying cancer patients into different categories based on the presence or absence of one or more particular splice variants in patient samples or the detection of one or more particular splice variants at levels that are elevated or reduced relative to those in normal cell samples. Categories may be different prognostic categories, categories of patients with varying rates of recurrence, categories of patients that respond to treatment and those that do not, and categories of patients that may have particular negative side effects, and the like. According to the categories in which individual patients fall, optimal treatments may then be selected for those patients, or particular patients may be selected for clinical trials.

Also disclosed are methods of distinguishing cancerous cells with SF3B1 neomorphic mutations from normal cells by using the splice variants disclosed herein as markers. Such methods may be employed, for example, to assess the growth or loss of cancerous cells and to identify cancerous cells to be treated or removed. In some embodiments, the splice variants are measured in cancerous tissue having cells with a neomorphic SF3B1 mutation before and after anti-cancer treatment, for the purpose of monitoring the effect of the treatment on cancer progression.

Administering an SF3B1 modulator to a cell, such as a cancer cell, can alter the differential expression of splice variants. Accordingly, the change in expression of one or more splice variants can be used to evaluate the effect of the SF3B1 modulator on the SF3B1 protein. For instance, the effect of an SF3B1 modulator on a CLL cell is evaluated by applying an SF3B1 modulator to such a cell, then detecting or quantifying one or more of the splice variants in Table 1. The one or more splice variants are chosen from rows 279, 372 or 443 of Table 1. The one or more splice variants are chosen from row 443 of Table 1.

The effect of an SF3B1 modulator on a breast cancer cell is evaluated by applying an SF3B1 modulator to such a cell, then detecting or quantifying one or more of the splice variants in Table 1. In further embodiments, the one or more splice variants are chosen from rows 21, 31 or 81 of Table 1.

The the effect of an SF3B1 modulator on a melanoma cell is evaluated by applying an SF3B1 modulator to such a cell, then detecting or quantifying one or more of the splice variants in Table 1. In further embodiments, the one or more splice variants are chosen from rows 528, 543 or 545 of Table 1.

The the effect of an SF3B1 modulator on a cancer cell is evaluated by applying an SF3B1 modulator to such a cell, then detecting or quantifying one or more of the aberrant variants selected from rows 21, 31, 51, 81, 118, 279, 372, 401, 426, 443, 528, 543, 545, 548 or 566 of Table 1. In various embodiments, the cancer cell may be a CLL cell, a breast cancer cell, or a melanoma cell, for example.

The specific splice variants that are useful for demonstrating the effect of an SF3B1 modulator on one type of cancer cell may not be useful for demonstrating an effect of the modulator on another type of cancer cell. Aberrant splice variants that are appropriate for revealing such effects in particular cancer cells will be apparent from the description and examples provided herein.

In some embodiments, aberrant splice variants that are present at elevated levels in a cell having a neomorphic SF3B1 protein are used as markers. In other embodiments, splice variants that have reduced levels in a cell having a neomorphic SF3B1 protein are used as markers. In some embodiments, more than one splice variant will be measured. When more than one splice variant is used, they may all have elevated levels, all have reduced levels, or a mixture of splice variants with elevated and reduced levels may be used. In certain embodiments of the methods described herein, more than one aberrant splice variant is measured. In other embodiments, at least one aberrant and at least one canonical splice variant is measured. In some cases, both an aberrant and canonical splice variant associated with a particular genomic location will be measured. In other circumstances, a measured canonical splice variant will be at a different genomic location from the measured aberrant splice variant(s).

Before performing an assay for splice variants in a cell, one may determine whether the cell has a mutant SF3B1 protein. In certain embodiments, the assay for splice variants is performed if the cell has been determined to have a neomorphic SF3B1 mutant protein.

### Samples

Cell samples can be obtained from a variety of biological sources. Exemplary cell samples include but are not limited to a cell culture, a cell line, a tissue, oral tissue, gastrointestinal tissue, an organ, an organelle, a biological fluid, a blood sample, a urine sample, a skin sample, and the like. Blood samples may be whole blood, partially purified blood, or a fraction of whole or partially purified blood, such as peripheral blood mononucleated cells (PBMCs). The source of a cell sample may be a solid tissue sample such as a tissue biopsy. Tissue biopsy samples may be biopsies from breast tissue, skin, lung, or lymph nodes. Samples may be samples of bone marrow, including bone marrow aspirates and bone marrow biopsies.

In certain embodiments, the cells are human cells. Cells may be cancer cells, for example hematological cancer cells or solid tumor cells. Hematological cancers include chronic lymphocytic leukemia, acute lymphoblastic leukemia, acute myeloid leukemia, chronic myeloid leukemia, chronic myelomonocytic leukemia, acute monocytic leukemia, Hodgkin's lymphoma, Non-Hodgkin's lymphoma, and multiple myeloma. Solid tumors include carcinomas, such as adenocarcinomas, and may be selected from breast, lung, liver, prostate, pancreatic, colon, colorectal, skin, ovarian, uterine, cervical, or renal cancers. Cell samples may be obtained directly from a patient or derived from cells obtained from a patient, such as cultured cells derived from a biological fluid or tissue sample. Samples may be archived samples, such as kryopreserved samples, of cells obtained directly from a subject or of cells derived from cells obtained from a patient.

In certain embodiments, cells are obtained from patients suspected of having cancer. The patients may show signs and symptoms of cancer, such as one or more common symptoms of CLL, which include enlarged lymph nodes, liver, or spleen, higher-than-normal white blood cell counts, recurring infections, loss of appetite or early satiety, abnormal bruising, fatigue, and night sweats. In additional embodiments, the cells have a mutant SF3B1 protein.

Cell samples described herein may be used in any of the methods presently disclosed.

### Detection of splice variants

Certain embodiments of the methods described herein involve detection or quantification of splice variants. A variety of methods exists for detecting and quantifying nucleic acids, and each may be adapted for detection of splice variants in the described embodiments. Exemplary methods include an assay to quantify nucleic acid such as nucleic acid barcoding, nanoparticle probes, in situ hybridization, microarray, nucleic acid sequencing, and PCR-based methods, including real-time PCR (RT-PCR).

Nucleic acid assays utilizing barcoding technology such as NanoString® assays (NanoString Technologies) may be performed, for example, as described in U.S. Patent No. 8,519,115; U.S. Patent No. 7,919,237; and in Kulkarni, M. M., 2011, "Digital Multiplexed Gene Expression Analysis Using the NanoString nCounter System." Current Protocols in Molecular Biology, 94:25B.10.1-25B.10.17. In an exemplary assay, a pair of probes is used to detect a particular nucleotide sequence of interest, such as a particular splice variant of interest. The probe pair consists of a capture probe and a reporter probe and that each include a sequence of from about 35 to 50 bases in length that is specific for a target sequence. The capture probe includes an affinity label such as biotin at its 3' end that provides a molecular handle for surface-attachment of target mRNAs for digital detection, and the reporter probe includes a unique color code at its 5' end that provides molecular barcoding of the hybridized mRNA target sequence. Capture and reporter probe pairs are hybridized to target mRNA in solution, and after excess probes are removed, the target mRNA-probe complexes are immobilized in an nCounter® cartridge. A digital analyzer acquires direct images of the surface of the cartridge to detect color codes corresponding to specific mRNA splice variant sequences. The number of times a color-coded barcode for a particular splice variant is detected reflects the levels of a particular splice variant in the mRNA library. For the detection of splice variants, either the capture or the reporter probe may span a given splice variant's exon-exon or intron-exon junction. In other embodiments, one or both of the capture and reporter probes' target sequences correspond to the terminal sequences of two exons at an exon-exon junction or to the terminal sequences of an intron and an exon at an intron-exon junction, whereby one probe extends to the exon-exon or intron-exon junction, but does not span the junction, and the other probe binds a sequence that begins on opposite side of the junction and extends into the respective exon or intron.

In exemplary PCR-based methods, a particular splice variant may be detected by specifically amplifying a sequence that contains the splice variant. For example, the method may employ a first primer specifically designed to hybridize to a first portion of the splice variant, where the splice variant is a sequence that spans an exon-exon or intron-exon junction at which alternative splicing occurs. The method may further employ a second opposing primer that hybridizes to a segment of the PCR extension product of the first primer that corresponds to another sequence in the gene, such as a sequence at an upstream or downstream location. The PCR detection method may be quantitative (or real-time) PCR. In some embodiments of quantitative PCR, an amplified PCR product is detected using a nucleic acid probe, wherein the probe may contain one or more detectable labels. In certain quantitative PCR methods, the amount of a splice variant of interest is determined by detecting and comparing levels of the splice variant to an appropriate internal control.

Exemplary methods for detecting splice variants using an in situ hybridization assay such as RNAscope® (Advanced Cell Diagnostics) include those described by Wang, F., et al., "RNAscope: a novel in situ RNA analysis platform for formalin-fixed, paraffin-embedded tissues," J. Mol. Diagn. 2012 Jan;14(1):22-9. RNAscope® assays may be used to detect splice variants by designing a pair of probes that targets a given splice variant, and are hybridized to target RNA in fixed and permeabilized cells. Target probes are designed to hybridize as pairs which, when hybridized to the target sequence, create a binding site for a preamplifier nucleic acid. The preamplifier nucleic acid, in turn, harbors multiple binding sites for amplifier nucleic acids, which in turn contain multiple binding sites for a labeled probe carrying a chromogenic or fluorescent molecule. In some embodiments, one of the RNAscope® target probes spans a given splice variant's exon-exon or intron-exon junction. In other embodiments, the target probes' target sequences correspond to the terminal sequences of two exons at an exon-exon junction or to the terminal sequences of an intron and an exon at an intron-exon junction, whereby one probe in the target probe pair extends to the exon-exon or intron-exon junction, but does not span the junction, and the other probe binds a sequence beginning on opposite side of the junction and extending into the respective exon or intron.

Exemplary methods for detecting splice variants using nanoparticle probes such as SmartFlare™ (Millipore) include those described in Seferos et al., "Nano-flares: Probes for Transfection and mRNA Detection in Living Cells," J. Am. Chem. Soc. 129(50):15477-15479 (2007) and Prigodich, A.E., et al., "Multiplexed Nanoflares: mRNA Detection in Live Cells," Anal. Chem. 84(4):2062-2066 (2012). SmartFlare™ detection probes may be used to detect splice variants by generating gold nanoparticles that are modified with one or more nucleic acids that include nucleotide recognition sequences that (1) are each complementary to a particular splice variant to be detected and (2) are each hybridized to a complementary fluorophore-labeled reporter nucleic acid. Upon uptake of the probe by a cell, a target splice variant sequence may hybridize to the one or more nucleotide recognition sequences and displace the fluorophore-labeled reporter nucleic acid. The fluorophore-labeled reporter nucleic acid, whose fluorophore had been quenched due to proximity to the gold nanoparticle surface, is then liberated from the gold nanoparticle, and the fluorophore may then be detected when free of the quenching effect of the nanoparticle. In some embodiments, nucleotide recognition sequences in the probes recognize a sequence that spans a given splice variant's exon-exon or intron-exon junction. In some embodiments, nucleotide recognition sequences in the probes recognize a sequence that is only on one side of the splice variant's exon-exon or intron-exon junction, including a sequence that terminates at the junction and a sequence that terminates one or more nucleotides away from the junction.

Exemplary methods for detecting splice variants using nucleic acid sequencing include RNA sequencing (RNA-Seq) described in Ren, S. et al. "RNA-Seq analysis of prostate cancer in the Chinese population identifies recurrent gene fusions, cancer-associated long noncoding RNAs and aberrant alternative splicings." Cell Res 22, 806-821, doi:10.1038/cr.2012.30 (2012); and van Dijk et al., "Ten years of next-generation sequencing technology." Trends Genet 30(9):418-26 (2014). In some embodiments, high-throughput sequencing, such as next-generation sequencing (NGS) technologies, may be used to dected splice variants. For example, the method may employ commercial sequencing platforms available for RNA-Seq, such as, e.g., Illumina, SOLID, Ion Torrent, and Roche 454. In some embodiments, the sequencing method may include pyrosequencing. For example, a sample may be mixed with sequencing enzymes and primer and exposed to a flow of one unlabeled nucleotide at a time, allowing synthesis of the complementary DNA strand. When a nucleotide is incorporated, pyrophosphate is released leading to light emission, which is monitored in real time. In some embodiments, the sequencing method may include semiconductor sequencing. For example, proton instead of pyrophosphate may be released during nucleotide incorporation and detected in real time by ion sensors. In some embodiments, the method may include sequencing with reversible terminators. For example, the synthesis reagents may include primers, DNA polymerase, and four differently labelled, reversible terminator nucleotides. After incorporation of a nucleotide, which is identified by its color, the 3' terminator on the base and the fluorophore are removed, and the cycle is repeated. In some embodiments, the method may include sequencing by ligation. For example, a sequencing primer may be hybridized to an adapter, with the 5' end of the primer available for ligation to an oligonucleotide hybridizing to the adjacent sequence. A mixture of octamers, in which bases 4 and 5 are encoded by one of four color labels, may compete for ligation to the primer. After color detection, the ligated octamer may be cleaved between position 5 and 6 to remove the label, and the cycle may be repeated. Thereby, in the first round, the process may determine possible identities of bases in positions 4, 5, 9, 10, 14, 15, etc. The process may be repeated, offset by one base using a shorter sequencing primer, to determine positions 3, 4, 8, 9, 13, 14, etc., until the first base in the sequencing primer is reached.

Other nucleic acid detection and analytical methods that also distinguish between splice variants of a given exon-exon or intron-exon junction in a gene by identifying the nucleotide sequence on both sides of the junction may be utilized to detect or quantify the splice variants disclosed herein. For example, splice variants of an exon-exon junction may be detected by primer extension methods in which a primer that binds to one exon is extended into the exon on the other side of the junction according to the sequence of that adjacent exon. See, for example, McCullough, R.M., et al., "High-throughput alternative splicing quantification by primer extension and matrix-assisted laser desorption/ionization time-of-flight mass spectrometry," Nucleic Acids Research, 2005 Jun 20;33(11):e99; and Milani, L., et al., " Detection of alternatively spliced transcripts in leukemia cell lines by minisequencing on microarrays," Clin. Chem. 52: 202-211 (2006). Detection of variants on a large scale may be performed using expression microarrays that carry exon-exon or intron-exon junction probes, as described, for example, in Johnson, J.M. et al., "Genome-wide survey of human alternative pre-mRNA splicing with exon junction microarrays," Science 302: 2141-2144 (2003); and Modrek, B., et al., "Genome-wide detection of alternative splicing in expressed sequences of human genes," Nucleic Acids Res 29: 2850-2859 (2001).

Also disclosed are reagents for detecting splice variants of the invention. In one example, reagents include NanoString® probes designed to measure the amount of one or more of the aberrant splice variants listed in Table 1. Probes for nucleic acid quantification assays such as barcoding (e.g. NanoString®), nanoparticle probes (e.g. SmartFlare™), in situ hybridization (e.g. RNAscope®), microarray, nucleic acid sequencing, and PCR-based assays may be designed as set forth above.

In these exemplary methods or in other methods for nucleic acid detection, aberrant splice variants may be identified using probes, primers, or other reagents which specifically recognize the nucleic acid sequence that is present in the aberrant splice variant but absent in the canonical splice variant. In other embodiments, the aberrant splice variant is identified by detecting the sequence that is specific to the aberrant splice variant in the context of the junction in which it occurs, i.e., the unique sequence is flanked by the sequences which are present on either side of the splice junction in the canonical splice variant. In such cases, the portion of the probe, primer, or other detection reagent that specifically recognizes its target sequence may have a length that corresponds to the length of the aberrant sequence or to or a portion of the aberrant sequence. In other embodiments, the portion of the probe, primer, or other detection reagent that specifically recognizes its target sequence may have a length that corresponds to the length of the aberrant sequence plus the length of a chosen number of nucleotides from one or both of the sequences which flank the aberrant sequence at the splice junction. Generally, the probe or primer should be designed with a sufficient length to reduce non-specific binding. Probes, primers, and other reagents that detect aberrant or canonical splice variants may be designed according to the technical features and formats of a variety of methods for detection of nucleic acids.

### SF3B1 modulators

A variety of SF3B1 modulating compounds are known in the art, and can be used in accordance with the methods described herein. In some embodiments, the SF3B1 modulating compound is a pladienolide or pladienolide analog. A "pladienolid analog" refers to a compound which is structurally related to a member of the family of natural products known as the pladienolides. Plandienolides were first identified in the bacteria *Streptomyces platensis* (Sakai, Takashi; Sameshima, Tomohiro; Matsufuji, Motoko; Kawamura, Naoto; Dobashi, Kazuyuki; Mizui, Yoshiharu. "Pladienolides, New Substances from Culture of Streptomyces platensis Mer-11107. I. Taxonomy, Fermentation, Isolation and Screening." The Journal of Antibiotics. 2004, Vol. 57, No.3). One of these compounds, pladienolide B, targets the SF3B spliceosome to inhibit splicing and alter the pattern of gene expression (Kotake et al., "Splicing factor SF3b as a target of the antitumor natural product pladienolide", Nature Chemical Biology 3:570-575 [2007]). Certain pladienolide B analogs are described in WO 2002/060890; WO 2004/011459; WO 2004/011661; WO 2004/050890; WO 2005/052152; WO 2006/009276; and WO 2008/126918.

U.S. Patent Nos. 7,884,128 and 7,816,401, both entitled "Process for Total Synthesis of Pladienolide B and Pladienolide D," describe methods for synthesizing pladienolide B and D. Synthesis of pladienolide B and D may also be performed using methods described in Kanada et al., "Total Synthesis of the Potent Antitumor Macrolides Pladienolide B and D," Angew. Chem. Int. Ed. 46:4350-4355 (2007). Kanada et al., U.S. Patent No. 7,550,503, and International Publication No. WO 2003/099813 (WO '813), entitled "Novel Physiologically Active Substances," describe methods for synthesizing E7107 (Compound 45 of WO '813) from pladienolide D (11107D of WO '813). In some embodiments, the SF3B1 modulator is pladienolide B. In other embodiments, the SF3B1 modulator is pladienolide D. In further embodiments, the SF3B1 modulator is E7107.

In some embodiments, the SF3B1 modulator is a compound described in U.S. Publication Number 2015-0329528, filed May 13, 2015. In some embodiments, the SF3B1 modulating compound is a compound having one of formulas 1-4 as set forth in Table 2.

**Table 2. Exemplary SF3B1 modulating compounds.**

| Compound Structure | |
|---|---|
| 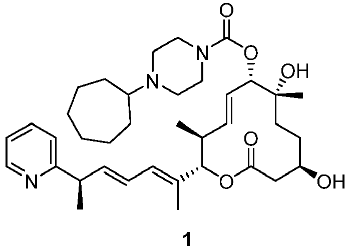 | 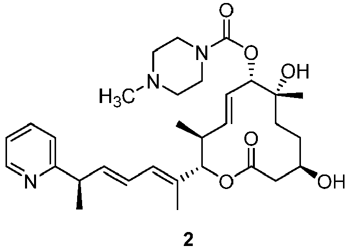 |
| 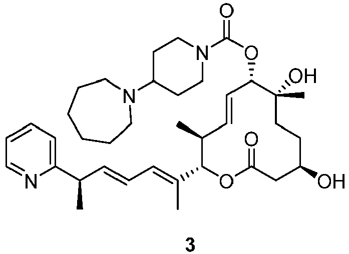 | 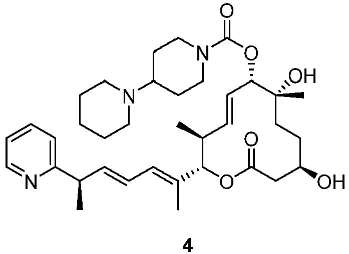 |

The methods described herein may be used to evaluate known and novel SF3B1 modulating compounds.

### Uses

Various embodiments of the invention include use of a SF3B1 modulator in a method of treating a patient diagnosed with cancer. In certain instances, cancer cells from the patient have been determined to have a mutant SF3B1 protein. Specific SF3B1 mutants include E622D, E622K, E622Q, E622V, Y623C, Y623H, Y623S, R625C, R625G, R625H, R625L, R625P, R625S, N626D, N626H, N626I, N626S, N626Y, H662D, H662L, H662Q, H662R, H662Y, T663I, T663P, K666E, K666M, K666N, K666Q, K666R, K666S, K666T, K700E, V701A, V701F, V701I, I704F, I704N, I704S, I704V, G740E, G740K, G740R, G740V, K741N, K741Q, K741T, G742D, D781E, D781G, or D781N. In certain embodiments, SF3B1 mutants are chosen from K700E, K666N, R625C, G742D, R625H, E622D, H662Q, K666T, K666E, K666R, G740E, Y623C, T663I, K741N, N626Y, T663P, H662R, G740V, D781E, or R625L. In additional embodiments, the cancer cells have been tested to measure the amount of one or more splice variants selected from Table 1. Specific splice variants associated with neomorphic SF3B1 mutations are shown in Table 1 and described in the section on splice variants above.

In certain embodiments, a cancer patient determined to have a mutant SF3B1 protein is treated with an SF3B1 modulator as described in U.S. Publication Number 2015-0329528, filed May 13, 2015.

### EXAMPLES

### Example 1: SF3B1 Mutations induce abnormal splicing in a lineage-specific manner

To investigate splicing alterations associated with SF3B1 mutations ("SF3B1^{MUT}") across multiple tumor types, an RNA-Seq quantification and differential splicing pipeline was developed and used to analyze RNA-Seq profiles from the following samples:
- all SF3B1^{MUT} samples in The Cancer Genome Atlas (TCGA; from 81 patients in all, representing 16 cancer types), and 40 wild type SF3B1 (SF3B1^{WT}) samples from each of the breast cancer (20) and melanoma (20) cohorts in TCGA,
- seven SF3B1^{MUT} and seven SF3B1 ^{WT} CLL patient samples obtained from the Lymphoma/Myeloma Service in the Division of Hematology/Oncology at the New York Weill Cornell Medical Center.

### RNA-Seq Quantification Methods

Splice junctions were quantified directly from alignments (BAM files) to facilitate discovery of unannotated splice variants. For internally generated RNA-Seq data, reads were aligned to the human reference genome hg19 (GRCh37) by MapSplice and quantified by RSEM against the TCGA GAF 2.1 isoform and gene definition, emulating the TCGA "RNASeqV2" pipeline
(see https://cghub.ucsc.edu/docs/tcga/UNC_mRNAseq_summary.pdf). Splice junction counts generated by MapSplice were used for downstream processing. For TCGA RNA-Seq data, comprehensive splice junction counts generated by MapSplice were not available; instead TCGA "Level 3" splice junction data reports mapped read counts for a predefined set of splice junctions from reference transcriptomes. To reconstruct genome-wide splice junction counts comparable to internally-generated RNA-Seq samples, raw RNA-Seq alignments (BAM files) were obtained from CGHub (https://cghub.ucsc.edu/) and any reads that span across a potential splice junction were directly counted. RSEM-estimated gene expression read counts were gathered directly from the TCGA RNA-SeqV2 Level 3 data matrices.

Because MapSplice only provides exon-exon junction counts, estimates of read counts spanning each intron-exon junction were required for identification of intron-retention splice variants. For every splice junction in each BAM file, reads with at least a 3-bp overhang across each of the 3' and 5' intron-exon junctions were counted.

For all manipulation of spliced reads within BAM files, a custom Python module "splicedbam" (available at https://github.com/h3biomed/splicedbam) was used, which uses the "pysam" extension of samtools (Li, H., et al., "The Sequence Alignment/Map format and SAMtools." Bioinformatics, 2009 Aug 15; 25(16):2078-9).

In some instances, splice junctions had very low counts, occasionally due to sequencing and alignment errors. Therefore, only splice junctions that had at least a total of 10 counts on average from either SF3B1^{WT} or SF3B1^{MUT} cohorts were included in downstream analyses.

### Differential Splicing Detection Methods

In order to detect differential usage of a splice variant in one cohort relative to another, independent of gene expression changes and pre-defined alternative splicing models, a computational differential splicing pipeline was developed that converts splice junction counts into percentages of junction usage at splice sites with multiple possible junctions. The percentage of junction usage is a measurement of the occurrence of one splice variant relative to all other splice variants that share the same splice site. For instance, a splice variant with an alternative 3' splice site must share its 5' splice site with another splice variant. Therefore, for each shared splice site, the raw counts of each splice variant were divided by the total counts of all splice variants that utilize the shared splice site in order to derive a ratio. This ratio was then multiplied by 100 to convert it to a percentage. For each sample, the sum of all of the percentages of splice variants that share the same splice site will equal 100. The transformation of raw counts of each splice variant into a percentage of all splice variants sharing a splice site is itself a normalization to reduce the effect of gene expression changes. The percentages for canonical and aberrant junctions are listed in Table 1 as "Avg WT %" and "Avg Ab. %," respectively. Differences between these percentages were assessed for statistical significance by using the *moderated t-test* defined in the Bioconductor's *limma* package (available at http://www.bioconductor.org). The statistical p-values were corrected into q values using the Benjamini-Hochberg procedure, and listed as "FDR Q-Values" in Table 1. Any splice variant that satisfied a q value of less than or equal to 0.05 was considered statistically significant.

The conversion of raw junction counts into percentage junction usage can introduce noise in some instances, i.e., when a gene in which a splice variant occurs is expressed in one cohort but has very low expression or is not expressed at all in another cohort. To address this, an additional filtering step was introduced. For each up-regulated splice variant in an SF3B1^{MUT} sample that satisfies the above q value threshold, its corresponding canonical splice variant must be down-regulated in the SF3B1^{MUT} sample and also must also satisfy the q value threshold for the up-regulated splice variant to be considered an aberrant splice variant.

### Identification of Aberrant Splice Variants in Neomorphic SF3B1^{MUT} Patient Samples

Initially, this framework was applied to a subset of known SF3B1^{MUT} cancers or wild-type counterparts from The Cancer Genome Atlas (TCGA; luminal A primary breast cancer: 7 SF3B1^{K700E} and 20 SF3B1^{WT}; metastatic melanoma: 4 SF3B1^{MUT}; and 20 SF3B1^{WT}) and internally generated 7 SF3B1^{MUT} and 7 SF3B1^{WT} CLL patient samples. This analysis revealed 626 aberrant splice junctions to be significantly upregulated in SF3B1^{MUT} compared to SF3B1^{WT}. The vast majority of aberrant splicing events use an alternative 3'ss (see Table 1, "Event" column).

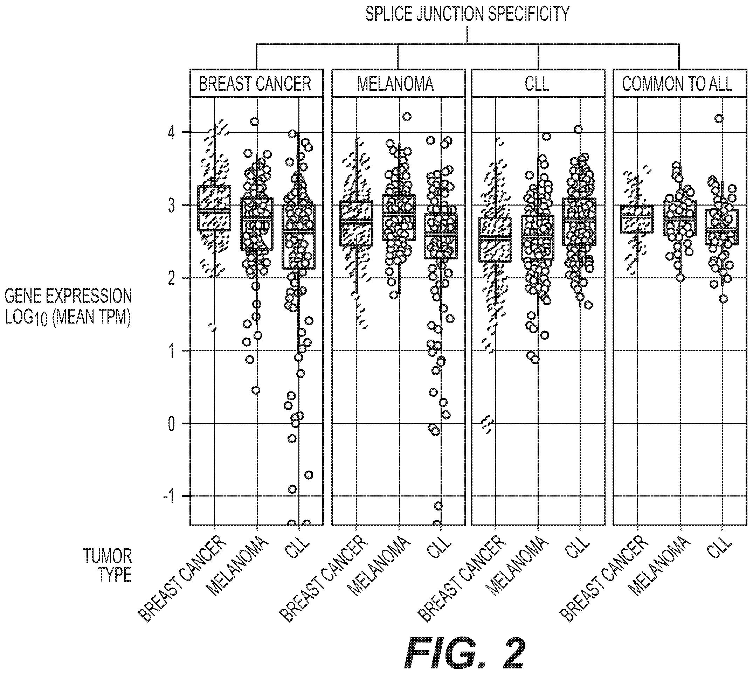

The computational screening of aberrant splicing events revealed a pattern of tumor-specific splicing events in breast cancer, melanoma and CLL in neomorphic SF3B1^{MUT} samples (Table 1). In addition, a set of tumor-non-specific events (i.e., splicing events found in at least two tumor types) was observed. Some splice variants of genes with tumor-specific splicing events occur in genes with higher mRNA expression, indicating that some of the observed tumor-specific splicing results from gene expression differences (Fig. 2).

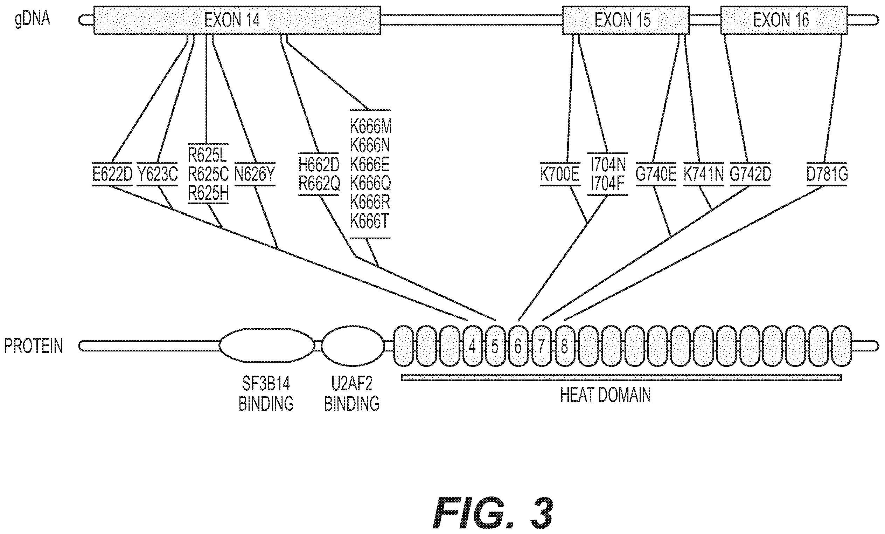

To characterize the effect of aberrant splicing in all SF3B1 variants across cancer types, RNA-Seq data for the remaining 70 SF3B1^{MUT} patients from 14 cancer types in TCGA were quantified, and an unsupervised clustering analysis was done using all 136 samples. This clustering separated splicing events associated with neomorphic SF3B1 mutants from those associated with wild-type SF3B1 or non-neomorphic SF3B1 mutants. For example, splicing patterns associated with neomorphic SF3B1 mutants were observed in breast cancer (SF3B1^{K666E}, SF3B1^{N626D}), lung adenocarcinoma (SF3B1^{K741N}, SF3B1^{G740V}), and bladder cancer (SF3B1^{R625C}) patient samples, as splicing events in these samples clustered with those in SF3B1^{K700E} neomorphic samples, whereas the splicing profiles for other SF3B1 mutant samples were similar to those of SF3B1^{WT} samples of the same tumor type. A listing of SF3B1 mutants whose splicing profiles clustered with those of neomorphic SF3B1 mutants is provided in Table 3, column 1. Additional SF3B1 mutations that are predicted to be neomorphic are listed in Table 3, column 2. A schematic diagram showing the locations of all mutations provided in Table 3 is shown in Fig. 3.

**Table 3. Select SF3B1 mutations**

| **SF3B1 Mutations with Splicing Profiles Clustering with Neomorphic SF3B1 Mutations** | **Predicted Neomorphic SF3B1 Mutations** |
|---|---|
| K700E | K666Q |
| K666N | K666M |
| R625C | H662D |
| G742D | D781G |
| R625H | I704F |
| E622D | I704N |
| H662Q | V701F |
| K666T | R625P |
| K666E | R625G |
| K666R | N626D |
| G740E | H662Y |
| Y623C | N626S |
| T663I | G740R |
| K741N | N626I |
| N626Y | N626H |
| T663P | V701I |
| H662R | R625S |
| G740V | K741T |
| D781E | K741Q |
| R625L | I704V |
| | I704S |
| | E622V |
| | Y623S |
| | Y623H |
| | V701A |
| | K666S |
| | H662L |
| | G740K |
| | E622Q |
| | E622K |
| | D781N |

### Example 2: Validation of Aberrant Splice Variants in Cell Lines

Aberrant splicing in cell line models was analyzed by collecting RNA-Seq profiles for a panel of cell lines with endogenous SF3B1 neomorphic mutations (pancreatic adenocarcinoma Panc 05.04: SF3B1^{Q699H/K700E} double mutant; metastatic melanoma Colo829: SF3B1^{P718L}; and lung cancer NCI-H358: SF3B1^{A745V}; obtained from the American Type Culture Collection [ATCC] or RIKEN BioResource Center and cultured as instructed) and from several SF3B1^{WT} cell lines from either the same tumor types (pancreatic adenocarcinoma Panc 10.05, HPAF-II, MIAPaCa-2, Panc04.03, PK-59, lung cancer NCI-H358, NCI-H1792, NCI-H1650, NCI-H1975, NCI H1838) or normal control cells of the same patient (Epstein-Barr virus [EBV]-transformed B lymphoblast colo829BL). RNA-Seq profiles were also collected from isogenic pre B-cell lines (Nalm-6) engineered via AAV-mediated homology to express SF3B1^{K700E} (Nalm-6 SF3B1^{K700E}) or a synonymous mutation (Nalm-6 SF3B1^{K700K}). The isogenic cell lines Nalm-6 SF3B1^{K700E} and Nalm-6 SF3B1^{K700K} , generated at Horizon Discovery, were cultured in presence of Geneticin (0.7 mg/ml, Life Technologies) for selection. All RNA-Seq analysis was performed using the same pipeline described for patient samples in Example 1. Unsupervised clustering of cell lines using the aberrant splice junctions identified in patients resulted in clear segregation of Panc 05.04 and Nalm-6 SF3B1^{K700E} from wild-type and other SF3B1-mutant cells.

A NanoString® assay was developed to quantify aberrant and canonical splice variants and was validated using the same cell panel. For the NanoString® assay, 750 ng of purified total RNA was used as template for the nCounter® (NanoString Technologies®) expression assay using a custom panel of NanoString® probes. The sample preparation was set up as recommended (NanoString® Technologies protocol no. C-0003-02) for an overnight hybridization at 65°C. The following day, samples were processed through the automated nCounter® Analysis System Prep Station using the high sensitivity protocol (NanoString® Technologies protocol no. MAN-C0029-05) followed by processing through the nCounter® Analysis System Digital Analyzer (protocol no. MAN-C0021-01) using 1150 FOVs for detection. Data was downloaded and analyzed for quality control metrics and normalization using the nSolver™ Analysis Software (NanoString Technologies®). The data was first normalized for lane-to-lane variation using the positive assay controls provided by the manufacturer (NanoString® positive controls A-F [containing *in vitro* transcribed RNA transcripts at concentrations of 128 fM, 32 fM, 8 fM, 2 fM, 0.5 fM, and 0.125 fM, each pre-mixed with NanoString® Reporter CodeSet probes])). Any samples with normalization factors <0.3 and >3 were not considered for further analysis. This was followed by content normalization using the geo-mean of GAPDH, EEF1A1 and RPLP0. All samples were within the recommended 0.1-10 normalization factor range. Each normalized value was then checked to ensure that it was at least two standard deviations higher than the average of background signal recorded for that lane. Any value below that was considered below detection limit. These normalized values were taken for further bioinformatics and statistical analysis.

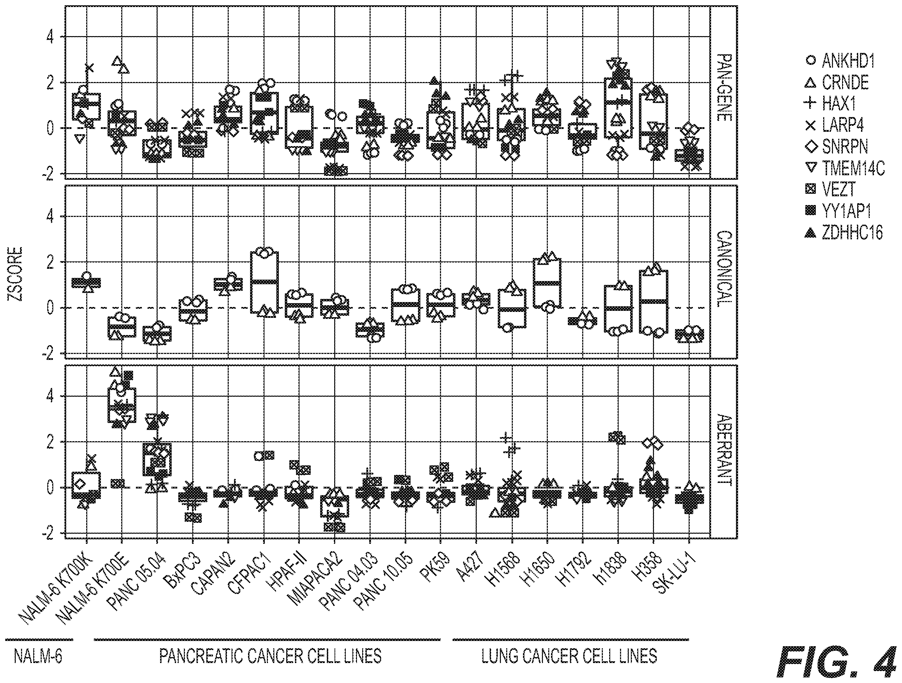

As observed in the RNA-Seq analysis, only the Panc 05.04 and isogenic Nalm-6 SF3B1^{K700E} cell lines showed clear presence of aberrant splicing (Fig. 4).

### Analysis of SF3B1 Mutant SF3B1^{Q699H}

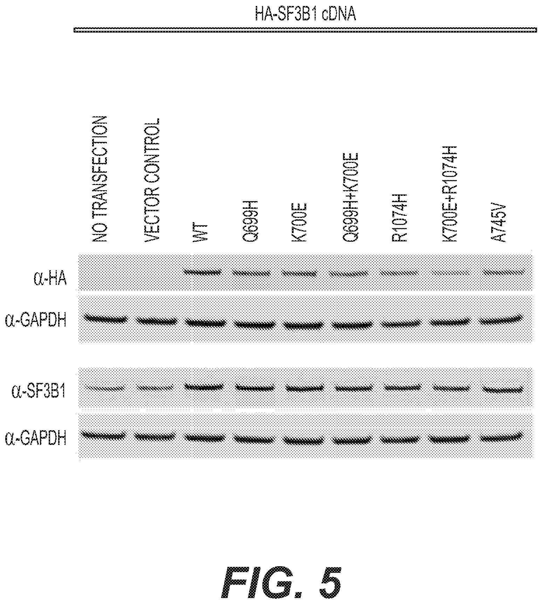

The Panc 05.04 cell line carries the neomorphic mutation SF3B1^{K700E} and an additional mutation at position 699 (SF3B1^{Q699H}). To evaluate the functional relevance of this second mutation, SF3B1^{Q699H} and SF3B1^{K700E} mutant SF3B1 proteins were expressed alone or in combination in 293FT cells (Fig. 5) for analysis of RNA by NanoString®. To express the mutants in 293FT cells, mammalian expression plasmids were generated using the Gateway technology (Life Technologies). First, the HA-tag mxSF3B1 wild-type (Yokoi, A. et al. "Biological validation that SF3b is a target of the antitumor macrolide pladienolide." FEBS J 278:4870-4880 [2011]) was cloned by PCR into the pDONR221, then the mutations were introduced using the site-directed mutagenesis kit (QuikChange II XL, Agilent). LR reaction was performed to clone all the HA-tag mxSF3B1 wild-type and mutants into the pcDNA-DEST40 (Life Technologies). 293FT cells (Life Technologies), cultured according to the manufacturer's instructions, were seeded on 6 wells/plate and transfected with generated plasmid using Fugene (Roche). One µg of DNA per pcDNA-DEST40 HA-mxSF3B1 construct was used for each transient transfection, generated in triplicates. Forty-eight hours after transfection, cells were collected to isolate protein and RNA for western blot and NanoString® analysis, respectively. Protein extracts were prepared by lysing the cells with RIPA (Boston BioProducts). Twenty-three µg of protein was loaded in a SDS-PAGE gel and identified using SF3B1 antibody (a-SAP 155, MBL) and anti-GAPDH (Sigma). Li-Cor donkey-anti-mouse 800CW and Li-Cor donkey-anti-rabbit 800CW were used as secondary antibodies and detected by Odyssey imager (Li-Cor). RNA was isolated from the cells and retrotranscribed using MagMax for Microarray and Superscript VILO II (Life Technologies), respectively, according to the manufacturer manual, and then analyzed with the NanoString® assay.

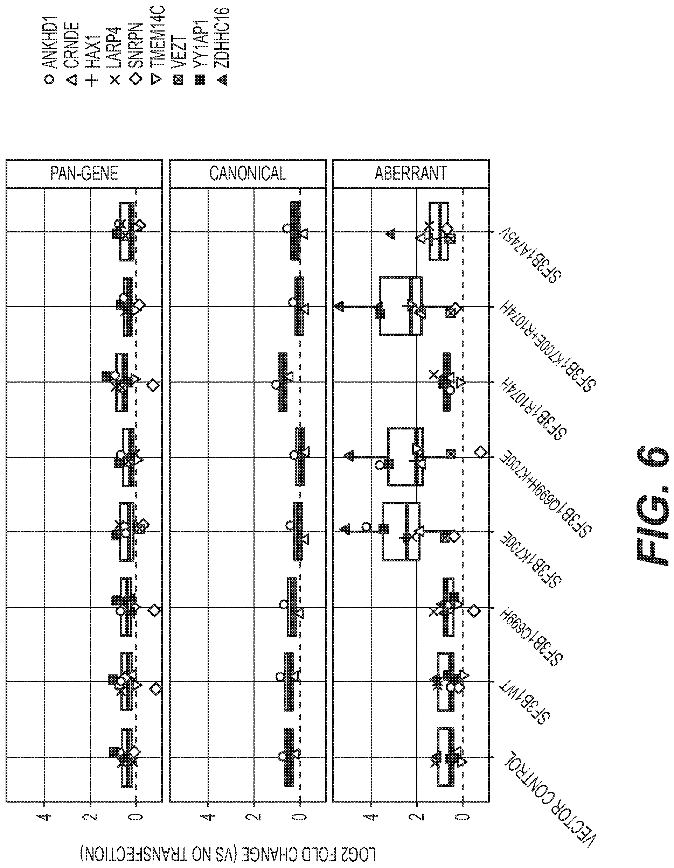

Expression of SF3B1^{K700E} and SF3B1^{Q699H/K700E} induced aberrant splicing, whereas SF3B1^{Q699H} alone or SF3B1^{A745V} or SF3B1^{R1074H} (a substitution conferring resistance to the spliceosome inhibitor pladienolide B) did not induce aberrant splicing (Fig. 6), indicating that SF3B1^{Q999H} is a non-functional substitution.

These data confirm that Panc 05.04 and Nalm-6 SF3B1^{K700E} isogenic cells are representative models to study the functional activity of SF3B1 neomorphic mutations and the activity of splicing inhibitors *in vitro* and *in vivo.*

### Example 3: Neomorphic SF3B1 Mutations Induce Abnormal mRNA Splicing

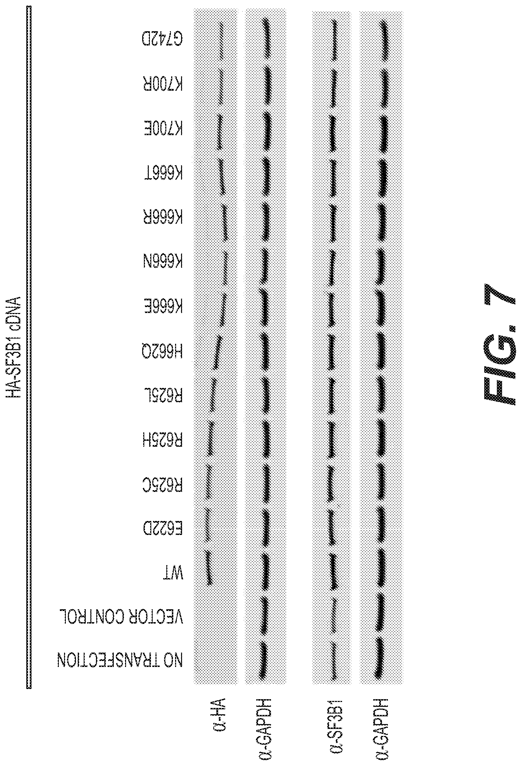

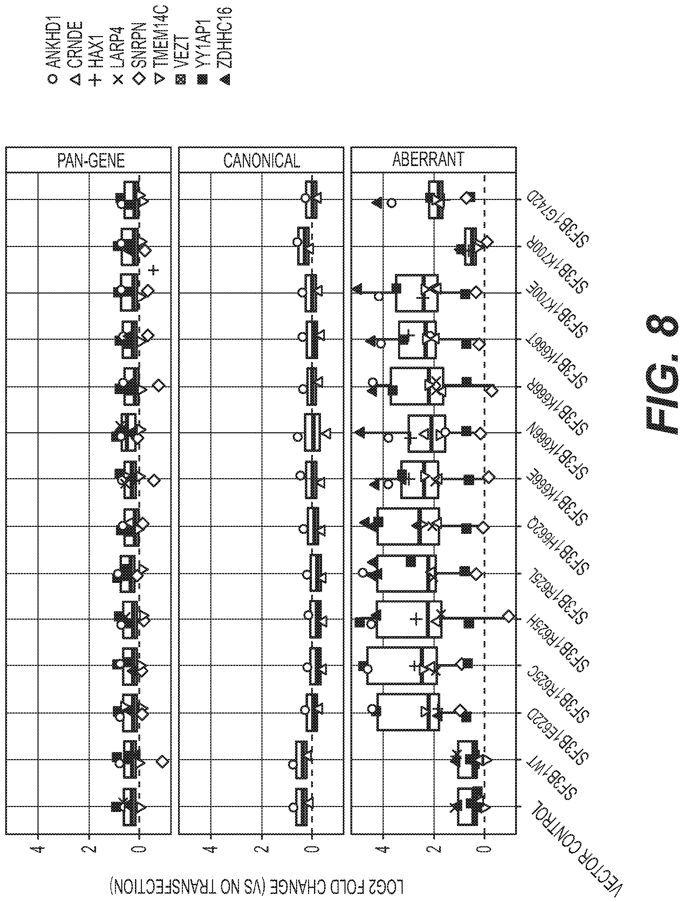

The functional activity of neomorphic mutations found in SF3B1^{MUT} cancers was analyzed by expressing SF3B1^{WT}, neomorphic SF3B1 mutants, or SF3B1^{K700R} (the mutation observed in a renal clear cell carcinoma patient that clusters with SF3B1^{WT} patients) in 293FT cells and determining splicing aberrations by NanoString®. The expression of all constructs was confirmed by western blot (Fig. 7). All SF3B1 neomorphic mutations tested demonstrated the same usage of alternative splice sites observed in patient samples ("MUT isoform" in Fig. 8), but SF3B1^{K700R} and SF3B1^{WT} did not show aberrant splicing (Fig. 8). Moreover, the expression of none of the SF3B1 constructs changed the overall gene expression ("PAN - gene" in Fig. 8) or the canonical splice isoforms ("WT isoform" in Fig. 8). This indicated both a correlation between the presence of the neomorphic SF3B1 mutations and alternative splicing as well as similar functional activity of the different neomorphic mutations, as was indicated by the RNA-Seq analysis of patient samples.

The correlation between the SF3B1^{K700E} neomorphic mutation and aberrant splicing was analyzed using tetracycline-inducible shRNA to selectively knockdown the neomorphic SF3B1 mutant or SF3B1^{WT} allele in Panc 05.04 and Panc 10.05 cell lines (neomorphic SF3B1^{MUT} and SF3B1^{WT} cell lines, respectively; obtained from the American Type Culture Collection [ATCC] or from RIKEN BioResource Center and cultured as instructed).

For the knockdown experiment, virus encoding shRNA was prepared in LentiX-293T cells (Clontech), which were cultured according to the manufacturer's instruction. The inducible shRNA were cloned into AgeI and EcoRI of the pLKO-iKD-H1 puro vector. The sequences of hairpins were:
shRNA #13 SF3B1^{PAN} GCGAGACACACTGGTATTAAG (SEQ ID NO: 1180),
shRNA #8 SF3B1^{WT} TGTGGATGAGCAGCAGAAAGT (SEQ ID NO: 1181); and
shRNA #96 SF3B1^{MUT} GATGAGCAGCATGAAGTTCGG (SEQ ID NO: 1182).

Cells were transfected with 2.4 µg of target pLKO-shRNA plasmid, plus 2.4 µg of p Δ 8.91 (packaging), and 0.6 µg VSVG (envelope) using TransIT reagent (Mirus). The virus was used to infect Panc 05.04 and Panc 10.05 by spin infection using Polybrene (Millipore). The day after infection, the cells were cultured in selecting media (1.25 µg/ml Puromycin [Life Technologies]) for 7 days to select for shRNA-expressing cells. The selected cells were cultured in the presence or absence of Doxycycline hyclate (100 ng/mL; Sigma) to induce the shRNA. Cells were harvested for protein and RNA at day 4 post-induction. In addition, cells were seeded for colony forming assay and CellTiter-Glo® assay (Promega). At day 9, cells were fixed with formaldehyde and stained with crystal violet.

To confirm SF3B1 knockdown using western blots, protein extracts were prepared by lysing the cells with RIPA (Boston BioProducts). Twenty to 25 µg of protein from each sample was separated by SDS-PAGE and transferred to nitrocellulose membranes (iblot, Life Technologies). Membranes were first blocked with Odyssey Blocking Buffer (Li-Cor) and then incubated with SF3B1 antibody (a-SAP 155, MBL) and anti-GAPDH (Sigma). Li-Cor donkey-anti-mouse 800CW and Li-Cor donkey-anti-rabbit 800CW were used as secondary antibodies and detected by Odyssey imager (Li-Cor).

To confirm SF3B1 knockdown by allele specific qPCR, RNA was isolated from the cells and retrotranscribed using MagMax for Microarray and Superscript VILO II (Life Technologies), respectively according to the manufacturer manual. qPCR was performed using ViiA7 (Life Technologies). The reaction included 20-50 ng cDNA, Power SYBR green master mix (Life Technologies) and 300 nM primers. The following primers were used:
SF3B1^{WT}: FW 5'-GACTTCCTTCTTTATTGCCCTTC (SEQ ID NO: 1183) and
   RW 5'-AGCACTGATGGTCCGAACTTTC (SEQ ID NO: 1184),
SF3B1^{MUT}: FW 5'-GTGTGCAAAAGCAAGAAGTCC (SEQ ID NO: 1185) and
   RW 5'-GCACTGATGGTCCGAACTTCA (SEQ ID NO: 1186),
SF3B1^{PAN}: FW 5'-GCTTGGCGGTGGGAAAGAGAAATTG (SEQ ID NO: 1187) and
   RW 5'-AACCAGTCATACCACCCAAAGGTGTTG (SEQ ID NO: 1188),
β-actin (internal control): FW 5'-GGCACCCAGCACAATGAAGATCAAG (SEQ ID NO: 1189) and
   RW 5'-ACTCGTCATACTCCTGCTTGCTGATC (SEQ ID NO: 1190).
Biological and technical triplicates were performed.

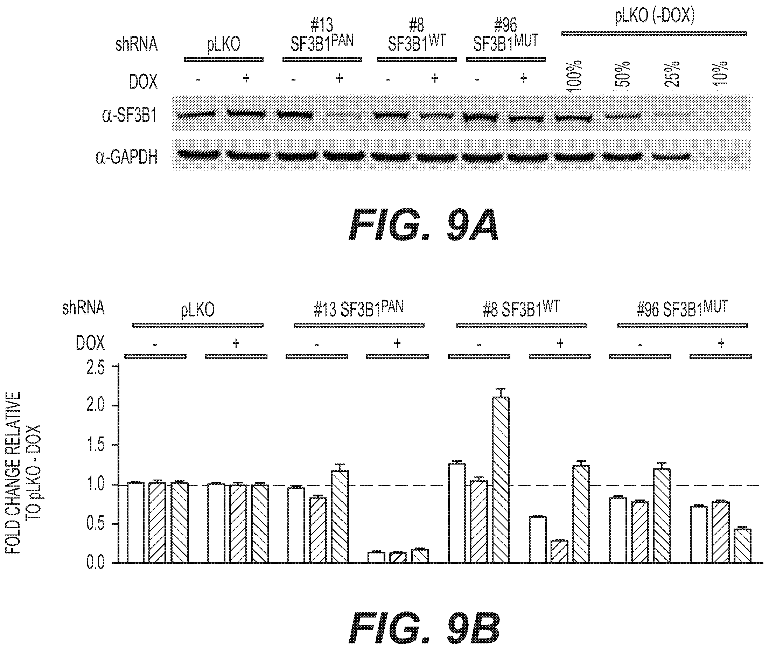

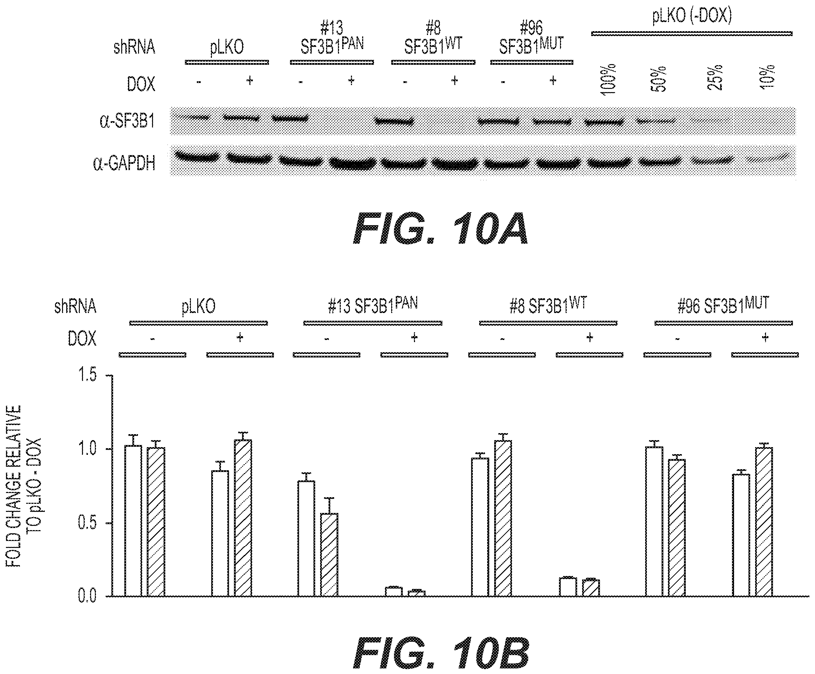

The western blotting and allele specific PCR both confirmed knockdown of the SF3B1 alleles (Figs. 9 and 10).

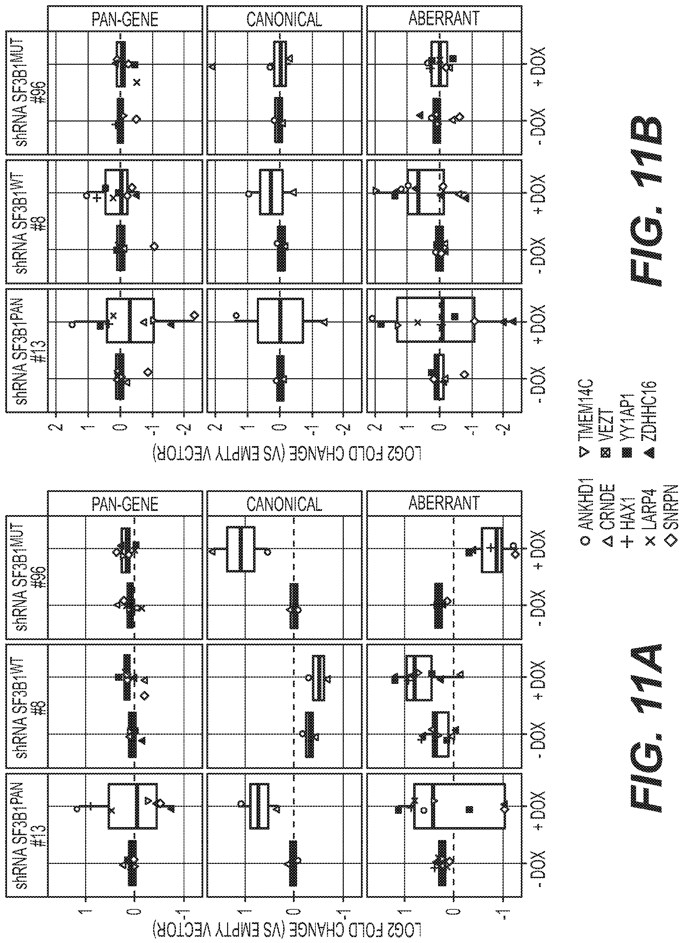

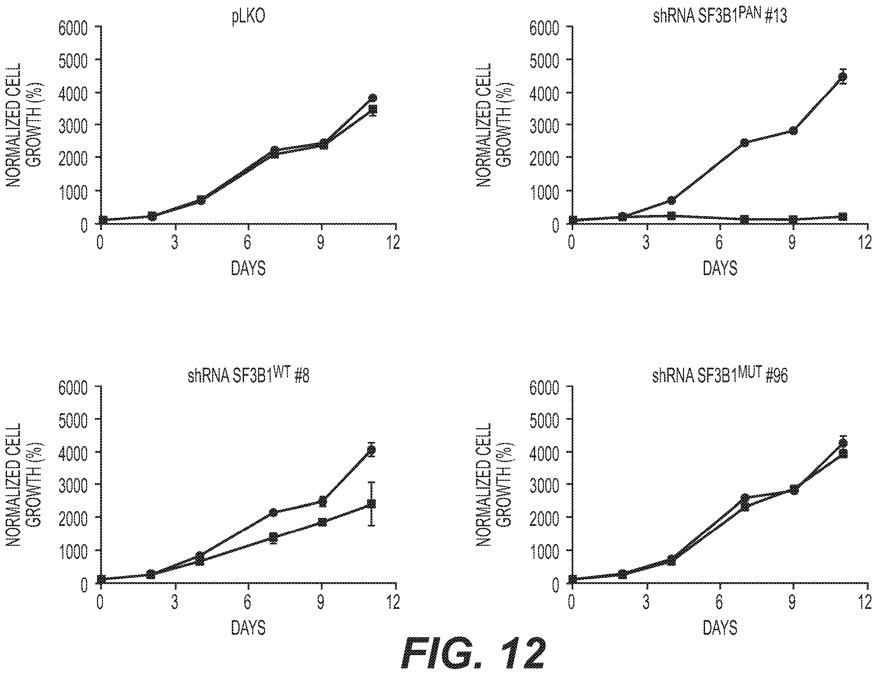

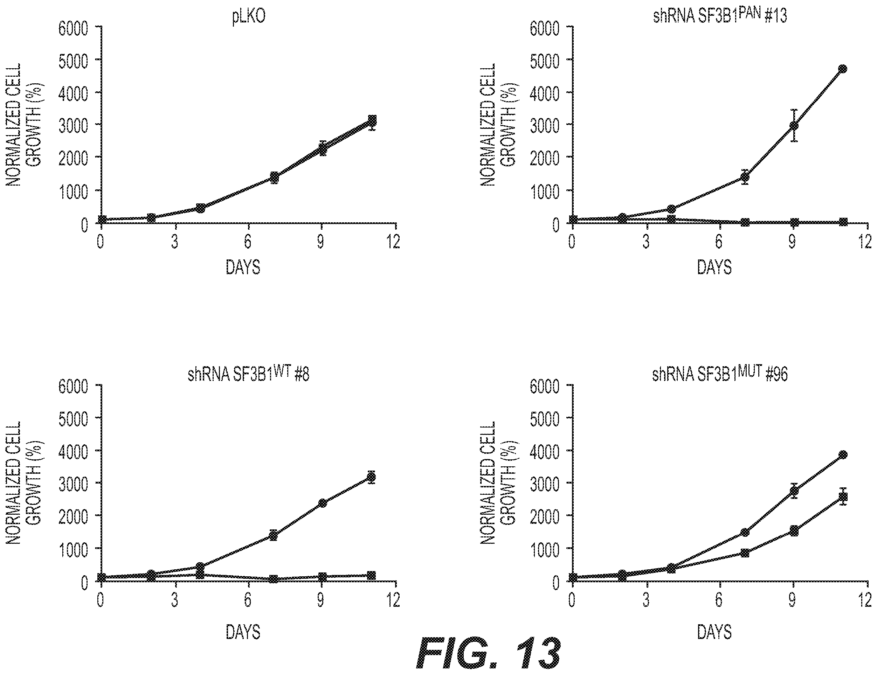

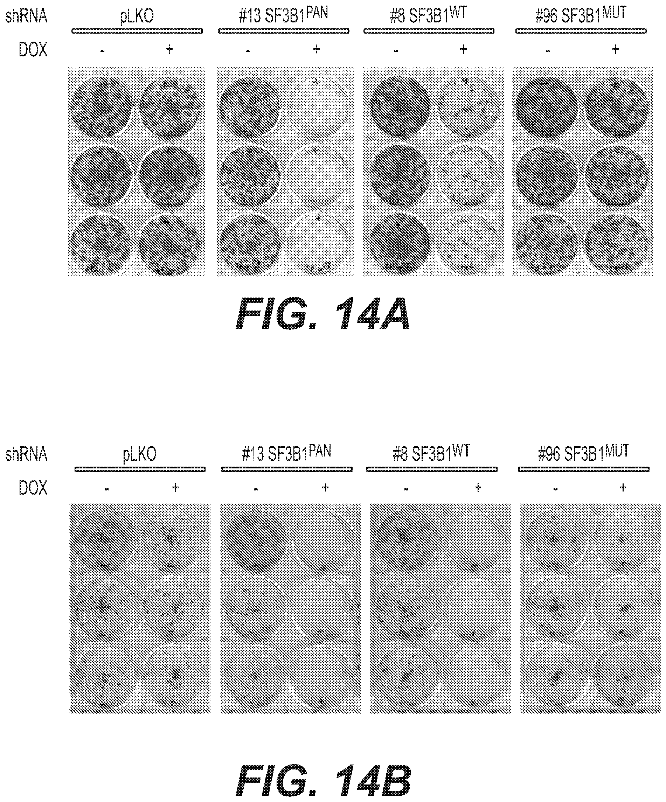

To determine the association between the expression of SF3B1 mutations and aberrant splicing, RNA isolated from the cells following doxycycline-induced knockdown was analyzed by NanoString®. In Panc 05.04, after knockdown of the neomorphic SF3B1^{MUT} allele, the aberrant splice variants were downregulated and the canonical splice variants were upregulated, whereas the opposite was observed with selective depletion of the SF3B1^{WT} allele (Fig. 11A), indicating that the neomorphic SF3B1^{MUT} protein does not possess wild-type splicing activity. The expression of a pan shRNA induced the regulation of all splice variants as well as the depletion of SF3B1^{WT} in Panc 10.05 cells (Fig. 11B). SF3B1^{PAN} knockdown impaired growth and colony formation in both cell lines, while a minimal effect was observed with selective depletion of neomorphic SF3B1^{MUT} in Panc05.04 cells (Figs. 12 and 13). When the SF3B1^{WT} allele was knocked down in Panc 05.04 cells, only a partial viability effect was observed, whereas SF3B1^{PAN} knockdown prevented colony formation and cell proliferation (Figs. 12 and 14), indicating that pan-inhibition of SF3B1 leads to antitumor activity *in vitro* and *in vivo.*

### Example 4: Modulation of neomorphic SF3B1^{MUT} splicing

### Overall Effect of E7107 on Splicing

E7107 is a small-molecule compound that inhibits splicing by targeting the U2 snRNP-associated complex SF3B (Kotake, Y. et al. "Splicing factor SF3b as a target of the antitumor natural product pladienolide." Nat Chem Biol 3, 570-575, doi:10.1038/nchembio.2007.16 [2007]). The ability of E7107 to inhibit splicing was observed in an *in vitro* splicing assay (IVS) using the substrate Ad2 (Pellizzoni, L., Kataoka, N., Charroux, B. & Dreyfuss, G. "A novel function for SMN, the spinal muscular atrophy disease gene product, in pre-mRNA splicing." Cell 95, 615-624 [1998]) and nuclear extracts from the Nalm-6 isogenic cell lines or 293F cells (Life Technologies; cultured according to the manufacturer's instructions) expressing Flag-tag SF3B1^{WT} or SF3B1^{K700E}, as follows.

Nuclear extracts were prepared from 293F cells transfected with pFLAG-CMV-2-SF3B1 plasmids, or from isogenic Nalm-6 cells (SBH Sciences). The plasmids were generated by cloning the mxSF3B1 gene into the HindIII and KpnI sites of pFLAG-CMV2 (Sigma), and the mutations mxSF3B1^{K700E}, mxSF3B1^{R1074H} and mxSF3B1^{K700E-R1074H} were introduced using the same site-directed mutagenesis kit. Cell pellets were resuspended in hypotonic buffer (10 mM HEPES pH 7.9, 1.5 mM MgCl₂, 10 mM KCl, 0.2 mM PMSF, and 0.5 mM DTT; for Nalm-6 cells, 40 mM KCl was used). The suspension was brought up to a total of five packed cell volumes (PCV). After centrifugation, the supernatant was discarded, and the cells were brought up to 3 PCV with hypotonic buffer, and incubated on ice for 10 minutes. Cells were lysed using a dounce homogenizer and then centrifuged. The supernatant was discarded, and the pellet was resuspended with ½ packed nuclear volume (PNV) of low salt buffer (20 mM HEPES pH 7.9, 1.5 mM MgCl₂, 20 mM KCl, 0.2 mM EDTA, 25% glycerol, 0.2 mM PMSF, 0.5 mM DTT), followed by ½ PNV of high salt buffer (same as low salt buffer except 1.4M KCl was used). The nuclei were gently mixed for 30 minutes before centrifuging. The supernatant (nuclear extract) was then dialyzed into storage buffer (20 mM HEPES pH 7.9, 100 mM KCl, 0.2 mM EDTA, 20% glycerol, 0.2 mM PMSF, 0.5 mM DTT). Protein concentration was determined using NanoDrop 8000 UV-Vis spectrophotometer (Thermo Scientific).

For *in vitro* splicing (IVS) reactions, an Ad2-derived sequence (Pellizzoni, L., Kataoka, N., Charroux, B. & Dreyfuss, G. "A novel function for SMN, the spinal muscular atrophy disease gene product, in pre-mRNA splicing." Cell 95, 615-624 [1998]) was cloned into the pGEM-3Z vector (Promega) using EcoRI and XbaI restriction sites. The resulting pGEM-3Z-Ad2 plasmid was linearized using XbaI, purified, resuspended in TE buffer, and used as a DNA template in the *in vitro* transcription reaction. The Ad2 pre-mRNA was generated and purified using MEGAScript T7 and MegaClear kits, respectively (Invitrogen). Twenty µL splicing reactions were prepared using 80 µg nuclear extracts, 20U RNAsin Ribonuclease inhibitor (Promega), 10 ng Ad2 pre-mRNA, and varying concentrations of E7107. After a 15 minute pre-incubation, activation buffer (0.5 mM ATP, 20 mM creatine phosphate, 1.6 mM MgCl₂) was added to initiate splicing, and the reactions were incubated for 90 minutes. RNA was extracted using a modified protocol from a RNeasy 96 Kit (Qiagen). The splicing reactions were quenched in 350 µL Buffer RLT Plus (Qiagen), and 1.5 volume ethanol was added. The mixture was transferred to an RNeasy 96 plate, and the samples were processed as described in the kit protocol. RNA was diluted 1/10 with dH₂O. 10 µL RT-qPCR reactions were prepared using TaqMan RNA-to-C_{T} 1-step kit (Life Technologies), 8.5 µL RNA, and 1 µL of Ad2 mRNA primers/probe set (FW 5' ACTCTCTTCCGCATCGCTGT (SEQ ID NO: 1191), RW 5'-CCGACGGGTTTCCGATCCAA (SEQ ID NO: 1192) and probe 5' CTGTTGGGCTCGCGGTTG (SEQ ID NO: 1193)).

To evaluate pSF3B1, *in vitro* splicing reactions were prepared as described above. To quench the reactions, 6X Laemmli Buffer (Boston Bioproducts) was added, and the samples were subjected to SDS-PAGE gels (Life Technologies). The separated proteins were transferred onto nitrocellulose membranes then blocked with blocking buffer (50% Odyssey Blocking Buffer (Li-Cor Biosciences) and 50% TBST). The blots were incubated with anti-SF3B1 antibody overnight, after several washes in TBST, they were incubated with IRDye 680LT donkey-α-mouse-IgG antibody and visualized using an Odyssey CLx imaging system (Li-Cor Biosciences).

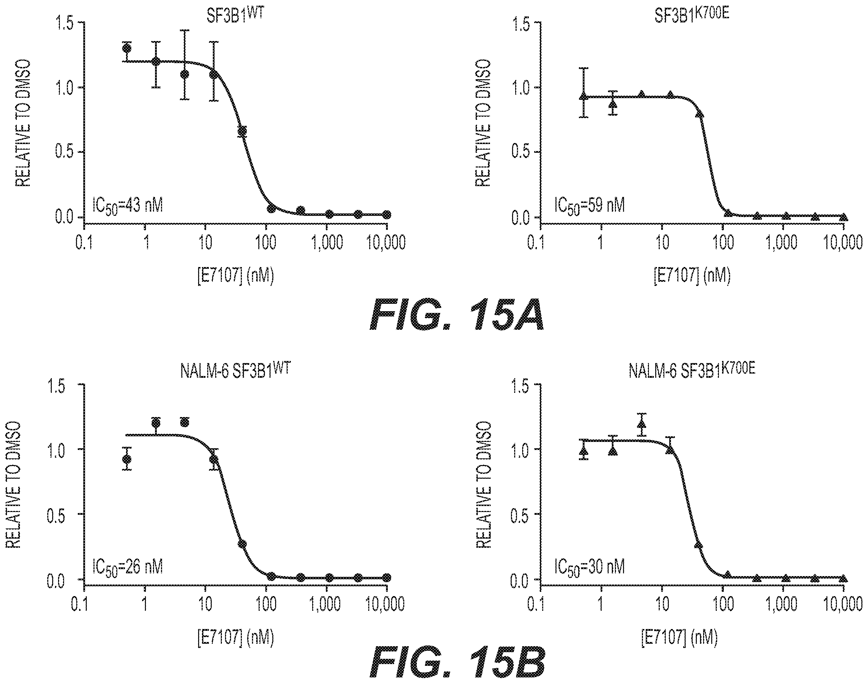

E7107 was able to inhibit splicing in nuclear extracts from both the Nalm-6 cells or the 293F cells expressing Flag-tag SF3B1^{WT} or SF3B1^{K700E} (Figs. 15A and 15B).

### E7107 Binds Both SF3B1^{WT} and SF3B1^{K700E} Proteins

The ability of E7107 to bind both SF3B1^{WT} and SF3B1^{K700E} proteins was evaluated in a competitive binding assay using Flag-tag SF3B1 proteins immunoprecipitated with anti-Flag antibody from transiently transfected 293F cells. Batch immobilization of antibody to beads was prepared by incubating 80 µg of anti-SF3B1 antibody (MBL International) and 24 mg anti-mouse PVT SPA scintillation beads (PerkinElmer) for 30 minutes. After centrifugation, the antibody-bead mixture was resuspended in PBS supplemented with PhosSTOP phosphatase inhibitor cocktail (Roche) and complete ULTRA protease inhibitor cocktail (Roche). Nuclear extracts were prepared by diluting 40 mg into a total volume of 16 mL PBS with phosphatase and protease inhibitors, and the mixture was centrifuged. The supernatant was transferred into a clean tube, and the antibody-bead mixture was added and incubated for two hours. The beads were collected by centrifuging, washed twice with PBS + 0.1% Triton X-100, and resuspended with 4.8 mL of PBS. 100 µL binding reactions were prepared using slurry and varying concentrations of E7107. After 15 minutes pre-incubation at room temperature, one nM ³H-probe molecule (described in Kotake, Y. et al. Splicing factor SF3b as a target of the antitumor natural product pladienolide. Nat Chem Biol 3, 570-575, doi:10.1038/nchembio.2007.16 [2007]) was added. The mixture was incubated at room temperature for 15 minutes, and luminescence signals were read using a MicroBeta2 Plate Counter (PerkinElmer).

As shown in Fig. 16A, E7107 was able to competitively inhibit binding of the ³H-probe molecule in a similar manner to either SF3B1^{WT} (IC₅₀: 13 nM) or SF3B1^{K700E} (IC₅₀: 11 nM).

### Effect of E7107 and Other Compounds on Normal and Aberrant Splicing

E7107 was also tested *in vitro* in Nalm-6 isogenic cell lines for the ability to modulate normal and aberrant splicing induced by SF3B1^{WT} and SF3B1^{K700E} protein. Nalm-6 isogenic cells were treated with increasing concentrations of E7107 for six hours and RNA was analyzed by qPCR. As shown in Fig. 16B, canonical splicing was observed, with accumulation of pre-mRNA for EIF4A1 and downregulation of the mature mRNA SLC25A19 observed in both cell lines. Additionally, downregulation of mature mRNA of the two abnormally spliced isoforms of COASY and ZDHHC16 was observed in Nalm-6 SF3B1^{K700E} (Fig. 16B).

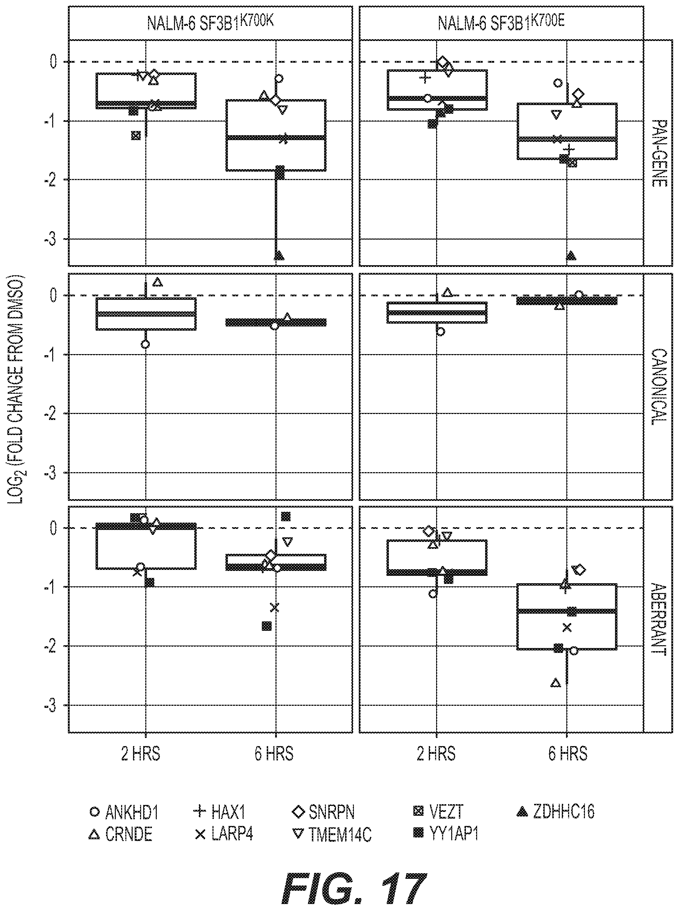

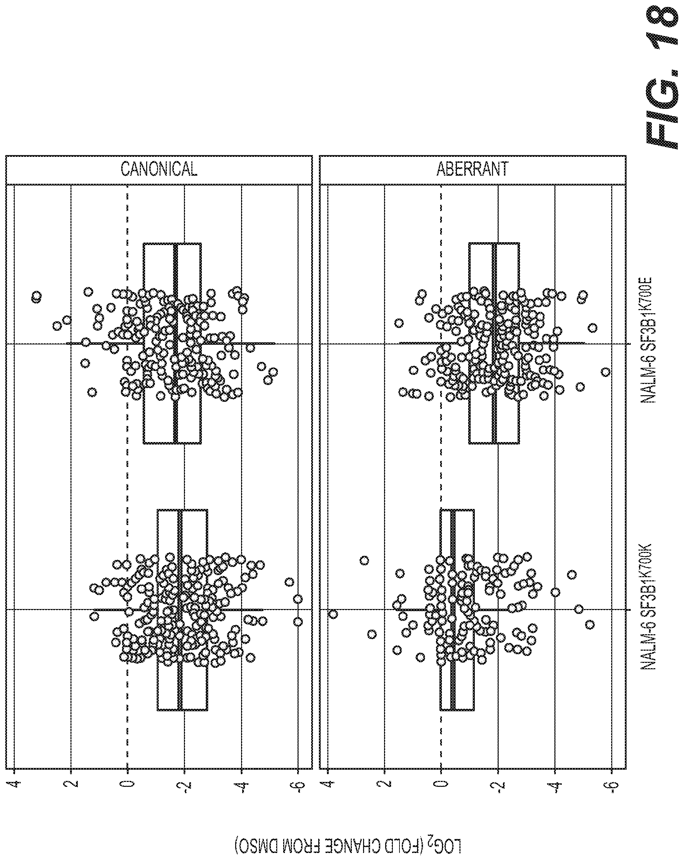

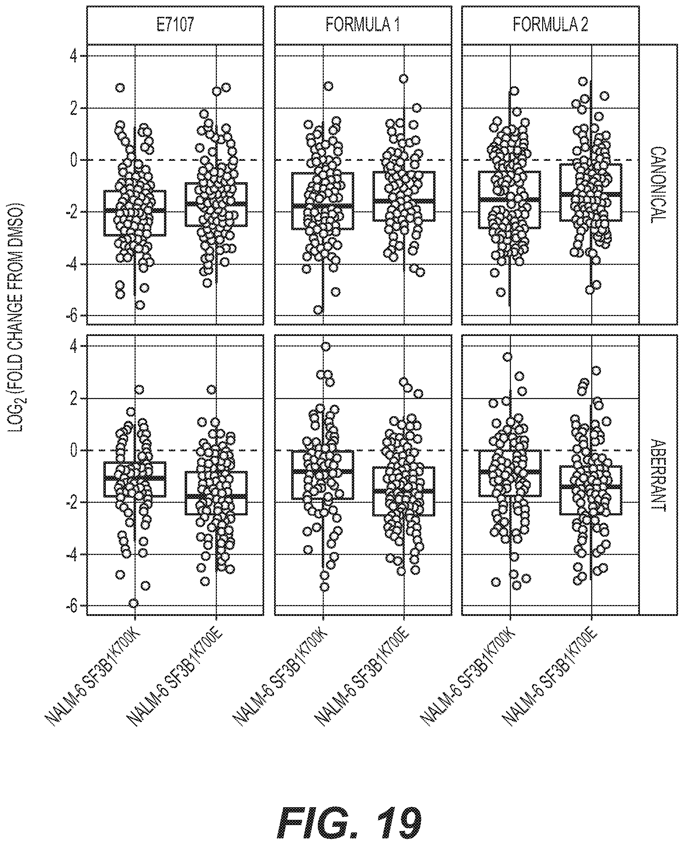

To investigate the broader activity of E7107 on normal and aberrant splicing, RNA from Nalm-6 isogenic cells treated for two and six hours at 15 nM was analyzed by NanoString®. Only partial inhibition of splicing was observed at two hours in both isogenic cell lines, and at the level of gene, WT-associated isoforms, and MUT-associated isoform expression. After six hours of treatment, clear inhibition was detected for all isoforms quantified (Fig. 17). Similar results were obtained by RNA-Seq analysis of isogenic cell lines treated for six hours with E7107 at 15 nM (Fig. 18). Normal and aberrant splicing in the isogenic cell lines was also analyzed by RNA-Seq following treatment with one of additional compounds having formulas 1 or 2. Like E7107, each of these additional compounds inhibited expression of both WT-associated and MUT-associated RNA isoforms (Fig. 19; compound is indicated by formula number above each vertical pair of graphs). For the RNA-Seq analysis, cells were washed with PBS after treatment with E7107 or other test compound, and RNA was isolated using PureLink (Life Technology) as reported in the manufacturer's manual. cDNA library preparation, sequencing and raw read filtering was performed as described in Ren, S. et al. "RNA-Seq analysis of prostate cancer in the Chinese population identifies recurrent gene fusions, cancer-associated long noncoding RNAs and aberrant alternative splicings." Cell Res 22, 806-821, doi:10.1038/cr.2012.30 (2012).

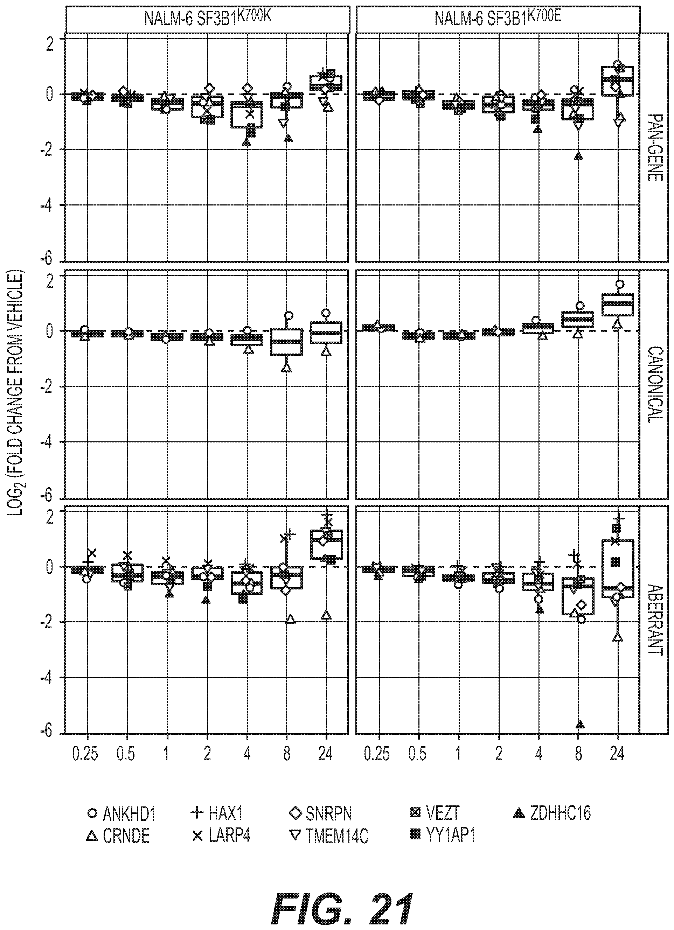

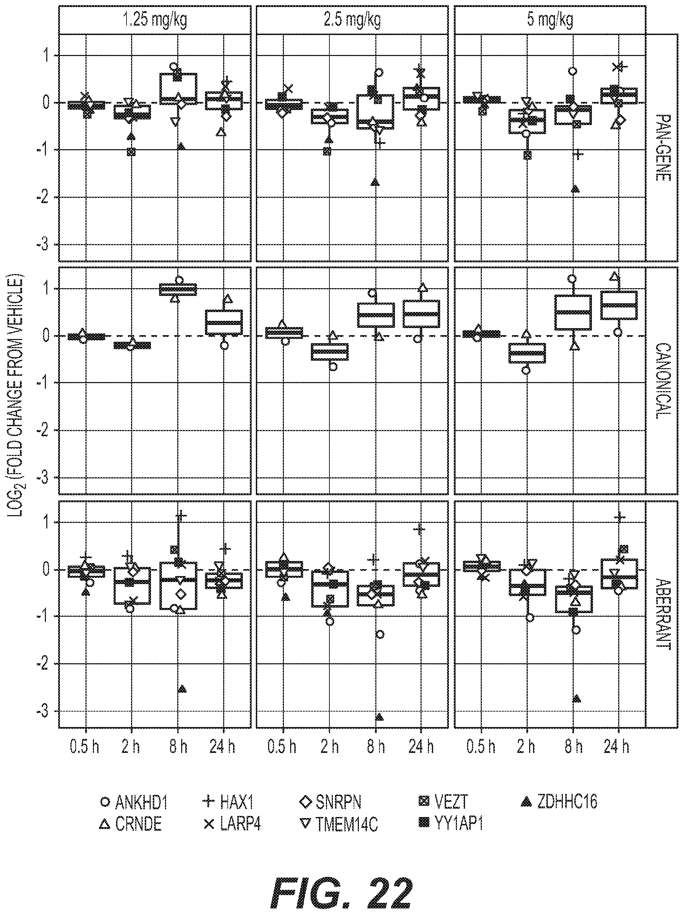

In addition, the ability of E7107 to modulate splicing was tested in mice bearing human tumor xenografts. Nalm-6 isogenic xenograft mice were generated by subcutaneously implanting 10x10⁶Nalm-6 isogenic cells into the flank of CB17-SCID mice, and tumors from these mice were collected at different timepoints after a single intravenous (IV) dose of E7107 (5 mg/kg) and analyzed to determine compound concentrations and splicing regulation. RNA was isolated from the tumors using RiboPure™ RNA purification kit (Ambion®) and used for NanoString® assay or qPCR. The RNA was retrotranscribed according to the instructions of the SuperScript® VILO™ cDNA synthesis kit (Invitrogen™) and 0.04 µl of cDNA was used for qPCR. qPCR for pre-mRNA EIF4A1 and mature mRNA SLC24A19 and pharmacokinetic evaluation were performed as described in Eskens, F. A. et al. "Phase I pharmacokinetic and pharmacodynamic study of the first-in-class spliceosome inhibitor E7107 in patients with advanced solid tumors." Clin Cancer Res 19, 6296-6304, doi:10.1158/1078-0432.CCR-13-0485 (2013). The primers and probes used for ZDHHC16 were the following: FW 5'-TCTTGTCTACCTCTGGTTCCT (SEQ ID NO: 1194), RW 5' CCTTCTTGTTGATGTGCCTTTC (SEQ ID NO: 1195) and probe 5' FAM CAGTCTTCGCCCCTCTTTTCTTAG (SEQ ID NO: 1196). The primers and probes used for COASY were the following: FW 5'-CGGTGGTGCAAGTGGAA (SEQ ID NO: 1197), RW 5'-GCCTTGGTGTCCTCATTTCT (SEQ ID NO: 1198) and probe 5'-FAM-CTTGAGGTTTCATTTCCCCCTCCC (SEQ ID NO: 1199). E7107 reached similar drug concentrations and modulated canonical splicing (accumulation of pre-mRNA for EIF4A1 and downregulation of the mature mRNA SLC25A19) in both Nalm-6 SF3B1^{K700K} and Nalm-6 SF3B1^{K700E} models and downregulated abnormal splicing of COASY and ZDHHC16 in the Nalm-6 SF3B1^{K700E} cells (Fig. 20), as observed *in vitro.* The canonical and aberrant splice mRNA isoforms were downregulated by E7107 as early as one hour following administration of the compound, and expression normalized shortly after treatment (Fig. 21), consistent with E7107 pharmacokinetic profile. Similar results were observed in a Panc 05.04 neomorphic SF3B1 xenograft model (Fig. 22). All these data indicate that E7107 is a pan-splicing modulator that can bind and inhibit SF3B1^{WT} and SF3B1^{K700E} proteins *in vitro* and *in vivo.*

### Example 5: E7107 Has Anti-Tumor Activity via SF3B1 Modulation

SF3B1 modulator E7107 was tested for antitumor activity *in vivo* by determining the effect of E7107 in a subcutaneous model of Nalm-6 SF3B1^{K700E}. 10x10⁶Nalm-6 SF3B1^{K700E} were subcutaneously implanted into the flank of CB17-SCID mice, and mice were administered E7107 intravenously once a day for 5 consecutive days (QDx5) at three well tolerated dose levels (1.25, 2.5 and 5 mg/kg). After this dosing, the animals were monitored until they reached either of the following endpoints: 1) excessive tumor volume measured three times a week (tumor volume calculated by using the ellipsoid formula: (length × width²)/2), or 2) development of any health problem such as paralysis or excessive body weight loss. Partial regression (PR) and complete regression (CR) are defined as 3 consecutive tumor measurements <50% and <30% of starting volume respectively.

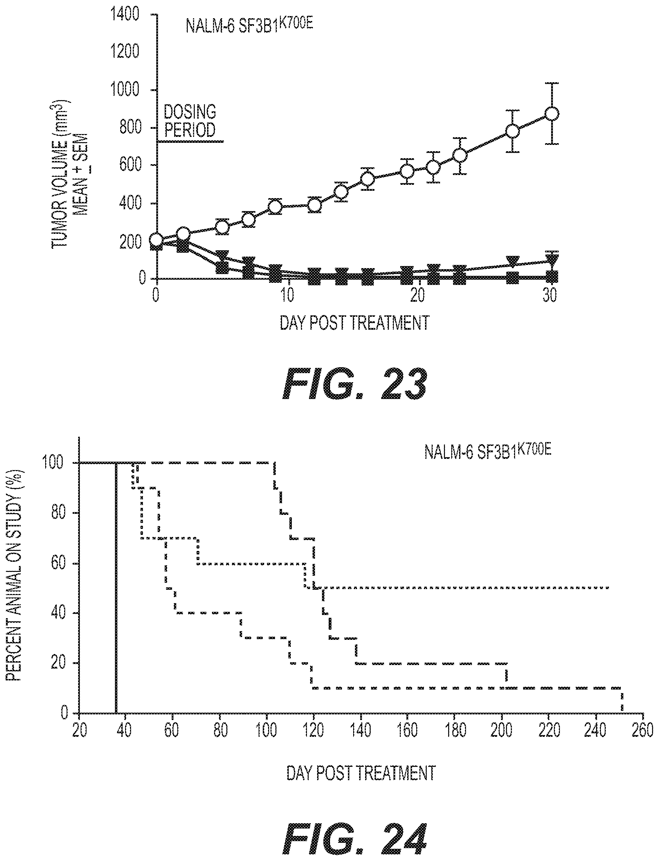

In the 1.25 mg/kg group, all animals (n=10) reached complete regression (CR) in the Nalm-6 SF3B1^{K700E} xenograft group. In the 2.5 mg/kg group, 10/10 CRs were observed in the Nalm-6 SF3B1^{K700E} group by day 9. In the 5 mg/kg group all Nalm-6 SF3B1^{K700E} xenograft animals reached CR as early as 9 days post treatment and had mean survival times of over 250 days (Figs. 23 and 24). These data demonstrate antitumor activity of SF3B1 modulator in SF3B1^{K700E} xenografts *in vivo.*

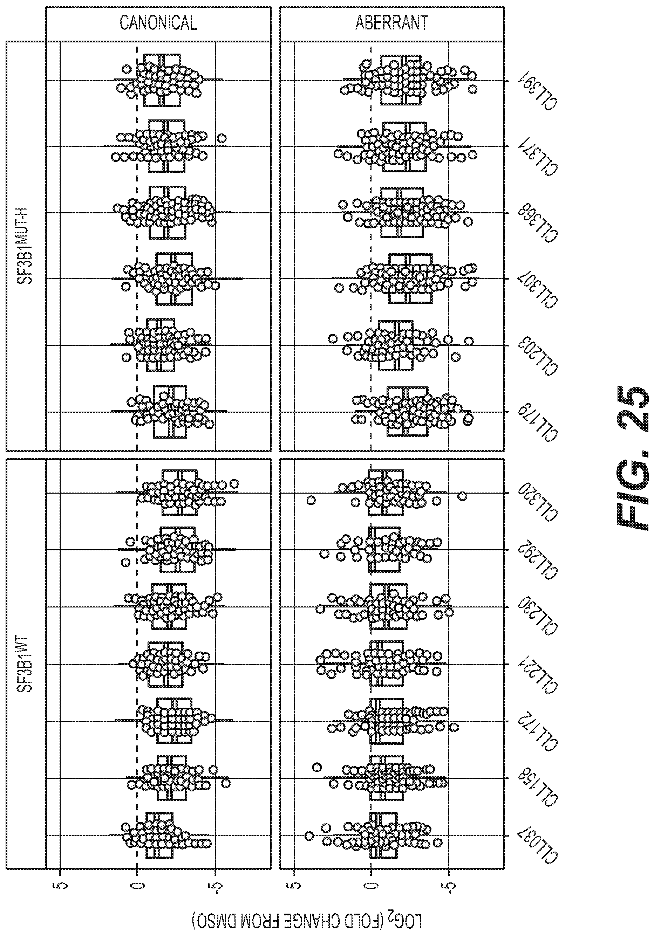

The ability of E7107 to inhibit splicing in CLL patient samples *in vitro* was determined by isolating RNA from samples of E7107-treated patient cells treated for 6 hours with E7107 at 10 nM and performing RNA-Seq analysis. To do so, cells were washed with PBS after treatment with E7107, and RNA was isolated using PureLink (Life Technology) as reported in the manufacturer's manual. cDNA library preparation, sequencing and raw read filtering was performed as described in Ren, S. et al. "RNA-Seq analysis of prostate cancer in the Chinese population identifies recurrent gene fusions, cancer-associated long noncoding RNAs and aberrant alternative splicings." Cell Res 22, 806-821, doi:10.1038/cr.2012.30 (2012). As shown in Fig. 25, E7107 inhibited the expression of canonical splice isoforms in SF3B1^{WT} and neomorphic SF3B1^{MUT} patient samples. E7107 was able to modulate aberrant splicing in all CLL patient samples carrying neomorphic SF3B1 mutations.

Other aspects of the disclosure will be apparent to those skilled in the art from consideration of the specification and practice of the invention disclosed herein. It is intended that the specification and examples be considered as exemplary only, with a true scope of the invention being indicated by the following claims.

## Claims

1. A method of identifying a patient having cancer with a neomorphic SF3B1 mutation, comprising detecting, or measuring the amount of, one or more splice variants selected from SEQ ID NO: 41, SEQ ID NO: 61, SEQ ID NO: 101, SEQ ID NO: 160, and SEQ ID NO: 230 in a sample comprising one or more cells from the patient.

2. A method of identifying a cancerous cell with a SF3B1 neomorphic mutation, comprising detecting, or measuring the amount of, one or more splice variants selected from SEQ ID NO: 41, SEQ ID NO: 61, SEQ ID NO: 101, SEQ ID NO: 160, and SEQ ID NO: 230 in a cell.

3. The method of claim 1 or 2, wherein the sample or cell comprises:
(a) a human cell;
(b) a cell from a patient suspected of having cancer;
(c) a cell from a patient showing a sign or symptom of cancer; or
(d) a sample selected from a cell culture, a cell line, a tissue sample, a solid tissue sample, a tissue biopsy, a sample of oral tissue, a sample of gastrointestinal tissue, a sample from an organ, a biological fluid, a blood sample, a blood fraction, a bone marrow sample, a urine sample, and a skin sample.

4. The method of any one of the preceding claims, wherein the cancer is a hematological cancer or solid tumor.

5. The method of any one of the preceding claims, wherein the cancer is selected from breast cancer, melanoma, and chronic lymphocytic leukemia.

6. The method of any one of the preceding claims, comprising
a) contacting the sample or cell with one or more nucleic acid probes capable of specifically hybridizing to the one or more splice variants, and
b) detecting the binding of the one or more probes to the one or more splice variants.

7. The method of claim 6, wherein the one or more nucleic acid probes each comprise a label.

8. The method of claim 6, further comprising contacting the biological sample with one or more additional nucleic acid probes, wherein the additional probes are each labeled with a molecular barcode.

9. The method of any one of the preceding claims, wherein the step of measuring the expression level of one or more splice variants comprises using a nucleic acid quantification assay selected from nucleic acid barcoding, RT-PCR, microarray, nucleic acid sequencing, nanoparticle probes, and in situ hybridization.

10. The method of any one of the preceding claims, wherein the step of measuring the expression level of one or more splice variants comprises measuring the number of copies of the one or more splice variant RNAs in the target cell.

11. An SF3B1-modulating compound for use in a method for treating a patient with a neoplastic disorder, the method comprising administering a therapeutically effective amount of an SF3B1-modulating compound to the patient, wherein a cell from the patient has been determined to:
a) contain a neomorphic mutant SF3B1 protein; and
b) express one or more aberrant splice variants selected from SEQ ID NO: 41, SEQ ID NO: 61, SEQ ID NO: 101, SEQ ID NO: 160, and SEQ ID NO: 230 at a level that is increased or decreased relative to the level in a cell not having the neomorphic mutant SF3B1 protein.

12. The method or SF3B1-modulating compound for use according to any one of the preceding claims, wherein the neomorphic mutant SF3B1 protein is selected from E622D, E622K, E622Q, E622V, Y623C, Y623H, Y623S, R625C, R625G, R625H, R625L, R625P, R625S, N626D, N626H, N626I, N626S, N626Y, H662D, H662L, H662Q, H662R, H662Y, T663I, T663P, K666E, K666M, K666N, K666Q, K666R, K666S, K666T, K700E, V701A, V701F, V701I, I704F, I704N, I704S, I704V, G740E, G740K, G740R, G740V, K741N, K741Q, K741T, G742D, D781E, D781G, and D781N.

13. An SF3B1 modulator for use in a method of treating cancer in a patient, wherein one or more cancer cells from the patient have been tested to measure the amount of one or more splice variants selected from SEQ ID NO: 41, SEQ ID NO: 61, SEQ ID NO: 101, SEQ ID NO: 160, and SEQ ID NO: 230.

14. An SF3B1 modulator for use according to claim 11 or 13, wherein the method comprises identifying a patient having cancer with a neomorphic SF3B1 mutation or identifying a cancerous cell with a SF3B1 neomorphic mutation, by a method of any one of claims 1 to 10 or 12.

15. The SF3B1 modulator for use according to any one of claims 11-14, wherein the SF3B1 modulator is selected from a small molecule, an antibody, an antisense molecule, an aptamer, an RNA molecule, and a peptide.

16. The SF3B1 modulator for use according to claim 15, wherein the SF3B1 modulator is a pladienolide or a pladienolide analog.

17. The SF3B1 modulator for use according to claim 16, wherein the pladienolide analog is selected from pladienolide B, pladienolide D, E7107, a compound of formula 1: 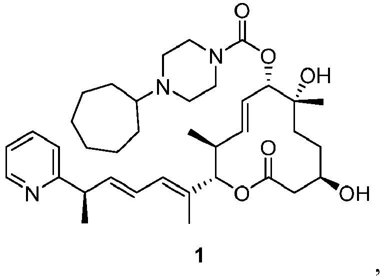 a compound of formula 2: 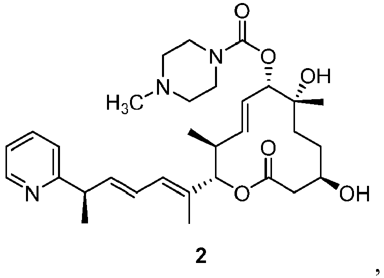 a compound of formula 3: 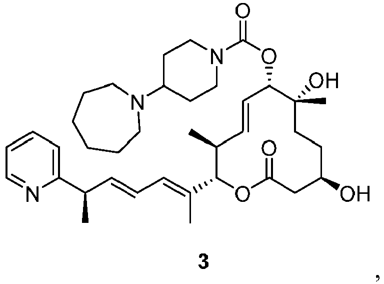 and a compound of formula 4:

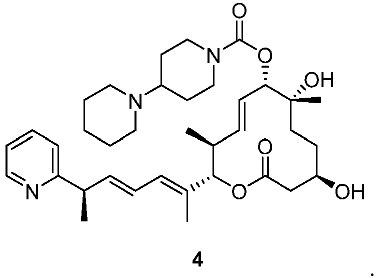

## Patentansprüche

1. Verfahren zur Identifizierung eines an Krebs mit einer neomorphen SF3B1-Mutation leidenden Patienten, umfassend Nachweisen, oder Messen der Menge, einer oder mehrerer Spleißvarianten ausgewählt aus SEQ ID NO: 41, SEQ ID NO: 61, SEQ ID NO: 101, SEQ ID NO: 160 und SEQ ID NO: 230 in einer Probe, die eine oder mehrere Zellen von dem Patienten umfasst.

2. Verfahren zur Identifizierung einer kanzerösen Zelle mit einer neomorphen SF3B1-Mutation, umfassend Nachweisen, oder Messen der Menge, einer oder mehrerer Spleißvarianten ausgewählt aus SEQ ID NO: 41, SEQ ID NO: 61, SEQ ID NO: 101, SEQ ID NO: 160 und SEQ ID NO: 230 in einer Zelle.

3. Verfahren nach Anspruch 1 oder 2, wobei die Probe oder Zelle Folgendes umfasst:
(a) eine menschliche Zelle;
(b) eine Zelle von einem Patienten, bei dem Krebs vermutet wird;
(c) eine Zelle von einem Patienten, der ein Anzeichen oder Symptom von Krebs zeigt; oder
(d) eine Probe ausgewählt aus einer Zellkultur, einer Zelllinie, einer Gewebeprobe, einer festen Gewebeprobe, einer Gewebebiopsie, einer Probe von oralem Gewebe, einer Probe von gastrointestinalem Gewebe, einer Probe von einem Organ, einer biologischen Flüssigkeit, einer Blutprobe, einer Blutfraktion, einer Knochenmarkprobe, einer Urinprobe und einer Hautprobe.

4. Verfahren nach einem der vorhergehenden Ansprüche, wobei es sich bei dem Krebs um einen hämatologischen Krebs oder soliden Tumor handelt.

5. Verfahren nach einem der vorhergehenden Ansprüche, wobei der Krebs ausgewählt ist aus Brustkrebs, Melanom und chronischer lymphatischer Leukämie.

6. Verfahren nach einem der vorhergehenden Ansprüche, umfassend
a) In-Kontakt-Bringen der Probe oder Zelle mit einer oder mehreren Nukleinsäuresonden mit der Fähigkeit zur spezifischen Hybridisierung an die eine oder mehreren Spleißvarianten und
b) Nachweisen der Bindung der einen oder mehreren Sonden an die eine oder mehreren Spleißvarianten.

7. Verfahren nach Anspruch 6, wobei die eine oder mehreren Nukleinsäuresonden jeweils eine Markierung eine Markierung.

8. Verfahren nach Anspruch 6, ferner umfassend In-Kontakt-Bringen der biologischen Probe mit einer oder mehreren zusätzlichen Nukleinsäuresonden, wobei die zusätzlichen Sonden jeweils mit einem molekularen Barcode markiert sind.

9. Verfahren nach einem der vorhergehenden Ansprüche, wobei der Schritt zur Messung des Expressionsniveaus einer oder mehrerer Spleißvarianten Verwenden eines aus Nukleinsäure-Barcodierung, RT-PCR, Mikroarray, Nukleinsäuresequenzierung, Nanopartikelsonden und Insitu-Hybridisierung ausgewählten Testverfahrens zur Nukleinsäurequantifizierung umfasst.

10. Verfahren nach einem der vorhergehenden Ansprüche, wobei der Schritt zur Messung des Expressionsniveaus einer oder mehrerer Spleißvarianten Messen der Anzahl an Kopien der einen oder mehreren Spleißvarianten-RNAs in der Zielzelle umfasst.

11. SF3B1 modulierende Verbindung zur Verwendung bei einem Verfahren zur Behandlung eines Patienten mit einer neoplastischen Erkrankung, wobei das Verfahren Verabreichen einer therapeutisch wirksamen Menge einer SF3B1 modulierenden Verbindung an den Patienten umfasst, wobei bestimmt wurde, dass eine Zelle vom Patienten:
a) ein neomorph mutiertes SF3B1-Protein enthält; und
b) eine oder mehrere aberrante Spleißvarianten ausgewählt aus SEQ ID NO: 41, SEQ ID NO: 61, SEQ ID NO: 101, SEQ ID NO: 160 und SEQ ID NO: 230 auf einem Niveau exprimiert, das relativ zum Niveau in einer Zelle, die das neomorph mutierte SF3B1-Protein nicht aufweist, erhöht oder vermindert ist.

12. Verfahren oder SF3B1 modulierende Verbindung zur Verwendung gemäß einem der vorhergehenden Ansprüche, wobei das neomorph mutierte SF3B1-Protein ausgewählt ist aus E622D, E622K, E622Q, E622V, Y623C, Y623H, Y623S, R625C, R625G, R625H, R625L, R625P, R625S, N626D, N626H, N626I, N626S, N626Y, H662D, H662L, H662Q, H662R, H662Y, T663I, T663P, K666E, K666M, K666N, K666Q, K666R, K666S, K666T, K700E, V701A, V701F, V701I, I704F, I704N, I704S, I704V, G740E, G740K, G740R, G740V, K741N, K741Q, K741T, G742D, D781E, D781G und D781N.

13. SF3B1-Modulator zur Verwendung bei einem Verfahren zur Behandlung von Krebs bei einem Patienten, wobei eine oder mehrere Krebszellen vom Patienten zur Messung der Menge einer oder mehrerer Spleißvarianten ausgewählt aus SEQ ID NO: 41, SEQ ID NO: 61, SEQ ID NO: 101, SEQ ID NO: 160 und SEQ ID NO: 230 getestet wurden.

14. SF3B1-Modulator zur Verwendung gemäß Anspruch 11 oder 13, wobei das Verfahren Identifizieren eines an Krebs mit einer neomorphen SF3B1-Mutation leidenden Patienten oder Identifizieren einer kanzerösen Zelle mit einer neomorphen SF3B1-Mutation mit einem Verfahren nach einem der Ansprüche 1 bis 10 oder 12 umfasst.

15. SF3B1-Modulator zur Verwendung gemäß einem der Ansprüche 11-14, wobei der SF3B1-Modulator ausgewählt ist aus einem kleinen Molekül, einem Antikörper, einem Antisense-Molekül, einem Aptamer, einem RNA-Molekül und einem Peptid.

16. SF3B1-Modulator zur Verwendung gemäß Anspruch 15, wobei es sich bei dem SF3B1-Modulator um ein Pladienolid oder ein Pladienolid-Analog handelt.

17. SF3B1-Modulator zur Verwendung gemäß Anspruch 16, wobei das Pladienolid-Analog ausgewählt ist aus Pladienolid B, Pladienolid D, E7107, einer Verbindung der Formel 1: 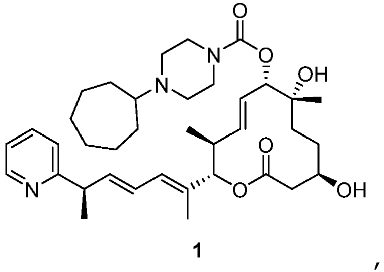 einer Verbindung der Formel 2: 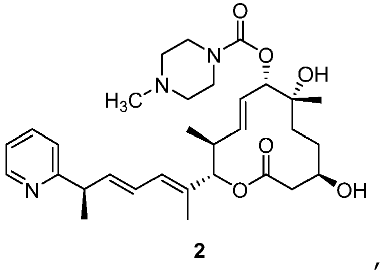 einer Verbindung der Formel 3: 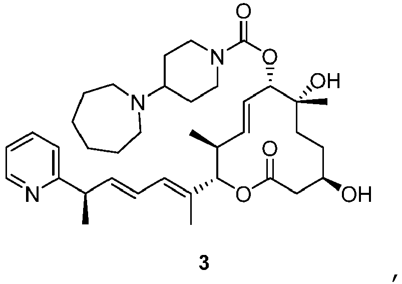 und einer Verbindung der Formel 4:

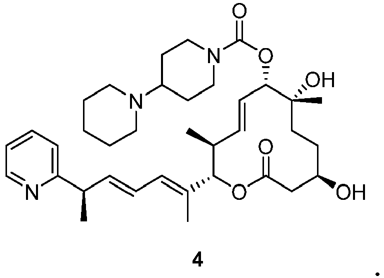

## Revendications

1. Procédé d'identification d'un patient ayant un cancer avec une mutation SF3B1 néomorphe, comprenant une détection d'un ou de plusieurs variant(s) d'épissage, ou une mesure de la quantité de celui-ci/ceux-ci, choisi(s) parmi les SEQ ID n° : 41, SEQ ID n° : 61, SEQ ID n° : 101, SEQ ID n° : 160 et SEQ ID n° : 230, dans un échantillon comprenant une ou plusieurs cellule(s) provenant du patient.

2. Procédé d'identification d'une cellule cancéreuse avec une mutation SF3B1 néomorphe, comprenant une détection dans une cellule d'un ou de plusieurs variant(s) d'épissage, ou une mesure de la quantité de celui-ci/ceux-ci, choisi (s) parmi les SEQ ID n° : 41, SEQ ID n° : 61, SEQ ID n° : 101, SEQ ID n° : 160 et SEQ ID n° : 230.

3. Procédé des revendications 1 ou 2, dans lequel l'échantillon ou la cellule comprend :
(a) une cellule humaine ;
(b) une cellule provenant d'un patient suspecté d'avoir un cancer ;
(c) une cellule provenant d'un patient présentant un signe ou des symptômes d'un cancer ; ou
(d) un échantillon choisi parmi une culture cellulaire, une lignée cellulaire, un échantillon tissulaire, un échantillon de tissu solide, une biopsie tissulaire, un échantillon de tissu oral, un échantillon de tissu gastro-intestinal, un échantillon provenant d'un organe, un liquide biologique, un échantillon sanguin, une fraction sanguine, un échantillon de moelle osseuse, un échantillon d'urine et un échantillon de peau.

4. Procédé de l'une quelconque des revendications précédentes, dans lequel le cancer est un cancer hématologique ou une tumeur solide.

5. Procédé de l'une quelconque des revendications précédentes, dans lequel le cancer est choisi parmi un cancer du sein, un mélanome et une leucémie lymphoïde chronique.

6. Procédé de l'une quelconque des revendications précédentes, comprenant les étapes consistant à
a) mettre en contact l'échantillon ou la cellule avec une ou plusieurs sonde(s) d'acides nucléiques capable(s) de s'hybrider spécifiquement au ou aux plusieurs variant(s) d'épissage, et
b) détecter la liaison de la ou des plusieurs sonde(s) au ou aux plusieurs variant(s) d'épissage.

7. Procédé de la revendication 6, dans lequel la ou les plusieurs sonde(s) d'acides nucléiques comprend/comprennent chacune un marqueur.

8. Procédé de la revendication 6, comprenant en outre une mise en contact de l'échantillon biologique avec une ou plusieurs sonde(s) d'acides nucléiques supplémentaire(s), dans lequel les sondes supplémentaires sont chacune marquées avec un code-barres moléculaire.

9. Procédé de l'une quelconque des revendications précédentes, dans lequel l'étape de mesure du niveau d'expression d'un ou de plusieurs variant(s) d'épissage comprend l'utilisation d'un dosage de quantification d'acides nucléiques choisi parmi un codage à barres des acides nucléiques, une RT-PCR, un micro-arrangement, un séquençage d'acides nucléiques, des sondes nano-particulaires et une hybridation in situ.

10. Procédé de l'une quelconque des revendications précédentes, dans lequel l'étape de mesure du niveau d'expression d'un ou de plusieurs variant(s) d'épissage comprend la mesure du nombre de copies des ARN du ou des plusieurs variant(s) d'épissage dans la cellule cible.

11. Composé de modulation de SF3B1 destiné à être utilisé dans un procédé destiné à traiter un patient ayant un trouble néoplasique, le procédé comprenant une administration au patient d'une quantité efficace, d'un point de vue thérapeutique, d'un composé de modulation de SF3B1, dans lequel on a déterminé qu'une cellule provenant du patient :
a) contient une protéine SF3B1 mutante néomorphe ; et
b) exprime un ou plusieurs variant(s) d'épissage aberrant (s) choisi (s) parmi les SEQ ID n° : 41, SEQ ID n° : 61, SEQ ID n° : 101, SEQ ID n° : 160 et SEQ ID n° : 230 à un niveau qui est accru ou diminué par rapport au niveau dans une cellule n'ayant pas la protéine SF3B1 mutante néomorphe.

12. Procédé ou composé de modulation de SF3B1 destiné à être utilisé selon l'une quelconque des revendications précédentes, dans lequel la protéine SF3B1 mutante néomorphe est choisie parmi les E622D, E622K, E622Q, E622V, Y623C, Y623H, Y623S, R625C, R625G, R625H, R625L, R625P, R625S, N626D, N626H, N626I, N626S, N626Y, H662D, H662L, H662Q, H662R, H662Y, T663I, T663P, K666E, K666M, K666N, K666Q, K666R, K666S, K666T, K700E, V701A, V701F, V701I, I704F, I704N, I704S, I704V, G740E, G740K, G740R, G740V, K741N, K741Q, K741T, G742D, D781E, D781G et D781N.

13. Modulateur de SF3B1 destiné à être utilisé dans un procédé de traitement d'un cancer chez un patient, dans lequel une ou plusieurs cellule(s) cancéreuse(s) provenant du patient a/ont été testée(s) pour mesurer la quantité d'un ou de plusieurs variant(s) d'épissage choisi(s) parmi les SEQ ID n° : 41, SEQ ID n° : 61, SEQ ID n° : 101, SEQ ID n° : 160 et SEQ ID n° : 230.

14. Modulateur de SF3B1 destiné à être utilisé selon les revendications 11 ou 13, dans lequel le procédé comprend une identification d'un patient ayant un cancer avec une mutation SF3B1 néomorphe ou une identification d'une cellule cancéreuse avec une mutation SF3B1 néomorphe, par un procédé de l'une quelconque des revendications 1 à 10 ou 12.

15. Modulateur de SF3B1 destiné à être utilisé selon les revendications 11 à 14, le modulateur de SF3B1 étant choisi parmi une petite molécule, un anticorps, une molécule antisens, un aptamère, une molécule d'ARN et un peptide.

16. Modulateur de SF3B1 destiné à être utilisé selon la revendication 15, le modulateur de SF3B1 étant un pladiénolide ou un analogue de pladiénolide.

17. Modulateur de SF3B1 destiné à être utilisé selon la revendication 16, dans lequel l'analogue de pladiénolide est choisi parmi le pladiénolide B, le pladiénolide D, l'E7107, un composé de formule 1 : 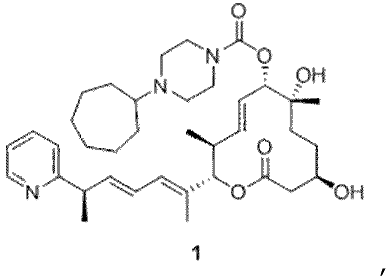 un composé de formule 2 : 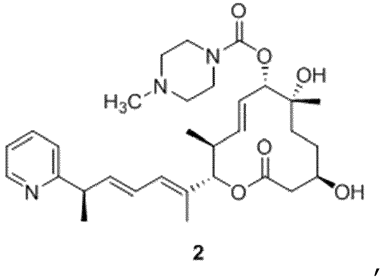 un composé de formule 3 : 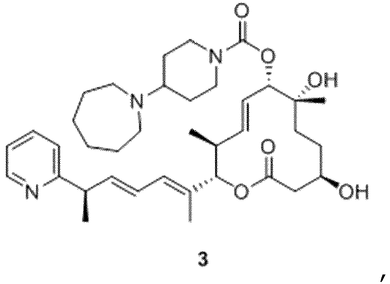 et un composé de formule 4 :


### SEQUENCE LISTING

<110> EISAI R&D MANAGEMENT CO., LTD.
   YU, LIHUA
   LIM, KIAN HUAT
   FEALA, JACOB D.
   BUONAMICI, SILVIA
   MIZUI, YOSHIHARU
   SMITH, PETER G.
   ZHU, PING
   PARK, EUNICE SUN
   SEILER, MICHAEL W.
   FEKKES, MARCO PETER
<120> SPLICE VARIANTS ASSOCIATED WITH NEOMORPHIC SF3B1 MUTANTS
<130> 12636.6-304
<150> 62/212,876
   <151> 2015-09-01
<160> 1200
<170> PatentIn version 3.5
<210> 1
   <211> 40
   <212> DNA
   <213> Homo sapiens
<400> 1
   agcaagtaga agtctataaa atttaccccc agatacagct 40
<210> 2
   <211> 40
   <212> DNA
   <213> Homo sapiens
<400> 2
   agcaagtaga agtctataaa atacagctgg ctgaaataac 40
<210> 3
   <211> 40
   <212> DNA
   <213> Homo sapiens
<400> 3
   cgggccgcat catccgggag agcactgtgt tccagctgcc 40
<210> 4
   <211> 40
   <212> DNA
   <213> Homo sapiens
<400> 4
   cgggccgcat catccgggag ctgcccggtg tccaccctga 40
<210> 5
   <211> 40
   <212> DNA
   <213> Homo sapiens
<400> 5
   ctggagccgg cgggaaggag tgtgctggtt cctctcccca 40
<210> 6
   <211> 40
   <212> DNA
   <213> Homo sapiens
<400> 6
   ctggagccgg cgggaaggag gcaagctgca gcagttcgag 40
<210> 7
   <211> 40
   <212> DNA
   <213> Homo sapiens
<400> 7
   ggcccttttg tcctcactag catttctgtt ctgacaggtt 40
<210> 8
   <211> 40
   <212> DNA
   <213> Homo sapiens
<400> 8
   ggcccttttg tcctcactag gttcttggca tggagctgag 40
<210> 9
   <211> 40
   <212> DNA
   <213> Homo sapiens
<400> 9
   tgggaggagc atgtcaacag agtttccctt ataggactgg 40
<210> 10
   <211> 40
   <212> DNA
   <213> Homo sapiens
<400> 10
   tgggaggagc atgtcaacag gactggctgg acaatggccc 40
<210> 11
   <211> 40
   <212> DNA
   <213> Homo sapiens
<400> 11
   gatggtggat gaacccacag tttttttttt tcaggtatat 40
<210> 12
   <211> 40
   <212> DNA
   <213> Homo sapiens
<400> 12
   gatggtggat gaacccacag gtatatgtcc tcattttcct 40
<210> 13
   <211> 40
   <212> DNA
   <213> Homo sapiens
<400> 13
   tacctctggt tcctgtgcag tcttcgcccc tcttttctta 40
<210> 14
   <211> 40
   <212> DNA
   <213> Homo sapiens
<400> 14
   tacctctggt tcctgtgcag ttctgtggca cttgccctgg 40
<210> 15
   <211> 40
   <212> DNA
   <213> Homo sapiens
<400> 15
   ttggaccgga aaagactttg agtctctttt tgcagatgat 40
<210> 16
   <211> 40
   <212> DNA
   <213> Homo sapiens
<400> 16
   ttggaccgga aaagactttg atgatggatg ccaaccagcg 40
<210> 17
   <211> 40
   <212> DNA
   <213> Homo sapiens
<400> 17
   acccaagcct tgaggtttca tttccccctc ccaggatttc 40
<210> 18
   <211> 40
   <212> DNA
   <213> Homo sapiens
<400> 18
   acccaagcct tgaggtttca gcctgggcag catggccgta 40
<210> 19
   <211> 40
   <212> DNA
   <213> Homo sapiens
<400> 19
   agcattgcta gaagcagcag cttttgcaga tcctgaggta 40
<210> 20
   <211> 40
   <212> DNA
   <213> Homo sapiens
<400> 20
   agcattgcta gaagcagcag gaattggcaa attgtcaact 40
<210> 21
   <211> 40
   <212> DNA
   <213> Homo sapiens
<400> 21
   caagtatatg actgaagaag atcctgaatt ccagcaaaac 40
<210> 22
   <211> 40
   <212> DNA
   <213> Homo sapiens
<400> 22
   caagtatatg actgaagaag gtgagccttt ttctcaagag 40
<210> 23
   <211> 40
   <212> DNA
   <213> Homo sapiens
<400> 23
   tgcagtttgg tcagtctgtg ccttcctcac ccctctcctc 40
<210> 24
   <211> 40
   <212> DNA
   <213> Homo sapiens
<400> 24
   tgcagtttgg tcagtctgtg ggctctgtgg tatatgactg 40
<210> 25
   <211> 40
   <212> DNA
   <213> Homo sapiens
<400> 25
   tctttggaaa atctaatcaa ttttctgcct ataggggaag 40
<210> 26
   <211> 40
   <212> DNA
   <213> Homo sapiens
<400> 26
   tctttggaaa atctaatcaa gggaaggaag atctatgaac 40
<210> 27
   <211> 40
   <212> DNA
   <213> Homo sapiens
<400> 27
   gtatcaaagt gtggactgag atttgtcttc ctttaggatt 40
<210> 28
   <211> 40
   <212> DNA
   <213> Homo sapiens
<400> 28
   gtatcaaagt gtggactgag gattccattg caaagccaca 40
<210> 29
   <211> 40
   <212> DNA
   <213> Homo sapiens
<400> 29
   agaactgcac ctacacacag ccctgttcac aggtgcagac 40
<210> 30
   <211> 40
   <212> DNA
   <213> Homo sapiens
<400> 30
   agaactgcac ctacacacag gtgcagaccc gcagctctga 40
<210> 31
   <211> 40
   <212> DNA
   <213> Homo sapiens
<400> 31
   ggagcagtgc agttgtgaaa tcattacttc tagatgatgc 40
<210> 32
   <211> 40
   <212> DNA
   <213> Homo sapiens
<400> 32
   ggagcagtgc agttgtgaaa gttttgattc atggattcac 40
<210> 33
   <211> 40
   <212> DNA
   <213> Homo sapiens
<400> 33
   ctatttcact ctcccccgaa cctatccagg ttcctcctcc 40
<210> 34
   <211> 40
   <212> DNA
   <213> Homo sapiens
<400> 34
   ctatttcact ctcccccgaa atgagcccat ccagccaatt 40
<210> 35
   <211> 40
   <212> DNA
   <213> Homo sapiens
<400> 35
   tttgcaggga atgggctaca tccccttggt tctctgttac 40
<210> 36
   <211> 40
   <212> DNA
   <213> Homo sapiens
<400> 36
   tttgcaggga atgggctaca taccatctgc cagcatgact 40
<210> 37
   <211> 40
   <212> DNA
   <213> Homo sapiens
<400> 37
   tgaccacgga gtacctgggg cccttttttc tctttccttc 40
<210> 38
   <211> 40
   <212> DNA
   <213> Homo sapiens
<400> 38
   tgaccacgga gtacctgggg atcatgacca acacggggaa 40
<210> 39
   <211> 40
   <212> DNA
   <213> Homo sapiens
<400> 39
   agacctacca gaaggctatg tgtttattaa ttttacagaa 40
<210> 40
   <211> 40
   <212> DNA
   <213> Homo sapiens
<400> 40
   agacctacca gaaggctatg aacagaggac aacgcaacaa 40
<210> 41
   <211> 40
   <212> DNA
   <213> Homo sapiens
<400> 41
   atttggactc gctagcaatg atgtctgttt atttttagag 40
<210> 42
   <211> 40
   <212> DNA
   <213> Homo sapiens
<400> 42
   atttggactc gctagcaatg agcatgacct ctcaatggca 40
<210> 43
   <211> 40
   <212> DNA
   <213> Homo sapiens
<400> 43
   catgtggaat cccaatgccg gcccctgtcc tcctccccca 40
<210> 44
   <211> 40
   <212> DNA
   <213> Homo sapiens
<400> 44
   catgtggaat cccaatgccg ggcagccagg gccaaatcca 40
<210> 45
   <211> 40
   <212> DNA
   <213> Homo sapiens
<400> 45
   ctgggaggtg gcattcaaag ccccaccttt tgtctcccca 40
<210> 46
   <211> 40
   <212> DNA
   <213> Homo sapiens
<400> 46
   ctgggaggtg gcattcaaag gctcttcaga ggtgttcctg 40
<210> 47
   <211> 40
   <212> DNA
   <213> Homo sapiens
<400> 47
   ggatgaccgg gatgcctcag tcactttaca gctgcatcgt 40
<210> 48
   <211> 40
   <212> DNA
   <213> Homo sapiens
<400> 48
   ggatgaccgg gatgcctcag atggggagga tgagaagccc 40
<210> 49
   <211> 40
   <212> DNA
   <213> Homo sapiens
<400> 49
   acatgaaggt ggacggagag gctcccctcc caccccaggt 40
<210> 50
   <211> 40
   <212> DNA
   <213> Homo sapiens
<400> 50
   acatgaaggt ggacggagag gtactgagga caaatcagtt 40
<210> 51
   <211> 40
   <212> DNA
   <213> Homo sapiens
<400> 51
   agagaagtcg tttcattcaa gtcagctaag acacaagcag 40
<210> 52
   <211> 40
   <212> DNA
   <213> Homo sapiens
<400> 52
   agagaagtcg tttcattcaa gttggtgtaa tcagctgggg 40
<210> 53
   <211> 40
   <212> DNA
   <213> Homo sapiens
<400> 53
   tcactcaaac agtaaacgag ttttatcatt tacaggtatg 40
<210> 54
   <211> 40
   <212> DNA
   <213> Homo sapiens
<400> 54
   tcactcaaac agtaaacgag gtatgtgacg cattcccaga 40
<210> 55
   <211> 40
   <212> DNA
   <213> Homo sapiens
<400> 55
   cgatctccca aaaggagaag tctgaccagt cttttctaca 40
<210> 56
   <211> 40
   <212> DNA
   <213> Homo sapiens
<400> 56
   cgatctccca aaaggagaag cccctcccct cgccgagaaa 40
<210> 57
   <211> 40
   <212> DNA
   <213> Homo sapiens
<400> 57
   ttattttaca caatccaaag ccagttgcag ggtctgatga 40
<210> 58
   <211> 40
   <212> DNA
   <213> Homo sapiens
<400> 58
   ttattttaca caatccaaag cttatggtgc attaccagcc 40
<210> 59
   <211> 40
   <212> DNA
   <213> Homo sapiens
<400> 59
   tgcctgtgga catcaccaag cctcgtcctc cccaggtgcc 40
<210> 60
   <211> 40
   <212> DNA
   <213> Homo sapiens
<400> 60
   tgcctgtgga catcaccaag gtgccgcctg cccctgtcaa 40
<210> 61
   <211> 40
   <212> DNA
   <213> Homo sapiens
<400> 61
   agttagaatc caaaccagag tgttgtcttt tctcccccca 40
<210> 62
   <211> 40
   <212> DNA
   <213> Homo sapiens
<400> 62
   agttagaatc caaaccagag ctcctggtac agtttgttca 40
<210> 63
   <211> 40
   <212> DNA
   <213> Homo sapiens
<400> 63
   atatgctgga atggttcctt gtcacaatgc acgacacccg 40
<210> 64
   <211> 40
   <212> DNA
   <213> Homo sapiens
<400> 64
   atatgctgga atggttcctt accgaccgct cgggagctcg 40
<210> 65
   <211> 40
   <212> DNA
   <213> Homo sapiens
<400> 65
   atcagaaatt cgtacaacag gtttctttta aagctcctgg 40
<210> 66
   <211> 40
   <212> DNA
   <213> Homo sapiens
<400> 66
   atcagaaatt cgtacaacag ctcctggagc tttttgatag 40
<210> 67
   <211> 40
   <212> DNA
   <213> Homo sapiens
<400> 67
   aaatgaagaa actcctaaag cctctctctt tctttgttta 40
<210> 68
   <211> 40
   <212> DNA
   <213> Homo sapiens
<400> 68
   aaatgaagaa actcctaaag ataaagtcct gtttatgacc 40
<210> 69
   <211> 40
   <212> DNA
   <213> Homo sapiens
<400> 69
   cataaaattc taacagctaa ttctctttcc tctgtcttca 40
<210> 70
   <211> 40
   <212> DNA
   <213> Homo sapiens
<400> 70
   cataaaattc taacagctaa gcaagcactg agcgaggtga 40
<210> 71
   <211> 40
   <212> DNA
   <213> Homo sapiens
<400> 71
   gcctgccttt gatgccctgg attttgcccg aacaggtcag 40
<210> 72
   <211> 40
   <212> DNA
   <213> Homo sapiens
<400> 72
   gcctgccttt gatgccctgg gtcagttgac tggcggctat 40
<210> 73
   <211> 40
   <212> DNA
   <213> Homo sapiens
<400> 73
   ccaagctggt gtgcgcacag gcctctcttc ccgcccaggc 40
<210> 74
   <211> 40
   <212> DNA
   <213> Homo sapiens
<400> 74
   ccaagctggt gtgcgcacag gcatcatcgg gaagaagcac 40
<210> 75
   <211> 40
   <212> DNA
   <213> Homo sapiens
<400> 75
   ctcctttggg tttgggccag gccccaggtc ccaccacagc 40
<210> 76
   <211> 40
   <212> DNA
   <213> Homo sapiens
<400> 76
   ctcctttggg tttgggccag tgacctggct tgtcctcagc 40
<210> 77
   <211> 40
   <212> DNA
   <213> Homo sapiens
<400> 77
   aatattgctt taccaaacag ggaccccttc cccttcccca 40
<210> 78
   <211> 40
   <212> DNA
   <213> Homo sapiens
<400> 78
   aatattgctt taccaaacag gtcacggagg agtaaagtat 40
<210> 79
   <211> 40
   <212> DNA
   <213> Homo sapiens
<400> 79
   cagttataaa ctctagagtg agtttatttt ccttttacaa 40
<210> 80
   <211> 40
   <212> DNA
   <213> Homo sapiens
<400> 80
   cagttataaa ctctagagtg cttactgcag tgcatggtat 40
<210> 81
   <211> 40
   <212> DNA
   <213> Homo sapiens
<400> 81
   gcctgccccg gaaactcaag atgttcagcg atgcaggtag 40
<210> 82
   <211> 40
   <212> DNA
   <213> Homo sapiens
<400> 82
   gcctgccccg gaaactcaag atggcggtgg gaccccccga 40
<210> 83
   <211> 40
   <212> DNA
   <213> Homo sapiens
<400> 83
   ttcaggaggt ggagcaccag ataatttttt tcctcacaca 40
<210> 84
   <211> 40
   <212> DNA
   <213> Homo sapiens
<400> 84
   ttcaggaggt ggagcaccag ttgcggtctt gtagtaagag 40
<210> 85
   <211> 40
   <212> DNA
   <213> Homo sapiens
<400> 85
   ggatccttca cccgtgtctg tctttgcaga caggttctgt 40
<210> 86
   <211> 40
   <212> DNA
   <213> Homo sapiens
<400> 86
   ggatccttca cccgtgtctg gacccgtgca tctcttccga 40
<210> 87
   <211> 40
   <212> DNA
   <213> Homo sapiens
<400> 87
   atttggatcc tgtgttcctc tttttttctg ttaaagatac 40
<210> 88
   <211> 40
   <212> DNA
   <213> Homo sapiens
<400> 88
   atttggatcc tgtgttcctc atacaactag accaaaacga 40
<210> 89
   <211> 40
   <212> DNA
   <213> Homo sapiens
<400> 89
   agatgtcagg tgggagaaag cctttgattg tcttttcagc 40
<210> 90
   <211> 40
   <212> DNA
   <213> Homo sapiens
<400> 90
   agatgtcagg tgggagaaag ctgttggaga cacagttgca 40
<210> 91
   <211> 40
   <212> DNA
   <213> Homo sapiens
<400> 91
   agaaagagca taaattggaa atattggaca tgggcgtatc 40
<210> 92
   <211> 40
   <212> DNA
   <213> Homo sapiens
<400> 92
   agaaagagca taaattggaa gagtacaagc gcaagctagc 40
<210> 93
   <211> 40
   <212> DNA
   <213> Homo sapiens
<400> 93
   tcagccctct gaactacaaa ggtgtttgtt cacagagatc 40
<210> 94
   <211> 40
   <212> DNA
   <213> Homo sapiens
<400> 94
   tcagccctct gaactacaaa acagaagagc ctgcaagtga 40
<210> 95
   <211> 40
   <212> DNA
   <213> Homo sapiens
<400> 95
   ccggggcctt cgtgagaccg cttgttttct gcaggtgcag 40
<210> 96
   <211> 40
   <212> DNA
   <213> Homo sapiens
<400> 96
   ccggggcctt cgtgagaccg gtgcaggcct ggggtagtct 40
<210> 97
   <211> 40
   <212> DNA
   <213> Homo sapiens
<400> 97
   caagtccatc tctaattcag ggtctgactt gcagccaact 40
<210> 98
   <211> 40
   <212> DNA
   <213> Homo sapiens
<400> 98
   caagtccatc tctaattcag gcaaggccag gccccagccc 40
<210> 99
   <211> 40
   <212> DNA
   <213> Homo sapiens
<400> 99
   caagatagat attatagcag gtggcttttg ttttacagaa 40
<210> 100
   <211> 40
   <212> DNA
   <213> Homo sapiens
<400> 100
   caagatagat attatagcag aacttcgata tgacctgcca 40
<210> 101
   <211> 40
   <212> DNA
   <213> Homo sapiens
<400> 101
   gaaaccaact aaaggcaaag cccattttcc ttctttcgca 40
<210> 102
   <211> 40
   <212> DNA
   <213> Homo sapiens
<400> 102
   gaaaccaact aaaggcaaag gtaaaaaaca tgaagcagat 40
<210> 103
   <211> 40
   <212> DNA
   <213> Homo sapiens
<400> 103
   ggggacagtg aaatttggtg gcaagaatga ggtgacactg 40
<210> 104
   <211> 40
   <212> DNA
   <213> Homo sapiens
<400> 104
   ggggacagtg aaatttggtg ggcagctgct ttcctttgac 40
<210> 105
   <211> 40
   <212> DNA
   <213> Homo sapiens
<400> 105
   ctcagagcca ggctgtagag atgttttcta cctttccaca 40
<210> 106
   <211> 40
   <212> DNA
   <213> Homo sapiens
<400> 106
   ctcagagcca ggctgtagag tccgctctat caagctgaag 40
<210> 107
   <211> 40
   <212> DNA
   <213> Homo sapiens
<400> 107
   gaggagccac actctgacag atacctggct gagagctggc 40
<210> 108
   <211> 40
   <212> DNA
   <213> Homo sapiens
<400> 108
   gaggagccac actctgacag tgagggtgcg gggtcaggcg 40
<210> 109
   <211> 40
   <212> DNA
   <213> Homo sapiens
<400> 109
   actcgcgcct cttccatctg ttttgtcgca gccggaatac 40
<210> 110
   <211> 40
   <212> DNA
   <213> Homo sapiens
<400> 110
   actcgcgcct cttccatctg ccggaataca cctggcgtct 40
<210> 111
   <211> 40
   <212> DNA
   <213> Homo sapiens
<400> 111
   acttccttag tggtttccag gttgccaggg cactgcagct 40
<210> 112
   <211> 40
   <212> DNA
   <213> Homo sapiens
<400> 112
   acttccttag tggtttccag gtggtggtgc tcaccaacac 40
<210> 113
   <211> 40
   <212> DNA
   <213> Homo sapiens
<400> 113
   gtcttgagaa ttggaagcag gtggtggtgc tcaccaacac 40
<210> 114
   <211> 40
   <212> DNA
   <213> Homo sapiens
<400> 114
   tccagagccc acagtcccag ctgcacctta cctgctcccc 40
<210> 115
   <211> 40
   <212> DNA
   <213> Homo sapiens
<400> 115
   tccagagccc acagtcccag gggtccatga tgccgagctg 40
<210> 116
   <211> 40
   <212> DNA
   <213> Homo sapiens
<400> 116
   ccaagttttg tgaaagaaag tgtatgtttt gttcacgaca 40
<210> 117
   <211> 40
   <212> DNA
   <213> Homo sapiens
<400> 117
   ccaagttttg tgaaagaaag aacatcagat accaaaccta 40
<210> 118
   <211> 40
   <212> DNA
   <213> Homo sapiens
<400> 118
   tcttcacaga acacactcaa gtgcttgtag gtcttggtgc 40
<210> 119
   <211> 40
   <212> DNA
   <213> Homo sapiens
<400> 119
   tcttcacaga acacactcaa ccccctgcct gggatgcgcc 40
<210> 120
   <211> 40
   <212> DNA
   <213> Homo sapiens
<400> 120
   gagaagctca cgattaccag gcacctcatt gtgaacatgc 40
<210> 121
   <211> 40
   <212> DNA
   <213> Homo sapiens
<400> 121
   tcttggagga gccagtacag gcacctcatt gtgaacatgc 40
<210> 122
   <211> 40
   <212> DNA
   <213> Homo sapiens
<400> 122
   gtggggggcc attgctgcat tttgtatttt ccaggtacag 40
<210> 123
   <211> 40
   <212> DNA
   <213> Homo sapiens
<400> 123
   gtggggggcc attgctgcat gtacagtctt tgcccgctgc 40
<210> 124
   <211> 40
   <212> DNA
   <213> Homo sapiens
<400> 124
   tactgaaatg tgatgaacat atccaggtaa tcgagagacc 40
<210> 125
   <211> 40
   <212> DNA
   <213> Homo sapiens
<400> 125
   tactgaaatg tgatgaacat atccagaagc ttggaagctg 40
<210> 126
   <211> 40
   <212> DNA
   <213> Homo sapiens
<400> 126
   gaatctctta tcattgatgg ttcctgttca gattgtgatg 40
<210> 127
   <211> 40
   <212> DNA
   <213> Homo sapiens
<400> 127
   gaatctctta tcattgatgg tttatttatg gagattctta 40
<210> 128
   <211> 40
   <212> DNA
   <213> Homo sapiens
<400> 128
   ctccatgctc agctctctgg tttctttcag ggcctgccat 40
<210> 129
   <211> 40
   <212> DNA
   <213> Homo sapiens
<400> 129
   ctccatgctc agctctctgg ggaaggtgaa gaaggagctg 40
<210> 130
   <211> 40
   <212> DNA
   <213> Homo sapiens
<400> 130
   cttggagctg acgccgacgg ggaactgaca agatcacatt 40
<210> 131
   <211> 40
   <212> DNA
   <213> Homo sapiens
<400> 131
   cttggagctg acgccgacgg tttattgcag ggaactgaca 40
<210> 132
   <211> 40
   <212> DNA
   <213> Homo sapiens
<400> 132
   tccagcctgg gcgacagaag tcttgtctca agaagaaaac 40
<210> 133
   <211> 40
   <212> DNA
   <213> Homo sapiens
<400> 133
   ctatcaaaag aggatatgtt tcttgtctca agaagaaaac 40
<210> 134
   <211> 40
   <212> DNA
   <213> Homo sapiens
<400> 134
   tgcggagcaa gagtggacat cgtttgtttc ccatttctcc 40
<210> 135
   <211> 40
   <212> DNA
   <213> Homo sapiens
<400> 135
   tgcggagcaa gagtggacat aaactttaca ttttcctgtt 40
<210> 136
   <211> 40
   <212> DNA
   <213> Homo sapiens
<400> 136
   agtccagccc cagcatggca cctctcccca ctcctaggtc 40
<210> 137
   <211> 40
   <212> DNA
   <213> Homo sapiens
<400> 137
   agtccagccc cagcatggca gtcctgtaca tccaggcctt 40
<210> 138
   <211> 40
   <212> DNA
   <213> Homo sapiens
<400> 138
   caagcaggtc caaagagaga ttttggtaaa cagagctcca 40
<210> 139
   <211> 40
   <212> DNA
   <213> Homo sapiens
<400> 139
   caagcaggtc caaagagaga agctccaaga gtcaggatcg 40
<210> 140
   <211> 40
   <212> DNA
   <213> Homo sapiens
<400> 140
   ctctctccaa cctgcattct catctcgccc acagttggat 40
<210> 141
   <211> 40
   <212> DNA
   <213> Homo sapiens
<400> 141
   ctctctccaa cctgcattct ttggatcgat caacccggga 40
<210> 142
   <211> 40
   <212> DNA
   <213> Homo sapiens
<400> 142
   caccacgccg aggccacgag acattgatgg aagcagaaac 40
<210> 143
   <211> 40
   <212> DNA
   <213> Homo sapiens
<400> 143
   caccacgccg aggccacgag tatttcatag acattgatgg 40
<210> 144
   <211> 40
   <212> DNA
   <213> Homo sapiens
<400> 144
   gcctcactga gcaaccaaga gtagtgactt gtcaggagga 40
<210> 145
   <211> 40
   <212> DNA
   <213> Homo sapiens
<400> 145
   gcctcactga gcaaccaaga gtgtcagttg tacccgaggc 40
<210> 146
   <211> 40
   <212> DNA
   <213> Homo sapiens
<400> 146
   gggagatgga taccgacttg ctcaatttca gtgatcaacg 40
<210> 147
   <211> 40
   <212> DNA
   <213> Homo sapiens
<400> 147
   gggagatgga taccgacttg tgatcaacga tgggaagctg 40
<210> 148
   <211> 40
   <212> DNA
   <213> Homo sapiens
<400> 148
   aggatgtggc tggcacagaa gtgtcatcag gtccctgcag 40
<210> 149
   <211> 40
   <212> DNA
   <213> Homo sapiens
<400> 149
   aggatgtggc tggcacagaa atgagtcagt ctgacagtgg 40
<210> 150
   <211> 40
   <212> DNA
   <213> Homo sapiens
<400> 150
   ttctccagga ccttgccaga ccttttctat agggaatcaa 40
<210> 151
   <211> 40
   <212> DNA
   <213> Homo sapiens
<400> 151
   ttctccagga ccttgccaga ggaatcaaag actccatctg 40
<210> 152
   <211> 40
   <212> DNA
   <213> Homo sapiens
<400> 152
   agctgaaatt tccagtaaag gggggtttta ttcttctttt 40
<210> 153
   <211> 40
   <212> DNA
   <213> Homo sapiens
<400> 153
   agctgaaatt tccagtaaag cctggagatt tgaaaaagag 40
<210> 154
   <211> 40
   <212> DNA
   <213> Homo sapiens
<400> 154
   gatgtcactg tgactatcaa gggccgtctt tcttctaggt 40
<210> 155
   <211> 40
   <212> DNA
   <213> Homo sapiens
<400> 155
   gatgtcactg tgactatcaa gtcttccatc gacagtgaac 40
<210> 156
   <211> 40
   <212> DNA
   <213> Homo sapiens
<400> 156
   tatccattcc tgagttacag tataaacttc cttctcatgc 40
<210> 157
   <211> 40
   <212> DNA
   <213> Homo sapiens
<400> 157
   tatccattcc tgagttacag tgtcttaata ttgaaaatga 40
<210> 158
   <211> 40
   <212> DNA
   <213> Homo sapiens
<400> 158
   tacaagagct gggtggagag ggtcccaaca ggtattatcg 40
<210> 159
   <211> 40
   <212> DNA
   <213> Homo sapiens
<400> 159
   tacaagagct gggtggagag gtattatcga gacattgcaa 40
<210> 160
   <211> 40
   <212> DNA
   <213> Homo sapiens
<400> 160
   agccatttat ttgtcccgtg ggaaccaatc tgcccttttg 40
<210> 161
   <211> 40
   <212> DNA
   <213> Homo sapiens
<400> 161
   agccatttat ttgtcccgtg ggtttttttc cagggaacca 40
<210> 162
   <211> 40
   <212> DNA
   <213> Homo sapiens
<400> 162
   agttacaacg aacacctcag tgactctttt acaggaggca 40
<210> 163
   <211> 40
   <212> DNA
   <213> Homo sapiens
<400> 163
   agttacaacg aacacctcag gaggcaataa cagatggctt 40
<210> 164
   <211> 40
   <212> DNA
   <213> Homo sapiens
<400> 164
   tcacacagga taatttgaaa gtgtcagttg tacccgaggc 40
<210> 165
   <211> 40
   <212> DNA
   <213> Homo sapiens
<400> 165
   cggcgcgggc aacctggcgg cccccatttc aggtctgaag 40
<210> 166
   <211> 40
   <212> DNA
   <213> Homo sapiens
<400> 166
   cggcgcgggc aacctggcgg gtctgaaggg gcgtctcgat 40
<210> 167
   <211> 40
   <212> DNA
   <213> Homo sapiens
<400> 167
   ccaccgccat cgacgtgcag tacctctttt taccaccagg 40
<210> 168
   <211> 40
   <212> DNA
   <213> Homo sapiens
<400> 168
   ccaccgccat cgacgtgcag gtggggctcc tgtacgaaga 40
<210> 169
   <211> 40
   <212> DNA
   <213> Homo sapiens
<400> 169
   ctatgggctc actcctctgg tcctcctgtt gcagttcgtc 40
<210> 170
   <211> 40
   <212> DNA
   <213> Homo sapiens
<400> 170
   ctatgggctc actcctctgg ttcgtcgcct gcagcttcga 40
<210> 171
   <211> 40
   <212> DNA
   <213> Homo sapiens
<400> 171
   tatctctggg aaaaaacaca tttctttttt tgcaggggac 40
<210> 172
   <211> 40
   <212> DNA
   <213> Homo sapiens
<400> 172
   tatctctggg aaaaaacaca gggacctgat ggggtgcagc 40
<210> 173
   <211> 40
   <212> DNA
   <213> Homo sapiens
<400> 173
   tcatccagag cccagagcag gggatgtctg accagatgca 40
<210> 174
   <211> 40
   <212> DNA
   <213> Homo sapiens
<400> 174
   tcatccagag cccagagcag atgcaagtgc tgctggacca 40
<210> 175
   <211> 40
   <212> DNA
   <213> Homo sapiens
<400> 175
   ccaaggactg cactgtgaag gcccccgccc cgcgacctgg 40
<210> 176
   <211> 40
   <212> DNA
   <213> Homo sapiens
<400> 176
   ccaaggactg cactgtgaag atctggagca acgacctgac 40
<210> 177
   <211> 40
   <212> DNA
   <213> Homo sapiens
<400> 177
   cccgagctca gagagtaaat tctccttaca gacactgaaa 40
<210> 178
   <211> 40
   <212> DNA
   <213> Homo sapiens
<400> 178
   cccgagctca gagagtaaat atgagatcgc ctctgtccca 40
<210> 179
   <211> 40
   <212> DNA
   <213> Homo sapiens
<400> 179
   gtgcttggag ccctgtgcag actttccgca gggtgtgcgc 40
<210> 180
   <211> 40
   <212> DNA
   <213> Homo sapiens
<400> 180
   gtgcttggag ccctgtgcag cctggtgaca gactttccgc 40
<210> 181
   <211> 40
   <212> DNA
   <213> Homo sapiens
<400> 181
   gctggacacg ctgaccaagg catcacttag gagctgctac 40
<210> 182
   <211> 40
   <212> DNA
   <213> Homo sapiens
<400> 182
   gctggacacg ctgaccaagg tgttggtagc cttatatgaa 40
<210> 183
   <211> 40
   <212> DNA
   <213> Homo sapiens
<400> 183
   cccctgagat gaagaaagag ctccctgttg acagctgcct 40
<210> 184
   <211> 40
   <212> DNA
   <213> Homo sapiens
<400> 184
   cccctgagat gaagaaagag ctcctgagca gcctgactga 40
<210> 185
   <211> 40
   <212> DNA
   <213> Homo sapiens
<400> 185
   ctgaactttg ggcctgaatg atgtgtttgg accccgaata 40
<210> 186
   <211> 40
   <212> DNA
   <213> Homo sapiens
<400> 186
   ctgaactttg ggcctgaatg gctccgagct ctgtccagtg 40
<210> 187
   <211> 40
   <212> DNA
   <213> Homo sapiens
<400> 187
   agatcgcctg gctcagtcag tttttctctc tagacatggc 40
<210> 188
   <211> 40
   <212> DNA
   <213> Homo sapiens
<400> 188
   agatcgcctg gctcagtcag acatggccaa acgtgtagcc 40
<210> 189
   <211> 40
   <212> DNA
   <213> Homo sapiens
<400> 189
   ggaggtggac ctgagtgaac aatttctccc ctctttttag 40
<210> 190
   <211> 40
   <212> DNA
   <213> Homo sapiens
<400> 190
   ggaggtggac ctgagtgaac cacccaactg gtcagctaac 40
<210> 191
   <211> 40
   <212> DNA
   <213> Homo sapiens
<400> 191
   tacagatggt aaaatgcaag tttgattttt catatccagg 40
<210> 192
   <211> 40
   <212> DNA
   <213> Homo sapiens
<400> 192
   tacagatggt aaaatgcaag gaattgccac aagcagtctg 40
<210> 193
   <211> 40
   <212> DNA
   <213> Homo sapiens
<400> 193
   ccctgctcat cacctacggg tctgtcccag gctctctggg 40
<210> 194
   <211> 40
   <212> DNA
   <213> Homo sapiens
<400> 194
   ccctgctcat cacctacggg ccctatgcca tcaatgggaa 40
<210> 195
   <211> 40
   <212> DNA
   <213> Homo sapiens
<400> 195
   ggctcccatt ctggttaaag agtgttctca tttccaatag 40
<210> 196
   <211> 40
   <212> DNA
   <213> Homo sapiens
<400> 196
   ggctcccatt ctggttaaag gccagtctgc catccatcca 40
<210> 197
   <211> 40
   <212> DNA
   <213> Homo sapiens
<400> 197
   ctgcacttat aaatattcag tgttccacct tgcagacccg 40
<210> 198
   <211> 40
   <212> DNA
   <213> Homo sapiens
<400> 198
   ctgcacttat aaatattcag acccgagggg aagctgcagc 40
<210> 199
   <211> 40
   <212> DNA
   <213> Homo sapiens
<400> 199
   cgctggcacc atgaacccag tatttccagg accaagtgag 40
<210> 200
   <211> 40
   <212> DNA
   <213> Homo sapiens
<400> 200
   cgctggcacc atgaacccag agagcagtat ctttattgag 40
<210> 201
   <211> 40
   <212> DNA
   <213> Homo sapiens
<400> 201
   ccctagtctg attcctttag gttggtgtaa tcagctgggg 40
<210> 202
   <211> 40
   <212> DNA
   <213> Homo sapiens
<400> 202
   ttccccatca acatcaaaag ttttgttgtc tgcagttcca 40
<210> 203
   <211> 40
   <212> DNA
   <213> Homo sapiens
<400> 203
   ttccccatca acatcaaaag ttccaatggt ggcagtaaga 40
<210> 204
   <211> 40
   <212> DNA
   <213> Homo sapiens
<400> 204
   ccagctgcat tgcaagttcg gactgtgagt ccctgcaggc 40
<210> 205
   <211> 40
   <212> DNA
   <213> Homo sapiens
<400> 205
   ccagctgcat tgcaagttcg gggtgcggaa gactcacaac 40
<210> 206
   <211> 40
   <212> DNA
   <213> Homo sapiens
<400> 206
   ggccagcccc cttctccacg gccttgccca ctaggtaacc 40
<210> 207
   <211> 40
   <212> DNA
   <213> Homo sapiens
<400> 207
   ggccagcccc cttctccacg gtaaccatgt gcgaccgaaa 40
<210> 208
   <211> 40
   <212> DNA
   <213> Homo sapiens
<400> 208
   cgctctccgc cttccagaag gggtctcctt atgccaggga 40
<210> 209
   <211> 40
   <212> DNA
   <213> Homo sapiens
<400> 209
   agggagacgt tccctgcctg gggtctcctt atgccaggga 40
<210> 210
   <211> 40
   <212> DNA
   <213> Homo sapiens
<400> 210
   ttggaagcga atcccccaag tcctttgttc ttttgcagtg 40
<210> 211
   <211> 40
   <212> DNA
   <213> Homo sapiens
<400> 211
   ttggaagcga atcccccaag tgatgtatat ctctcatcaa 40
<210> 212
   <211> 40
   <212> DNA
   <213> Homo sapiens
<400> 212
   ctacggcggt gccctcctca cccccttttc atcccccgcc 40
<210> 213
   <211> 40
   <212> DNA
   <213> Homo sapiens
<400> 213
   ctacggcggt gccctcctca gcatctccct gatcatgtgg 40
<210> 214
   <211> 40
   <212> DNA
   <213> Homo sapiens
<400> 214
   cctggtcgca gttcaacaag atgaggaatc tgatgctcag 40
<210> 215
   <211> 40
   <212> DNA
   <213> Homo sapiens
<400> 215
   cctggtcgca gttcaacaag gagatcctgc tgggccgtgg 40
<210> 216
   <211> 40
   <212> DNA
   <213> Homo sapiens
<400> 216
   caccaagcag aggcttccag tctgtctgcc ctttctgtag 40
<210> 217
   <211> 40
   <212> DNA
   <213> Homo sapiens
<400> 217
   caccaagcag aggcttccag gccagaagcc ttttaaaagg 40
<210> 218
   <211> 40
   <212> DNA
   <213> Homo sapiens
<400> 218
   gggactcccc caaagacaag cttttctttc agtaaatgta 40
<210> 219
   <211> 40
   <212> DNA
   <213> Homo sapiens
<400> 219
   gggactcccc caaagacaag gtcccatttt cagtgcccaa 40
<210> 220
   <211> 40
   <212> DNA
   <213> Homo sapiens
<400> 220
   gcactgctgt tcaacctcgg cttctccctt cctctcaccc 40
<210> 221
   <211> 40
   <212> DNA
   <213> Homo sapiens
<400> 221
   gcactgctgt tcaacctcgg gggcaagtat agcgcatttg 40
<210> 222
   <211> 40
   <212> DNA
   <213> Homo sapiens
<400> 222
   acgagaccat tgccttcaag gagccctctc tgtcccccgc 40
<210> 223
   <211> 40
   <212> DNA
   <213> Homo sapiens
<400> 223
   acgagaccat tgccttcaag gtgccgagca gagagatcga 40
<210> 224
   <211> 40
   <212> DNA
   <213> Homo sapiens
<400> 224
   aagatgtccc tgtgaggatt gtgtgtttgt ttccacaggc 40
<210> 225
   <211> 40
   <212> DNA
   <213> Homo sapiens
<400> 225
   aagatgtccc tgtgaggatt gcactgggtg caagttcctg 40
<210> 226
   <211> 40
   <212> DNA
   <213> Homo sapiens
<400> 226
   agagaagtcg tttcattcaa tctgattcct ttaggtcagc 40
<210> 227
   <211> 40
   <212> DNA
   <213> Homo sapiens
<400> 227
   agcccagcag ttccgaaatg tctcccttct ccagcgcccc 40
<210> 228
   <211> 40
   <212> DNA
   <213> Homo sapiens
<400> 228
   agcccagcag ttccgaaatg cgcccccatt cctggaggac 40
<210> 229
   <211> 40
   <212> DNA
   <213> Homo sapiens
<400> 229
   ccctcccccg gctcctgtcg gcctgggcag catggccgta 40
<210> 230
   <211> 40
   <212> DNA
   <213> Homo sapiens
<400> 230
   tgattccaag caaaaaccag ccttccccta ggtcttcaga 40
<210> 231
   <211> 40
   <212> DNA
   <213> Homo sapiens
<400> 231
   tgattccaag caaaaaccag gctccatcta ctctttgaag 40
<210> 232
   <211> 40
   <212> DNA
   <213> Homo sapiens
<400> 232
   caagtccatc tctaattcag ccaactctca aggcaaggcc 40
<210> 233
   <211> 40
   <212> DNA
   <213> Homo sapiens
<400> 233
   ctatcaaaag aggatatgtt cattttagga ggccaaggca 40
<210> 234
   <211> 40
   <212> DNA
   <213> Homo sapiens
<400> 234
   gtcttccaat ggcccctcag ccttttctct aggaaatgat 40
<210> 235
   <211> 40
   <212> DNA
   <213> Homo sapiens
<400> 235
   gtcttccaat ggcccctcag gaaatgatac acctgaagaa 40
<210> 236
   <211> 40
   <212> DNA
   <213> Homo sapiens
<400> 236
   gcacctcccc gggacgcctg cccttgtctg gaaagaagtt 40
<210> 237
   <211> 40
   <212> DNA
   <213> Homo sapiens
<400> 237
   gcacctcccc gggacgcctg tcaccggact ttgctgagga 40
<210> 238
   <211> 40
   <212> DNA
   <213> Homo sapiens
<400> 238
   tggaccccag accacaccgg aagaaatgag ccagaagtga 40
<210> 239
   <211> 40
   <212> DNA
   <213> Homo sapiens
<400> 239
   gtcccggaac cacatgcacg aagaaatgag ccagaagtga 40
<210> 240
   <211> 40
   <212> DNA
   <213> Homo sapiens
<400> 240
   tctgtgttcc catcgcacag gaatcctacg ccaacgtgaa 40
<210> 241
   <211> 40
   <212> DNA
   <213> Homo sapiens
<400> 241
   tgtatgacgt cactgaccag gaatcctacg ccaacgtgaa 40
<210> 242
   <211> 40
   <212> DNA
   <213> Homo sapiens
<400> 242
   ggaatatgat cccaccctcg tacttctcaa agaggatggc 40
<210> 243
   <211> 40
   <212> DNA
   <213> Homo sapiens
<400> 243
   ggaatatgat cccaccctcg aatcaaccta ccgacaccaa 40
<210> 244
   <211> 40
   <212> DNA
   <213> Homo sapiens
<400> 244
   gaactggcac cgacagacag tgtcccctcc ctccccagat 40
<210> 245
   <211> 40
   <212> DNA
   <213> Homo sapiens
<400> 245
   gaactggcac cgacagacag atcctgtttc tggaccttgg 40
<210> 246
   <211> 40
   <212> DNA
   <213> Homo sapiens
<400> 246
   tgatgaagac ctttccccag atctcttagg tgaagacatg 40
<210> 247
   <211> 40
   <212> DNA
   <213> Homo sapiens
<400> 247
   tgatgaagac ctttccccag gccccgagca ttcctctgat 40
<210> 248
   <211> 40
   <212> DNA
   <213> Homo sapiens
<400> 248
   ccaggccgac atggagagca gccccaccca caggcaagga 40
<210> 249
   <211> 40
   <212> DNA
   <213> Homo sapiens
<400> 249
   ccaggccgac atggagagca gcaaggagcc cggcctgttt 40
<210> 250
   <211> 40
   <212> DNA
   <213> Homo sapiens
<400> 250
   acatgaaggt ggacggagag ttctctgtga ccagacatga 40
<210> 251
   <211> 40
   <212> DNA
   <213> Homo sapiens
<400> 251
   ttcgtccata tgtgcataag cttcttctct tttctctttt 40
<210> 252
   <211> 40
   <212> DNA
   <213> Homo sapiens
<400> 252
   ttcgtccata tgtgcataag atcctcgtgg tcattgaacc 40
<210> 253
   <211> 40
   <212> DNA
   <213> Homo sapiens
<400> 253
   agggatggcc agtggtagtg ggtctccaac tgaattcctt 40
<210> 254
   <211> 40
   <212> DNA
   <213> Homo sapiens
<400> 254
   agaagggagc gatactacag ggtctccaac tgaattcctt 40
<210> 255
   <211> 40
   <212> DNA
   <213> Homo sapiens
<400> 255
   ccaatgtggt tcaaaacaca ttatctcatc tgcagggtaa 40
<210> 256
   <211> 40
   <212> DNA
   <213> Homo sapiens
<400> 256
   ccaatgtggt tcaaaacaca ggtaaaagtg tcttaactgg 40
<210> 257
   <211> 40
   <212> DNA
   <213> Homo sapiens
<400> 257
   ccattgatgc aaacgcagca atggagtttc gctcctgttg 40
<210> 258
   <211> 40
   <212> DNA
   <213> Homo sapiens
<400> 258
   ccattgatgc aaacgcagca gaacttgcca catcagactc 40
<210> 259
   <211> 40
   <212> DNA
   <213> Homo sapiens
<400> 259
   gctgcatctg gaggtcctgg gaagcagaat ctggtaatat 40
<210> 260
   <211> 40
   <212> DNA
   <213> Homo sapiens
<400> 260
   cagtgttagt gaatgactat gaagcagaat ctggtaatat 40
<210> 261
   <211> 40
   <212> DNA
   <213> Homo sapiens
<400> 261
   acaaggacac agaaaacaag ccttcccaca caggccctgc 40
<210> 262
   <211> 40
   <212> DNA
   <213> Homo sapiens
<400> 262
   acaaggacac agaaaacaag ctggagcacc gctgcacctc 40
<210> 263
   <211> 40
   <212> DNA
   <213> Homo sapiens
<400> 263
   agctcggacc aagcgctcag ttttaaaatt gctatagctt 40
<210> 264
   <211> 40
   <212> DNA
   <213> Homo sapiens
<400> 264
   agctcggacc aagcgctcag cttagcctgc gacgcttatg 40
<210> 265
   <211> 40
   <212> DNA
   <213> Homo sapiens
<400> 265
   agggggctct ttatataatg tttgtgcctt tctttcgcag 40
<210> 266
   <211> 40
   <212> DNA
   <213> Homo sapiens
<400> 266
   agggggctct ttatataatg tgctgcatgg tgctgaacca 40
<210> 267
   <211> 40
   <212> DNA
   <213> Homo sapiens
<400> 267
   gcccccaact gagaagctgg gctggagtgc tgtggcacaa 40
<210> 268
   <211> 40
   <212> DNA
   <213> Homo sapiens
<400> 268
   gcccccaact gagaagctgg tgcccttggt gtggtggaag 40
<210> 269
   <211> 40
   <212> DNA
   <213> Homo sapiens
<400> 269
   gaacgagatc tcatcccact aactacaaag agctggagct 40
<210> 270
   <211> 40
   <212> DNA
   <213> Homo sapiens
<400> 270
   agtatcagaa ggacaaaaag aactacaaag agctggagct 40
<210> 271
   <211> 40
   <212> DNA
   <213> Homo sapiens
<400> 271
   tgaaggtcca gggcatggag cctgtctcct ggcagtgtct 40
<210> 272
   <211> 40
   <212> DNA
   <213> Homo sapiens
<400> 272
   tgaaggtcca gggcatggag tgtctctatg gctgctacgt 40
<210> 273
   <211> 40
   <212> DNA
   <213> Homo sapiens
<400> 273
   ggcggccgcg ccggctccag gaaatggcaa ctgctgacag 40
<210> 274
   <211> 40
   <212> DNA
   <213> Homo sapiens
<400> 274
   ggcggccgcg ccggctccag ggccatgaag cccccaggag 40
<210> 275
   <211> 40
   <212> DNA
   <213> Homo sapiens
<400> 275
   ccttccagct acatcgaaac gcatgaggat gttgtatttc 40
<210> 276
   <211> 40
   <212> DNA
   <213> Homo sapiens
<400> 276
   ccttccagct acatcgaaac tttacctaaa gcagtaaaaa 40
<210> 277
   <211> 40
   <212> DNA
   <213> Homo sapiens
<400> 277
   cttttctctt ctttttatag gttgaacaaa tcctggcaga 40
<210> 278
   <211> 40
   <212> DNA
   <213> Homo sapiens
<400> 278
   gatgtgatga actatcttcg gttgaacaaa tcctggcaga 40
<210> 279
   <211> 40
   <212> DNA
   <213> Homo sapiens
<400> 279
   gcactgggca ttcagaaaag tctctcttcc tcacccctgc 40
<210> 280
   <211> 40
   <212> DNA
   <213> Homo sapiens
<400> 280
   gcactgggca ttcagaaaag gttctccccg gaggtgctgg 40
<210> 281
   <211> 40
   <212> DNA
   <213> Homo sapiens
<400> 281
   ctgtcacagg ggagtttacg tcttgcatgt ctctcttaca 40
<210> 282
   <211> 40
   <212> DNA
   <213> Homo sapiens
<400> 282
   ctgtcacagg ggagtttacg ggaatgccag agcagtgggc 40
<210> 283
   <211> 40
   <212> DNA
   <213> Homo sapiens
<400> 283
   gggtgcaaaa gatcctgcag ccattccagg ttgctgaggt 40
<210> 284
   <211> 40
   <212> DNA
   <213> Homo sapiens
<400> 284
   gggtgcaaaa gatcctgcag gactacaaat ccctccagga 40
<210> 285
   <211> 40
   <212> DNA
   <213> Homo sapiens
<400> 285
   ggcaccccaa aagatggcag atcagtctct ccctgttctc 40
<210> 286
   <211> 40
   <212> DNA
   <213> Homo sapiens
<400> 286
   ggcaccccaa aagatggcag gtgcgagccc gaccaaggat 40
<210> 287
   <211> 40
   <212> DNA
   <213> Homo sapiens
<400> 287
   gcatctcagc ccaagagaag tttctttgca ggttatattc 40
<210> 288
   <211> 40
   <212> DNA
   <213> Homo sapiens
<400> 288
   gcatctcagc ccaagagaag gttatattcc cagaggatg 40
<210> 289
   <211> 40
   <212> DNA
   <213> Homo sapiens
<400> 289
   cttgccttcc catcctcctg caaacacctg ccacctttct 40
<210> 290
   <211> 40
   <212> DNA
   <213> Homo sapiens
<400> 290
   cttgccttcc catcctcctg aacttccagg tcctgagtca 40
<210> 291
   <211> 40
   <212> DNA
   <213> Homo sapiens
<400> 291
   ctacacagag ctgcagcaag gtgtgcaccc agctgcaggt 40
<210> 292
   <211> 40
   <212> DNA
   <213> Homo sapiens
<400> 292
   ctacacagag ctgcagcaag ctctgtccca aatgggctac 40
<210> 293
   <211> 40
   <212> DNA
   <213> Homo sapiens
<400> 293
   acctgttacc actttcaaaa tttctgtgct aaacagtgtt 40
<210> 294
   <211> 40
   <212> DNA
   <213> Homo sapiens
<400> 294
   acctgttacc actttcaaaa atctacagac agtcaatgtg 40
<210> 295
   <211> 40
   <212> DNA
   <213> Homo sapiens
<400> 295
   agacaaggga ttggtggaaa cattttattt tacagaattg 40
<210> 296
   <211> 40
   <212> DNA
   <213> Homo sapiens
<400> 296
   agacaaggga ttggtggaaa aattgacagc gtatgccatg 40
<210> 297
   <211> 40
   <212> DNA
   <213> Homo sapiens
<400> 297
   caacgagaac aagctatcag ttacttttac cccacagggc 40
<210> 298
   <211> 40
   <212> DNA
   <213> Homo sapiens
<400> 298
   caacgagaac aagctatcag ggctgctaag gaagcaaaaa 40
<210> 299
   <211> 40
   <212> DNA
   <213> Homo sapiens
<400> 299
   tctatatccc ctctaagacg cacttctttc ccctctgtag 40
<210> 300
   <211> 40
   <212> DNA
   <213> Homo sapiens
<400> 300
   tctatatccc ctctaagacg gacctgggtg cagccgcagg 40
<210> 301
   <211> 40
   <212> DNA
   <213> Homo sapiens
<400> 301
   tggagccagt tactgggcag gtgtgttttt gtgacagtca 40
<210> 302
   <211> 40
   <212> DNA
   <213> Homo sapiens
<400> 302
   tggagccagt tactgggcag gtgtctgtac tggtgatgtg 40
<211> 40
   <212> DNA
   <213> Homo sapiens
<400> 303
   aaaagaaact gaggaatcag tatcacaggc agaagctctg 40
<210> 304
   <211> 40
   <212> DNA
   <213> Homo sapiens
<400> 304
   aaaagaaact gaggaatcag ccttagtatc acaggcagaa 40
<210> 305
   <211> 40
   <212> DNA
   <213> Homo sapiens
<400> 305
   cagcactagg ttataaagag gagtctagta aaagccctaa 40
<210> 306
   <211> 40
   <212> DNA
   <213> Homo sapiens
<400> 306
   cagcactagg ttataaagag aggatgtctt atatcttaaa 40
<210> 307
   <211> 40
   <212> DNA
   <213> Homo sapiens
<400> 307
   gcccccgttt tcctgcccag cccttgtcct cagtgcaccc 40
<210> 308
   <211> 40
   <212> DNA
   <213> Homo sapiens
<400> 308
   gcccccgttt tcctgcccag tacctgaagc tgcgggagcg 40
<210> 309
   <211> 40
   <212> DNA
   <213> Homo sapiens
<400> 309
   gccgccgccg ccgccgccag gctctgatgc tggtgtctgg 40
<210> 310
   <211> 40
   <212> DNA
   <213> Homo sapiens
<400> 310
   caccttatga agtatagcag gctctgatgc tggtgtctgg 40
<210> 311
   <211> 40
   <212> DNA
   <213> Homo sapiens
<400> 311
   agtggcagtg gctgtaccag cccacaggaa acaacccgta 40
<210> 312
   <211> 40
   <212> DNA
   <213> Homo sapiens
<400> 312
   agtggcagtg gctgtaccag ctcttggtgg agggctccac 40
<210> 313
   <211> 40
   <212> DNA
   <213> Homo sapiens
<400> 313
   gagattctga agataaggag ttctcttgta ggatgccact 40
<210> 314
   <211> 40
   <212> DNA
   <213> Homo sapiens
<400> 314
   gagattctga agataaggag gtaaaacctg tttagaaatt 40
<210> 315
   <211> 40
   <212> DNA
   <213> Homo sapiens
<400> 315
   ccaagagaca gcacattcag ctcctgagca gcctgactga 40
<210> 316
   <211> 40
   <212> DNA
   <213> Homo sapiens
<400> 316
   tcagagcagt cgggacacag gacacctgac tgatagtgaa 40
<210> 317
   <211> 40
   <212> DNA
   <213> Homo sapiens
<400> 317
   ctacgacagt gaagattcag gacacctgac tgatagtgaa 40
<210> 318
   <211> 40
   <212> DNA
   <213> Homo sapiens
<400> 318
   ctgttgtgtc cgttttgaag agccctttgc tcctccctca 40
<210> 319
   <211> 40
   <212> DNA
   <213> Homo sapiens
<400> 319
   ctgttgtgtc cgttttgaag aatgaacgga gaccagaatt 40
<210> 320
   <211> 40
   <212> DNA
   <213> Homo sapiens
<400> 320
   ccggccctac aggctggcgg ataaacccac tgccctacag 40
<210> 321
   <211> 40
   <212> DNA
   <213> Homo sapiens
<400> 321
   ccctccgcct cctgatgcag ataaacccac tgccctacag 40
<210> 322
   <211> 40
   <212> DNA
   <213> Homo sapiens
<400> 322
   gagattctga agataaggag gatgccactg gaaatgttga 40
<210> 323
   <211> 40
   <212> DNA
   <213> Homo sapiens
<400> 323
   tgaaaagtcc agaggaagag gttgtggcag cactgcctga 40
<210> 324
   <211> 40
   <212> DNA
   <213> Homo sapiens
<400> 324
   tcctggagga gctacgcagg gttgtggcag cactgcctga 40
<210> 325
   <211> 40
   <212> DNA
   <213> Homo sapiens
<400> 325
   gtcatggcag aagacctcca tccaagacat ctctggcatc 40
<210> 326
   <211> 40
   <212> DNA
   <213> Homo sapiens
<400> 326
   ctatagctac tggatatggg tccaagacat ctctggcatc 40
<210> 327
   <211> 40
   <212> DNA
   <213> Homo sapiens
<400> 327
   ccggagcccc ttcaaaaaag acttttcgtg ttttacagtc 40
<210> 328
   <211> 40
   <212> DNA
   <213> Homo sapiens
<400> 328
   ccggagcccc ttcaaaaaag tctgttgcca gaatcggcca 40
<210> 329
   <211> 40
   <212> DNA
   <213> Homo sapiens
<400> 329
   ccacagatac tattaggagg ccataccacc ctgaacgcgc 40
<210> 330
   <211> 40
   <212> DNA
   <213> Homo sapiens
<400> 330
   ccacagatac tattaggagg gaatttatca tggcatccag 40
<210> 331
   <211> 40
   <212> DNA
   <213> Homo sapiens
<400> 331
   tgttcaagtt cccaaagcag gagatcctgc tgggccgtgg 40
<210> 332
   <211> 40
   <212> DNA
   <213> Homo sapiens
<400> 332
   actcccagct caatgcaatg gttccatacc atctggtact 40
<210> 333
   <211> 40
   <212> DNA
   <213> Homo sapiens
<400> 333
   actcccagct caatgcaatg gctcatcaga ttcaagagat 40
<210> 334
   <211> 40
   <212> DNA
   <213> Homo sapiens
<400> 334
   atcactgtga cttccctgag gtctctgctc ctcagctgct 40
<210> 335
   <211> 40
   <212> DNA
   <213> Homo sapiens
<400> 335
   atcactgtga cttccctgag ctgctgtccc ccagcaacgt 40
<210> 336
   <211> 40
   <212> DNA
   <213> Homo sapiens
<400> 336
   atcctctcaa tcaaaataag tttgtgtgca cttttctgct 40
<210> 337
   <211> 40
   <212> DNA
   <213> Homo sapiens
<400> 337
   atcctctcaa tcaaaataag ggtaaaccag acttgaatac 40
<210> 338
   <211> 40
   <212> DNA
   <213> Homo sapiens
<400> 338
   ctattccttt attgaatttg ttttcttcat cattctagat 40
<210> 339
   <211> 40
   <212> DNA
   <213> Homo sapiens
<400> 339
   ctattccttt attgaatttg atactttcat tcagaaaacc 40
<210> 340
   <211> 40
   <212> DNA
   <213> Homo sapiens
<400> 340
   agtcatacct ggagcagcag tttgtttctt ttctagaaaa 40
<210> 341
   <211> 40
   <212> DNA
   <213> Homo sapiens
<400> 341
   agtcatacct ggagcagcag aaaaaattga aagaactgtc 40
<210> 342
   <211> 40
   <212> DNA
   <213> Homo sapiens
<400> 342
   gcaaccagtt tgggcatcag ctgcccttct ctcctgtagg 40
<210> 343
   <211> 40
   <212> DNA
   <213> Homo sapiens
<400> 343
   gcaaccagtt tgggcatcag gagaacgcca agaacgaaga 40
<210> 344
   <211> 40
   <212> DNA
   <213> Homo sapiens
<400> 344
   ccatggtcaa aaaatggcag caccaacagg tccgccaaat 40
<210> 345
   <211> 40
   <212> DNA
   <213> Homo sapiens
<400> 345
   ccatggtcaa aaaatggcag acaatgattg aagctcacgt 40
<210> 346
   <211> 40
   <212> DNA
   <213> Homo sapiens
<400> 346
   gcctgatgcc cgaatttcag gccatgaagt acttgtcata 40
<210> 347
   <211> 40
   <212> DNA
   <213> Homo sapiens
<400> 347
   gcctgatgcc cgaatttcag tttggcactt acagcgaatc 40
<210> 348
   <211> 40
   <212> DNA
   <213> Homo sapiens
<400> 348
   agattgaagc taaaattaag ttttctgtct tacccattcc 40
<210> 349
   <211> 40
   <212> DNA
   <213> Homo sapiens
<400> 349
   agattgaagc taaaattaag gagctgacaa gtacttgtag 40
<210> 350
   <211> 40
   <212> DNA
   <213> Homo sapiens
<400> 350
   agcacaagct atgtatcaag cataactttc ttctacagga 40
<210> 351
   <211> 40
   <212> DNA
   <213> Homo sapiens
<400> 351
   agcacaagct atgtatcaag gattctggag tgaagcagat 40
<210> 352
   <211> 40
   <212> DNA
   <213> Homo sapiens
<400> 352
   agatgtaaaa gtgtcactgt tttggttttc agttacagct 40
<210> 353
   <211> 40
   <212> DNA
   <213> Homo sapiens
<400> 353
   agatgtaaaa gtgtcactgt ttacagcttt cttcctggct 40
<210> 354
   <211> 40
   <212> DNA
   <213> Homo sapiens
<400> 354
   ctgcagcctc cgcctcccag gaacttgcca catcagactc 40
<210> 355
   <211> 40
   <212> DNA
   <213> Homo sapiens
<400> 355
   aggatgatgc agcatccaac tggtcttttt gtgttctgtg 40
<210> 356
   <211> 40
   <212> DNA
   <213> Homo sapiens
<400> 356
   aggatgatgc agcatccaac gcgggcacat gaacgccccc 40
<210> 357
   <211> 40
   <212> DNA
   <213> Homo sapiens
<400> 357
   tggtgaaatg gaccccaaag tctttctctt tcaagtacct 40
<210> 358
   <211> 40
   <212> DNA
   <213> Homo sapiens
<400> 358
   tggtgaaatg gaccccaaag tacctgctat tgaggagaac 40
<210> 359
   <211> 40
   <212> DNA
   <213> Homo sapiens
<400> 359
   agcttaaaga actgtattcg tttgactgca accctggagt 40
<210> 360
   <211> 40
   <212> DNA
   <213> Homo sapiens
<400> 360
   gatcaaggca accgggaaag tttgactgca accctggagt 40
<210> 361
   <211> 40
   <212> DNA
   <213> Homo sapiens
<400> 361
   aacacaccaa ctttgtggag gtcctggcaa tctccgttgc 40
<210> 362
   <211> 40
   <212> DNA
   <213> Homo sapiens
<400> 362
   aacacaccaa ctttgtggag ttccggaact ttaagatcat 40
<210> 363
   <211> 40
   <212> DNA
   <213> Homo sapiens
<400> 363
   gcgggtctgc agcctacgca aactgaagca ggcccagacc 40
<210> 364
   <211> 40
   <212> DNA
   <213> Homo sapiens
<400> 364
   gttccaggtc ctcctggcag aactgaagca ggcccagacc 40
<210> 365
   <211> 40
   <212> DNA
   <213> Homo sapiens
<400> 365
   cccgctgccc cagctcaaag atcagtgcta acatcttccg 40
<210> 366
   <211> 40
   <212> DNA
   <213> Homo sapiens
<400> 366
   attctgatat agtaaaaatg atcagtgcta acatcttccg 40
<210> 367
   <211> 40
   <212> DNA
   <213> Homo sapiens
<400> 367
   atgagtttcc caccgatggg gaggaagacc gcaggaagga 40
<210> 368
   <211> 40
   <212> DNA
   <213> Homo sapiens
<400> 368
   atgagtttcc caccgatggg gagatgtcag cgcaggagga 40
<210> 369
   <211> 40
   <212> DNA
   <213> Homo sapiens
<400> 369
   agtttattta acatttgatg agcctacctt gtacaatgct 40
<210> 370
   <211> 40
   <212> DNA
   <213> Homo sapiens
<400> 370
   agtttattta acatttgatg aacttcgaga aaccaagacc 40
<210> 371
   <211> 40
   <212> DNA
   <213> Homo sapiens
<400> 371
   ccacctagca gccaccagag accagaggtg gcacaggcag 40
<210> 372
   <211> 40
   <212> DNA
   <213> Homo sapiens
<400> 372
   ccacctagca gccaccagag gttacaaggg gagagtggcc 40
<210> 373
   <211> 40
   <212> DNA
   <213> Homo sapiens
<400> 373
   tccaggatcc tgaggcatgg ccatatcagc gggaacaaga 40
<210> 374
   <211> 40
   <212> DNA
   <213> Homo sapiens
<400> 374
   ggcagcggag gggcgacaaa ccatatcagc gggaacaaga 40
<210> 375
   <211> 40
   <212> DNA
   <213> Homo sapiens
<400> 375
   ggcaacttcg ttaatatgag ctttctactc aacaggtcta 40
<210> 376
   <211> 40
   <212> DNA
   <213> Homo sapiens
<400> 376
   ggcaacttcg ttaatatgag gtctatccag gaaaatggtg 40
<210> 377
   <211> 40
   <212> DNA
   <213> Homo sapiens
<400> 377
   ggagcctggg catctcgttg ccctgcccgt ctccctccca 40
<210> 378
   <211> 40
   <212> DNA
   <213> Homo sapiens
<400> 378
   ggagcctggg catctcgttg gtggagctgg caacaggaca 40
<210> 379
   <211> 40
   <212> DNA
   <213> Homo sapiens
<400> 379
   ctggtgtgct tgggagccag ggttatcatg aagattaaat 40
<210> 380
   <211> 40
   <212> DNA
   <213> Homo sapiens
<400> 380
   ctggtgtgct tgggagccag agatcacctc ctacaccact 40
<210> 381
   <211> 40
   <212> DNA
   <213> Homo sapiens
<400> 381
   attggaggag cttctggaaa gatgccctct tcgcttccca 40
<210> 382
   <211> 40
   <212> DNA
   <213> Homo sapiens
<400> 382
   attggaggag cttctggaaa gtgctcttga tgatttcgat 40
<210> 383
   <211> 40
   <212> DNA
   <213> Homo sapiens
<400> 383
   aatgacgtgc tgcaccactg ggccctgacg cgcggaaagt 40
<210> 384
   <211> 40
   <212> DNA
   <213> Homo sapiens
<400> 384
   aatgacgtgc tgcaccactg ccagcgcaag caggcccggg 40
<210> 385
   <211> 40
   <212> DNA
   <213> Homo sapiens
<400> 385
   gcctggggtg gagagggcag ccccccagct accacaagaa 40
<210> 386
   <211> 40
   <212> DNA
   <213> Homo sapiens
<400> 386
   gcctggggtg gagagggcag tctgggatgt ggcattggct 40
<210> 387
   <211> 40
   <212> DNA
   <213> Homo sapiens
<400> 387
   ggaaatggga caggaggcag aggatcacag gctttaaaat 40
<210> 388
   <211> 40
   <212> DNA
   <213> Homo sapiens
<400> 388
   ggaaatggga caggaggcag cttttctctc aacagaggat 40
<210> 389
   <211> 40
   <212> DNA
   <213> Homo sapiens
<400> 389
   agaccgactg ccagtaatag gagattgtga agacctttga 40
<210> 390
   <211> 40
   <212> DNA
   <213> Homo sapiens
<400> 390
   agaccgactg ccagtaatag agcctgttag tattaatgaa 40
<210> 391
   <211> 40
   <212> DNA
   <213> Homo sapiens
<400> 391
   tcatgctagc cgaggcccag tggcggccag aggagtccga 40
<210> 392
   <211> 40
   <212> DNA
   <213> Homo sapiens
<400> 392
   tcatgctagc cgaggcccag gaaaccacta tcagcggcct 40
<210> 393
   <211> 40
   <212> DNA
   <213> Homo sapiens
<400> 393
   gaaggcagct gagcaaacag ttctctccct tgcagctgcc 40
<210> 394
   <211> 40
   <212> DNA
   <213> Homo sapiens
<400> 394
   gaaggcagct gagcaaacag ctgcccggga acaggcaaag 40
<210> 395
   <211> 40
   <212> DNA
   <213> Homo sapiens
<400> 395
   gccaacagcc aattctacag gtacaacaaa taacactgtg 40
<210> 396
   <211> 40
   <212> DNA
   <213> Homo sapiens
<400> 396
   gccaacagcc aattctacag ctaaacccac agttcagccc 40
<210> 397
   <211> 40
   <212> DNA
   <213> Homo sapiens
<400> 397
   cccatcaact gcacccactc ccctctgacg tccatcatct 40
<210> 398
   <211> 40
   <212> DNA
   <213> Homo sapiens
<400> 398
   cccatcaact gcacccactc ctgtgtggac ctggatgaca 40
<210> 399
   <211> 40
   <212> DNA
   <213> Homo sapiens
<400> 399
   gcggaaagaa ttgcatgaag agcgacaaca acacaaccag 40
<210> 400
   <211> 40
   <212> DNA
   <213> Homo sapiens
<400> 400
   gcggaaagaa ttgcatgaag tttgccatct cttggagcaa 40
<210> 401
   <211> 40
   <212> DNA
   <213> Homo sapiens
<400> 401
   tgtgggaatt acaattcaag cttatcacac agactttcag 40
<210> 402
   <211> 40
   <212> DNA
   <213> Homo sapiens
<400> 402
   aagaagggat ggcagagaag cttatcacac agactttcag 40
<210> 403
   <211> 40
   <212> DNA
   <213> Homo sapiens
<400> 403
   cttcctcaag tcgcccaaag ctcccccgtt tcttctcccc 40
<210> 404
   <211> 40
   <212> DNA
   <213> Homo sapiens
<400> 404
   cttcctcaag tcgcccaaag acaacgtgga cgaccccacg 40
<210> 405
   <211> 40
   <212> DNA
   <213> Homo sapiens
<400> 405
   tcttcgctgg tggcaaactg tatcgtgaag agcgcttccg 40
<210> 406
   <211> 40
   <212> DNA
   <213> Homo sapiens
<400> 406
   tcttcgctgg tggcaaactg cgggtgcatc tcgacatcca 40
<210> 407
   <211> 40
   <212> DNA
   <213> Homo sapiens
<400> 407
   gcaagaagta caaagtggag tatgtgcttt gttgtgacag 40
<210> 408
   <211> 40
   <212> DNA
   <213> Homo sapiens
<400> 408
   gcaagaagta caaagtggag tatcctatca tgtacagcac 40
<210> 409
   <211> 40
   <212> DNA
   <213> Homo sapiens
<400> 409
   tgtaggagca atgactgttg cattcttttt ctttaggtat 40
<210> 410
   <211> 40
   <212> DNA
   <213> Homo sapiens
<400> 410
   tgtaggagca atgactgttg gtatgggcta ttccatgtat 40
<210> 411
   <211> 40
   <212> DNA
   <213> Homo sapiens
<400> 411
   ccttcctgga tccccctaag gtggtattaa agataatcaa 40
<210> 412
   <211> 40
   <212> DNA
   <213> Homo sapiens
<400> 412
   aagtgcagat agatggcctt gtggtattaa agataatcaa 40
<210> 413
   <211> 40
   <212> DNA
   <213> Homo sapiens
<400> 413
   caggtctaac tcgcttccag gccccagcag atgaacctga 40
<210> 414
   <211> 40
   <212> DNA
   <213> Homo sapiens
<400> 414
   caggtctaac tcgcttccag gctgaagctt cagaaaagga 40
<210> 415
   <211> 40
   <212> DNA
   <213> Homo sapiens
<400> 415
   cctccccata cctgagctcg atggcggtgg gaccccccga 40
<210> 416
   <211> 40
   <212> DNA
   <213> Homo sapiens
<400> 416
   gcaaaaggat ataccaggag catttatttc aggggtcctc 40
<210> 417
   <211> 40
   <212> DNA
   <213> Homo sapiens
<400> 417
   gcaaaaggat ataccaggag gggtcctcaa gattcgagat 40
<210> 418
   <211> 40
   <212> DNA
   <213> Homo sapiens
<400> 418
   cactccaatt tatagattct gattcttcat catggtgtga 40
<210> 419
   <211> 40
   <212> DNA
   <213> Homo sapiens
<400> 419
   cactccaatt tatagattct ttcttaaaca cttccagtaa 40
<210> 420
   <211> 40
   <212> DNA
   <213> Homo sapiens
<400> 420
   tgagagtctt cagttactag tttgtctttc ctagatccag 40
<210> 421
   <211> 40
   <212> DNA
   <213> Homo sapiens
<400> 421
   tgagagtctt cagttactag aggcggattt ccctgactga 40
<210> 422
   <211> 40
   <212> DNA
   <213> Homo sapiens
<400> 422
   ttaacagcat tttgttttgc gattcctgcc agctcccagg 40
<210> 423
   <211> 40
   <212> DNA
   <213> Homo sapiens
<400> 423
   cccagtcatt caacaggaag gattcctgcc agctcccagg 40
<210> 424
   <211> 40
   <212> DNA
   <213> Homo sapiens
<400> 424
   gatgaatgct gacatggatg atctctctgc aagagtagat 40
<210> 425
   <211> 40
   <212> DNA
   <213> Homo sapiens
<400> 425
   gatgaatgct gacatggatg cagttgatgc tgaaaatcaa 40
<210> 426
   <211> 40
   <212> DNA
   <213> Homo sapiens
<400> 426
   gtcaatgctt ccatgtccag ctttctgtct tctaggttcc 40
<210> 427
   <211> 40
   <212> DNA
   <213> Homo sapiens
<400> 427
   gtcaatgctt ccatgtccag gttccctccc catatggtcc 40
<210> 428
   <211> 40
   <212> DNA
   <213> Homo sapiens
<400> 428
   tatggcaagg aggtcgacct tctctttccc agctgggcct 40
<210> 429
   <211> 40
   <212> DNA
   <213> Homo sapiens
<400> 429
   tatggcaagg aggtcgacct ctgggcctgt ggggtgatct 40
<210> 430
   <211> 40
   <212> DNA
   <213> Homo sapiens
<400> 430
   tggttttacc tcggatagag acatttgtta tcgctgtggt 40
<210> 431
   <211> 40
   <212> DNA
   <213> Homo sapiens
<400> 431
   tggttttacc tcggatagag gtttccagtt tgtttcctcg 40
<210> 432
   <211> 40
   <212> DNA
   <213> Homo sapiens
<400> 432
   gaatccgtat ctgggaacag agccctttgc tcctccctca 40
<210> 433
   <211> 40
   <212> DNA
   <213> Homo sapiens
<400> 433
   gaatccgtat ctgggaacag aatgaacgga gaccagaatt 40
<210> 434
   <211> 40
   <212> DNA
   <213> Homo sapiens
<400> 434
   tccaggagtt ccaggttccg tgtttcactt caagcccact 40
<210> 435
   <211> 40
   <212> DNA
   <213> Homo sapiens
<400> 435
   tttgactagg gtccaaccag tgtttcactt caagcccact 40
<210> 436
   <211> 40
   <212> DNA
   <213> Homo sapiens
<400> 436
   gggcctgatg aatgacatcg cttcctcggc agtcatggga 40
<210> 437
   <211> 40
   <212> DNA
   <213> Homo sapiens
<400> 437
   gggcctgatg aatgacatcg cagccttccc tgcacccacc 40
<210> 438
   <211> 40
   <212> DNA
   <213> Homo sapiens
<400> 438
   agccccagga tgcctcgcag ctctcggaag aactggttgt 40
<210> 439
   <211> 40
   <212> DNA
   <213> Homo sapiens
<400> 439
   agccccagga tgcctcgcag acgtgccttc tgccatgatt 40
<210> 440
   <211> 40
   <212> DNA
   <213> Homo sapiens
<400> 440
   acttgcctgt gaatttcgag tctttccctc tgaaacaggt 40
<210> 441
   <211> 40
   <212> DNA
   <213> Homo sapiens
<400> 441
   acttgcctgt gaatttcgag gtggcccggg agagtggccc 40
<210> 442
   <211> 40
   <212> DNA
   <213> Homo sapiens
<400> 442
   tacccgggac aaccccaagg ccgcccaccc caccccccat 40
<210> 443
   <211> 40
   <212> DNA
   <213> Homo sapiens
<400> 443
   tacccgggac aaccccaagg ggctctgtga cctctgcccc 40
<210> 444
   <211> 40
   <212> DNA
   <213> Homo sapiens
<400> 444
   catagtggaa gtgatagatc ttctttttca cattacagtg 40
<210> 445
   <211> 40
   <212> DNA
   <213> Homo sapiens
<400> 445
   catagtggaa gtgatagatc tggcctgaag cacgaggaca 40
<210> 446
   <211> 40
   <212> DNA
   <213> Homo sapiens
<400> 446
   gctgtacctt caggaacagg ccctttctcc caggtttcca 40
<210> 447
   <211> 40
   <212> DNA
   <213> Homo sapiens
<400> 447
   gctgtacctt caggaacagg gtttccatgc tgagctcctg 40
<210> 448
   <211> 40
   <212> DNA
   <213> Homo sapiens
<400> 448
   taaagcgact cattgagcag gaggtggtat aacagacaga 40
<210> 449
   <211> 40
   <212> DNA
   <213> Homo sapiens
<400> 449
   taaagcgact cattgagcag gcaaaaggca ggattgtggt 40
<210> 450
   <211> 40
   <212> DNA
   <213> Homo sapiens
<400> 450
   tgggaatctg gccagagaag tctttctgtc ttgttttgaa 40
<210> 451
   <211> 40
   <212> DNA
   <213> Homo sapiens
<400> 451
   tgggaatctg gccagagaag gtgcttgaca tcctccagca 40
<210> 452
   <211> 40
   <212> DNA
   <213> Homo sapiens
<400> 452
   agaaaacatc gaattcagag cttgataatg gaactataca 40
<210> 453
   <211> 40
   <212> DNA
   <213> Homo sapiens
<400> 453
   agaaaacatc gaattcagag agttccagaa gacagcgaac 40
<210> 454
   <211> 40
   <212> DNA
   <213> Homo sapiens
<400> 454
   cgtccgccag tcgtcccgag gcatgaagaa ctcttgactg 40
<210> 455
   <211> 40
   <212> DNA
   <213> Homo sapiens
<400> 455
   agccgggcgt tgggggaaag gcatgaagaa ctcttgactg 40
<210> 456
   <211> 40
   <212> DNA
   <213> Homo sapiens
<400> 456
   actaatcttc agcatgccat tcggcgtggc acaagcctaa 40
<210> 457
   <211> 40
   <212> DNA
   <213> Homo sapiens
<400> 457
   caaacacctc ttgattataa tcggcgtggc acaagcctaa 40
<210> 458
   <211> 40
   <212> DNA
   <213> Homo sapiens
<400> 458
   caccacaaaa tcacagacag cttgcttgcc ttttgtttta 40
<210> 459
   <211> 40
   <212> DNA
   <213> Homo sapiens
<400> 459
   caccacaaaa tcacagacag cagctgcagt atctcggaag 40
<210> 460
   <211> 40
   <212> DNA
   <213> Homo sapiens
<400> 460
   ctcctactac acaatctaag atttcagaaa tggccaaaga 40
<210> 461
   <211> 40
   <212> DNA
   <213> Homo sapiens
<400> 461
   agagctcaaa gaagtgttta atttcagaaa tggccaaaga 40
<210> 462
   <211> 40
   <212> DNA
   <213> Homo sapiens
<400> 462
   atttccagag gatttacact tttgcttgac agggtcagtg 40
<210> 463
   <211> 40
   <212> DNA
   <213> Homo sapiens
<400> 463
   atttccagag gatttacact ggtcagtgct gcttgcccat 40
<210> 464
   <211> 40
   <212> DNA
   <213> Homo sapiens
<400> 464
   gagtcggcgc cgagaacatg tttcctgtgg gccgcatcca 40
<210> 465
   <211> 40
   <212> DNA
   <213> Homo sapiens
<400> 465
   cacagagagc tgggctacag tttcctgtgg gccgcatcca 40
<210> 466
   <211> 40
   <212> DNA
   <213> Homo sapiens
<400> 466
   tgacgttctc tgtgctccag tggtttctcc cacaggttcc 40
<210> 467
   <211> 40
   <212> DNA
   <213> Homo sapiens
<400> 467
   tgacgttctc tgtgctccag gttcccggcc cccaagtcgc 40
<210> 468
   <211> 40
   <212> DNA
   <213> Homo sapiens
<400> 468
   caaacacctc ttgattataa cacgcaggta acatggatgt 40
<210> 469
   <211> 40
   <212> DNA
   <213> Homo sapiens
<400> 469
   tactccagct tcagcaacag cacctacaga agcggctcaa 40
<210> 470
   <211> 40
   <212> DNA
   <213> Homo sapiens
<400> 470
   tactccagct tcagcaacag caggtgatac cctgtcggtc 40
<210> 471
   <211> 40
   <212> DNA
   <213> Homo sapiens
<400> 471
   tctcagctga cgaatgcaag gcaccaacgg agagacagct 40
<210> 472
   <211> 40
   <212> DNA
   <213> Homo sapiens
<400> 472
   ggcatgcaac caggcaccag gcaccaacgg agagacagct 40
<210> 473
   <211> 40
   <212> DNA
   <213> Homo sapiens
<400> 473
   tcaaatcatt tacctccaag cagccagctc ctgtcaccat 40
<210> 474
   <211> 40
   <212> DNA
   <213> Homo sapiens
<400> 474
   tcaaatcatt tacctccaag aggactcctg atggatttga 40
<210> 475
   <211> 40
   <212> DNA
   <213> Homo sapiens
<400> 475
   agcaaaaagg ggtgtctcag aatctccggc ctgtgaaact 40
<210> 476
   <211> 40
   <212> DNA
   <213> Homo sapiens
<400> 476
   agcaaaaagg ggtgtctcag gccactcttc acctccacca 40
<210> 477
   <211> 40
   <212> DNA
   <213> Homo sapiens
<400> 477
   tgttgcctcc gcggccgcag gacagcaggt gccaggcttc 40
<210> 478
   <211> 40
   <212> DNA
   <213> Homo sapiens
<400> 478
   tggtcatggc caaaccctgg gacagcaggt gccaggcttc 40
<210> 479
   <211> 40
   <212> DNA
   <213> Homo sapiens
<400> 479
   tgcctaaggc ggatttgaat ctctttctct cccttcagaa 40
<210> 480
   <211> 40
   <212> DNA
   <213> Homo sapiens
<400> 480
   tgcctaaggc ggatttgaat aatcttatct tggctttgga 40
<210> 481
   <211> 40
   <212> DNA
   <213> Homo sapiens
<400> 481
   tggtcatggc caaaccctgg gctccaccct catccagctg 40
<210> 482
   <211> 40
   <212> DNA
   <213> Homo sapiens
<400> 482
   tgcagattcc aaaagaaacg aaagcagaag atgaggatat 40
<210> 483
   <211> 40
   <212> DNA
   <213> Homo sapiens
<400> 483
   ccttccaccc aagggaactg aaagcagaag atgaggatat 40
<210> 484
   <211> 40
   <212> DNA
   <213> Homo sapiens
<400> 484
   aggagggccc cctgccgctg gcaacaactc ccagccctgc 40
<210> 485
   <211> 40
   <212> DNA
   <213> Homo sapiens
<400> 485
   aggagggccc cctgccgctg ctgacccctt tggcccgctt 40
<210> 486
   <211> 40
   <212> DNA
   <213> Homo sapiens
<400> 486
   aacaactgcc cagctttgag tggcaataat attgaactgg 40
<210> 487
   <211> 40
   <212> DNA
   <213> Homo sapiens
<400> 487
   aacaactgcc cagctttgag gaaatctgaa atagagtact 40
<210> 488
   <211> 40
   <212> DNA
   <213> Homo sapiens
<400> 488
   gttgtgccca tgacctccag gttaggatta attgagtggc 40
<210> 489
   <211> 40
   <212> DNA
   <213> Homo sapiens
<400> 489
   gttgtgccca tgacctccag tgatcccagg gcaccgccgt 40
<210> 490
   <211> 40
   <212> DNA
   <213> Homo sapiens
<400> 490
   gttaatgggt ttaatggaga gggcggcgaa gaggacccgc 40
<210> 491
   <211> 40
   <212> DNA
   <213> Homo sapiens
<400> 491
   gttaatgggt ttaatggaga tgagaaggca accaaagtgc 40
<210> 492
   <211> 40
   <212> DNA
   <213> Homo sapiens
<400> 492
   gcaaggagca acagcgatgg tgagaaggca accaaagtgc 40
<210> 493
   <211> 40
   <212> DNA
   <213> Homo sapiens
<400> 493
   agtttgagat gaagcgaatg gatcctggct tcctggacaa 40
<210> 494
   <211> 40
   <212> DNA
   <213> Homo sapiens
<400> 494
   agtttgagat gaagcgaatg ctccccctac caggggtcgc 40
<210> 495
   <211> 40
   <212> DNA
   <213> Homo sapiens
<400> 495
   agaaaccttg aacgacaaag tggaattttt atactgtgac 40
<210> 496
   <211> 40
   <212> DNA
   <213> Homo sapiens
<400> 496
   agaaaccttg aacgacaaag agacgtgagt cttgctgtgt 40
<210> 497
   <211> 40
   <212> DNA
   <213> Homo sapiens
<400> 497
   acccctttgg catcgatcct gccctttcct cagcacaaga 40
<210> 498
   <211> 40
   <212> DNA
   <213> Homo sapiens
<400> 498
   acccctttgg catcgatcct atttggagcc tggctgccaa 40
<210> 499
   <211> 40
   <212> DNA
   <213> Homo sapiens
<400> 499
   taaagtgttg gctttactta aatttatctt tacagatact 40
<210> 500
   <211> 40
   <212> DNA
   <213> Homo sapiens
<400> 500
   taaagtgttg gctttactta atactgcaaa caatttagtt 40
<210> 501
   <211> 40
   <212> DNA
   <213> Homo sapiens
<400> 501
   acctcgtcag aaacaaccag agttcccccg tttctagagg 40
<210> 502
   <211> 40
   <212> DNA
   <213> Homo sapiens
<400> 502
   acctcgtcag aaacaaccag aggttggacc agcctcaatg 40
<210> 503
   <211> 40
   <212> DNA
   <213> Homo sapiens
<400> 503
   aaagatttca gaagaaatac tatttctctt tcaggtatac 40
<210> 504
   <211> 40
   <212> DNA
   <213> Homo sapiens
<400> 504
   aaagatttca gaagaaatac gtataccaac tgcagcctta 40
<210> 505
   <211> 40
   <212> DNA
   <213> Homo sapiens
<400> 505
   ccaaaagagg ggataatgag ggaaggtgaa gaaggagctg 40
<210> 506
   <211> 40
   <212> DNA
   <213> Homo sapiens
<400> 506
   tcatcttgaa aaatgaaaat tcctatttta cagctgagga 40
<210> 507
   <211> 40
   <212> DNA
   <213> Homo sapiens
<400> 507
   tcatcttgaa aaatgaaaat gtggataggc atgtagacct 40
<210> 508
   <211> 40
   <212> DNA
   <213> Homo sapiens
<400> 508
   accctgtcta ccagcctgtg ttttctgcca cctacaggat 40
<210> 509
   <211> 40
   <212> DNA
   <213> Homo sapiens
<400> 509
   accctgtcta ccagcctgtg gatagaccat gaagctgaag 40
<210> 510
   <211> 40
   <212> DNA
   <213> Homo sapiens
<400> 510
   tgacacagcc ctgcaggcag ggtccgtgca ggacctttcc 40
<210> 511
   <211> 40
   <212> DNA
   <213> Homo sapiens
<400> 511
   tgacacagcc ctgcaggcag aaggatcccg caaacgtgga 40
<210> 512
   <211> 40
   <212> DNA
   <213> Homo sapiens
<400> 512
   gcggggcgag ggcagctccg cgtttctctg aattctcccc 40
<210> 513
   <211> 40
   <212> DNA
   <213> Homo sapiens
<400> 513
   gcggggcgag ggcagctccg ggaaggaacg tcccagggat 40
<210> 514
   <211> 40
   <212> DNA
   <213> Homo sapiens
<400> 514
   ccacctcacc atcacccagg gcagcccctc cacagggccc 40
<210> 515
   <211> 40
   <212> DNA
   <213> Homo sapiens
<400> 515
   ccacctcacc atcacccagg ccctcaggca gcccctccac 40
<210> 516
   <211> 40
   <212> DNA
   <213> Homo sapiens
<400> 516
   gccaacctag agcccccctg ctctctgcct cttacagatg 40
<210> 517
   <211> 40
   <212> DNA
   <213> Homo sapiens
<400> 517
   gccaacctag agcccccctg atgactggca tagcctgggc 40
<210> 518
   <211> 40
   <212> DNA
   <213> Homo sapiens
<400> 518
   aaccggggga gcgaggcacg tttctttccc cacctttcta 40
<210> 519
   <211> 40
   <212> DNA
   <213> Homo sapiens
<400> 519
   aaccggggga gcgaggcacg gagtgtacct cacagccttc 40
<210> 520
   <211> 40
   <212> DNA
   <213> Homo sapiens
<400> 520
   cacacagact gcgttcgatg agtgtcttcc ccctgcctta 40
<210> 521
   <211> 40
   <212> DNA
   <213> Homo sapiens
<400> 521
   cacacagact gcgttcgatg ccttgctgtt caccctgatg 40
<210> 522
   <211> 40
   <212> DNA
   <213> Homo sapiens
<400> 522
   gtttttacct ctgcctcctg atctctcatc ctaggttttc 40
<210> 523
   <211> 40
   <212> DNA
   <213> Homo sapiens
<400> 523
   gtttttacct ctgcctcctg gttttcatac tctgcacacc 40
<210> 524
   <211> 40
   <212> DNA
   <213> Homo sapiens
<400> 524
   cactgctggg agagtggaag ttgcttccac agattcctga 40
<210> 525
   <211> 40
   <212> DNA
   <213> Homo sapiens
<400> 525
   cactgctggg agagtggaag attcctgaga gctgccggcc 40
<210> 526
   <211> 40
   <212> DNA
   <213> Homo sapiens
<400> 526
   gattttggag aggcaaccaa ctttgttttt cacagattcc 40
<210> 527
   <211> 40
   <212> DNA
   <213> Homo sapiens
<400> 527
   gattttggag aggcaaccaa attccctgga ctttgtcacc 40
<210> 528
   <211> 40
   <212> DNA
   <213> Homo sapiens
<400> 528
   tgcagaactg gataaagaag tgtatttttt tgtctcaatt 40
<210> 529
   <211> 40
   <212> DNA
   <213> Homo sapiens
<400> 529
   tgcagaactg gataaagaag gtgcttctaa agtaaagaaa 40
<210> 530
   <211> 40
   <212> DNA
   <213> Homo sapiens
<400> 530
   actcttatgc agtccccatg aggttatgct tatgtttctc 40
<210> 531
   <211> 40
   <212> DNA
   <213> Homo sapiens
<400> 531
   actcttatgc agtccccatg aggagatcct agtctcacca 40
<210> 532
   <211> 40
   <212> DNA
   <213> Homo sapiens
<400> 532
   agtgttttac catggatgtt gtcattccag ggctcctcag 40
<210> 533
   <211> 40
   <212> DNA
   <213> Homo sapiens
<400> 533
   agtgttttac catggatgtt ggctcctcag tggctgtgac 40
<210> 534
   <211> 40
   <212> DNA
   <213> Homo sapiens
<400> 534
   tttatgatgc tgctttaaag ttttgttaat gtttttcttt 40
<210> 535
   <211> 40
   <212> DNA
   <213> Homo sapiens
<400> 535
   tttatgatgc tgctttaaag ctcattaatg aaattgaaga 40
<210> 536
   <211> 40
   <212> DNA
   <213> Homo sapiens
<400> 536
   atctaaaaac agaagagcag gtcctttttt aggtgcaaaa 40
<210> 537
   <211> 40
   <212> DNA
   <213> Homo sapiens
<400> 537
   atctaaaaac agaagagcag gtgcaaaaac ttcaagctat 40
<210> 538
   <211> 40
   <212> DNA
   <213> Homo sapiens
<400> 538
   ggattgcagc caacacaaag tttctcttca taggaatgtc 40
<210> 539
   <211> 40
   <212> DNA
   <213> Homo sapiens
<400> 539
   ggattgcagc caacacaaag gaatgtccca aatgccatgt 40
<210> 540
   <211> 40
   <212> DNA
   <213> Homo sapiens
<400> 540
   ggtttcgagt ttgaatagtg ttttgcttgt ttgtttgttt 40
<210> 541
   <211> 40
   <212> DNA
   <213> Homo sapiens
<400> 541
   ggtttcgagt ttgaatagtg gtcagattga agttatcatg 40
<210> 542
   <211> 40
   <212> DNA
   <213> Homo sapiens
<400> 542
   cgcaagtact tcctgcccca tccagcagca cacagtggga 40
<210> 543
   <211> 40
   <212> DNA
   <213> Homo sapiens
<400> 543
   cgcaagtact tcctgcccca ggtagtggtg actgtgaacc 40
<210> 544
   <211> 40
   <212> DNA
   <213> Homo sapiens
<400> 544
   ttcataacaa accagtaaat cacattcagg aattcaccaa 40
<210> 545
   <211> 40
   <212> DNA
   <213> Homo sapiens
<400> 545
   tcatcaatgc cccgaccttg cacattcagg aattcaccaa 40
<210> 546
   <211> 40
   <212> DNA
   <213> Homo sapiens
<400> 546
   aaatttaaca ttactcatag tttttgctgt tttacagagt 40
<210> 547
   <211> 40
   <212> DNA
   <213> Homo sapiens
<400> 547
   aaatttaaca ttactcatag agtaagccat atcaaagact 40
<210> 548
   <211> 40
   <212> DNA
   <213> Homo sapiens
<400> 548
   caccgggagc tgcagggccg ccccttgtcc atcccaggca 40
<210> 549
   <211> 40
   <212> DNA
   <213> Homo sapiens
<400> 549
   caccgggagc tgcagggccg gcacgagcag ctgcaggccc 40
<210> 550
   <211> 40
   <212> DNA
   <213> Homo sapiens
<400> 550
   attggacaca gagatgggat atcgtgacgt ctgcatccac 40
<210> 551
   <211> 40
   <212> DNA
   <213> Homo sapiens
<400> 551
   ctgtctctag gctaagcaga atcgtgacgt ctgcatccac 40
<210> 552
   <211> 40
   <212> DNA
   <213> Homo sapiens
<400> 552
   gggacctcac caagcgcccg cccctcatca acctgcagat 40
<210> 553
   <211> 40
   <212> DNA
   <213> Homo sapiens
<400> 553
   gggacctcac caagcgcccg atctgcaggc aggccctgaa 40
<210> 554
   <211> 40
   <212> DNA
   <213> Homo sapiens
<400> 554
   gagtgtgaat catctgtgaa tttcacatca ctcatttaac 40
<210> 555
   <211> 40
   <212> DNA
   <213> Homo sapiens
<400> 555
   gagtgtgaat catctgtgaa ccagctgaaa gaaacattgg 40
<210> 556
   <211> 40
   <212> DNA
   <213> Homo sapiens
<400> 556
   tcatcaatgc cccgaccttg gttcatgaac acattgaggt 40
<210> 557
   <211> 40
   <212> DNA
   <213> Homo sapiens
<400> 557
   gcatttctga gaaggctcgg gtcctctccc gcaggggctg 40
<210> 558
   <211> 40
   <212> DNA
   <213> Homo sapiens
<400> 558
   gcatttctga gaaggctcgg gggctggctt tgacctacag 40
<210> 559
   <211> 40
   <212> DNA
   <213> Homo sapiens
<400> 559
   gccagtccag agccctcaag ttcttcttct cagctcttgt 40
<210> 560
   <211> 40
   <212> DNA
   <213> Homo sapiens
<400> 560
   gccagtccag agccctcaag ctcttgtggc catggagaag 40
<210> 561
   <211> 40
   <212> DNA
   <213> Homo sapiens
<400> 561
   tggccgaggc gctgaccaag accttactca ggggatcctc 40
<210> 562
   <211> 40
   <212> DNA
   <213> Homo sapiens
<400> 562
   tggccgaggc gctgaccaag gctgagggca gaggaggcct 40
<210> 563
   <211> 40
   <212> DNA
   <213> Homo sapiens
<400> 563
   tctacttggt gggcttcttg catttatttt gttttaggat 40
<210> 564
   <211> 40
   <212> DNA
   <213> Homo sapiens
<400> 564
   tctacttggt gggcttcttg gatttgtttg gtgtcagcat 40
<210> 565
   <211> 40
   <212> DNA
   <213> Homo sapiens
<400> 565
   gctcctgctc agtatatccg tttttatctg ctttcttcag 40
<210> 566
   <211> 40
   <212> DNA
   <213> Homo sapiens
<400> 566
   gctcctgctc agtatatccg atacacacca tctcagcaag 40
<210> 567
   <211> 40
   <212> DNA
   <213> Homo sapiens
<400> 567
   gaaatagggc acagatccag tttttcttta attttagact 40
<210> 568
   <211> 40
   <212> DNA
   <213> Homo sapiens
<400> 568
   gaaatagggc acagatccag actgtgatag atgccaacat 40
<210> 569
   <211> 40
   <212> DNA
   <213> Homo sapiens
<400> 569
   gcaacctgtg ttttacaaag gttttatttt ttagatggtg 40
<210> 570
   <211> 40
   <212> DNA
   <213> Homo sapiens
<400> 570
   gcaacctgtg ttttacaaag atggtgtcct acagcagcca 40
<210> 571
   <211> 40
   <212> DNA
   <213> Homo sapiens
<400> 571
   cccctgaagt actagcaaag catgttaata ttttataggt 40
<210> 572
   <211> 40
   <212> DNA
   <213> Homo sapiens
<400> 572
   cccctgaagt actagcaaag gtacaggcaa ttaaacttct 40
<210> 573
   <211> 40
   <212> DNA
   <213> Homo sapiens
<400> 573
   gttcctcact ttgaatgagg tgtttttgat tctgcaggtg 40
<210> 574
   <211> 40
   <212> DNA
   <213> Homo sapiens
<400> 574
   gttcctcact ttgaatgagg gtgcatggta ctcagtaggt 40
<210> 575
   <211> 40
   <212> DNA
   <213> Homo sapiens
<400> 575
   tcttggaagg cagagaaaag atatttctag agcatttggg 40
<210> 576
   <211> 40
   <212> DNA
   <213> Homo sapiens
<400> 576
   tcttggaagg cagagaaaag tctacctcga gacctatggc 40
<210> 577
   <211> 40
   <212> DNA
   <213> Homo sapiens
<400> 577
   aacccggaga gaaaagggag tttgttttta ggtcagagtc 40
<210> 578
   <211> 40
   <212> DNA
   <213> Homo sapiens
<400> 578
   aacccggaga gaaaagggag caactgatgt tgccatgcag 40
<210> 579
   <211> 40
   <212> DNA
   <213> Homo sapiens
<400> 579
   aatcttcccc aagatgtatg ttctatgttc cagcagagat 40
<210> 580
   <211> 40
   <212> DNA
   <213> Homo sapiens
<400> 580
   aatcttcccc aagatgtatg gttatatcaa tcagtgaaaa 40
<210> 581
   <211> 40
   <212> DNA
   <213> Homo sapiens
<400> 581
   ctctcttgtc agacaagcag ttgtctcttc caggtaatgg 40
<210> 582
   <211> 40
   <212> DNA
   <213> Homo sapiens
<400> 582
   ctctcttgtc agacaagcag gtaatggaga ctatacagtg 40
<210> 583
   <211> 40
   <212> DNA
   <213> Homo sapiens
<400> 583
   gcagagctgt ggcttaccag tccctccttg ttccagatgt 40
<210> 584
   <211> 40
   <212> DNA
   <213> Homo sapiens
<400> 584
   gcagagctgt ggcttaccag atgtggcaaa atctggcaaa 40
<210> 585
   <211> 40
   <212> DNA
   <213> Homo sapiens
<400> 585
   tgcaggagac cggcttttgg gtccccttct tatacccctc 40
<210> 586
   <211> 40
   <212> DNA
   <213> Homo sapiens
<400> 586
   tgcaggagac cggcttttgg atactgctaa tcagtcctag 40
<210> 587
   <211> 40
   <212> DNA
   <213> Homo sapiens
<400> 587
   tcttgccaga gctgcccacg ctctccaccc tcagctgcct 40
<210> 588
   <211> 40
   <212> DNA
   <213> Homo sapiens
<400> 588
   tcttgccaga gctgcccacg cttctttcct tgctgctgga 40
<210> 589
   <211> 40
   <212> DNA
   <213> Homo sapiens
<400> 589
   gcaggctgcc cgggactctg gctctctttc tctcagggga 40
<210> 590
   <211> 40
   <212> DNA
   <213> Homo sapiens
<400> 590
   gcaggctgcc cgggactctg gggacatgaa gggacagtgg 40
<210> 591
   <211> 40
   <212> DNA
   <213> Homo sapiens
<400> 591
   gccagtccag agccctcaag tctttaccag acttgcaggg 40
<210> 592
   <211> 40
   <212> DNA
   <213> Homo sapiens
<400> 592
   ttcccactgg tcgcctgcag gtatttctct ttagactggc 40
<210> 593
   <211> 40
   <212> DNA
   <213> Homo sapiens
<400> 593
   ttcccactgg tcgcctgcag actggcatcc ttcgaaccaa 40
<210> 594
   <211> 40
   <212> DNA
   <213> Homo sapiens
<400> 594
   tgtaaatggg gaagcgctgt tttctacaga ctgccattgc 40
<210> 595
   <211> 40
   <212> DNA
   <213> Homo sapiens
<400> 595
   tgtaaatggg gaagcgctgt gcgacgactg taagggcaag 40
<210> 596
   <211> 40
   <212> DNA
   <213> Homo sapiens
<400> 596
   tcaatgcaaa tatcatcatg gattttcttc ctaaatttct 40
<210> 597
   <211> 40
   <212> DNA
   <213> Homo sapiens
<400> 597
   tcaatgcaaa tatcatcatg cctgaatttg aaaccaagtg 40
<210> 598
   <211> 40
   <212> DNA
   <213> Homo sapiens
<400> 598
   acaaatcaac tggaaagcaa ttactgtttt caggcagtct 40
<210> 599
   <211> 40
   <212> DNA
   <213> Homo sapiens
<400> 599
   acaaatcaac tggaaagcaa gcagtctgca gaactaaata 40
<210> 600
   <211> 40
   <212> DNA
   <213> Homo sapiens
<400> 600
   caaagcgccc agccctgggg gctggaggct gagccccggc 40
<210> 601
   <211> 40
   <212> DNA
   <213> Homo sapiens
<400> 601
   caaagcgccc agccctgggg atccggaaac ggcactcaag 40
<210> 602
   <211> 40
   <212> DNA
   <213> Homo sapiens
<400> 602
   tgacacagcc ctgcaggcag gacctttccc cctccctagt 40
<210> 603
   <211> 40
   <212> DNA
   <213> Homo sapiens
<400> 603
   agttgccatt ccattacatg tctttacttt cctgaagctt 40
<210> 604
   <211> 40
   <212> DNA
   <213> Homo sapiens
<400> 604
   agttgccatt ccattacatg cttcaagctt agatgatgtt 40
<210> 605
   <211> 40
   <212> DNA
   <213> Homo sapiens
<400> 605
   actgattaaa aatcttggtg gtgatttctc tttgccagtt 40
<210> 606
   <211> 40
   <212> DNA
   <213> Homo sapiens
<400> 606
   actgattaaa aatcttggtg ttgatacaat acaaatggaa 40
<210> 607
   <211> 40
   <212> DNA
   <213> Homo sapiens
<400> 607
   aggctattgt tgcagaccgg gctgttttcc ttacagatgg 40
<210> 608
   <211> 40
   <212> DNA
   <213> Homo sapiens
<400> 608
   aggctattgt tgcagaccgg atggtagaaa tcctattcca 40
<210> 609
   <211> 40
   <212> DNA
   <213> Homo sapiens
<400> 609
   cctttcaaga aaacaaaaag tcgctttttc cagtggcggt 40
<210> 610
   <211> 40
   <212> DNA
   <213> Homo sapiens
<400> 610
   cctttcaaga aaacaaaaag gcaaagtgct cttaggagaa 40
<210> 611
   <211> 40
   <212> DNA
   <213> Homo sapiens
<400> 611
   acacggagct caagaaacag tttcttccag aactaccagc 40
<210> 612
   <211> 40
   <212> DNA
   <213> Homo sapiens
<400> 612
   acacggagct caagaaacag atggcaaacc aaaaagattt 40
<210> 613
   <211> 40
   <212> DNA
   <213> Homo sapiens
<400> 613
   gacttcgaac atttaaacag tgtgttacag gtagaagaga 40
<210> 614
   <211> 40
   <212> DNA
   <213> Homo sapiens
<400> 614
   gacttcgaac atttaaacag aggtatcctg ggcaagtcat 40
<210> 615
   <211> 40
   <212> DNA
   <213> Homo sapiens
<400> 615
   accacgaagg gtcacacaag tctatttggt ccaggggcag 40
<210> 616
   <211> 40
   <212> DNA
   <213> Homo sapiens
<400> 616
   accacgaagg gtcacacaag gggcagcctc acctgggcat 40
<210> 617
   <211> 40
   <212> DNA
   <213> Homo sapiens
<400> 617
   ttaacaaaca cgtgaatcta cagtgtttgg ccagcgcttg 40
<210> 618
   <211> 40
   <212> DNA
   <213> Homo sapiens
<400> 618
   tgctggcaca ccctgtggag cagtgtttgg ccagcgcttg 40
<210> 619
   <211> 40
   <212> DNA
   <213> Homo sapiens
<400> 619
   cgccccaggg caagcgaaag gtgttccttg acttgtgcgt 40
<210> 620
   <211> 40
   <212> DNA
   <213> Homo sapiens
<400> 620
   cgccccaggg caagcgaaag gtgatcaaca ctccggaaat 40
<210> 621
   <211> 40
   <212> DNA
   <213> Homo sapiens
<400> 621
   tggtacaact tcaggaaaag tctgtttgtt ttgcagtgtt 40
<210> 622
   <211> 40
   <212> DNA
   <213> Homo sapiens
<400> 622
   tggtacaact tcaggaaaag tgtttagccc tccaggccca 40
<210> 623
   <211> 40
   <212> DNA
   <213> Homo sapiens
<400> 623
   tggatttgct cggcttttga ttttgattcc agccttccgc 40
<210> 624
   <211> 40
   <212> DNA
   <213> Homo sapiens
<400> 624
   tggatttgct cggcttttga ctggaccgag tgactactat 40
<210> 625
   <211> 40
   <212> DNA
   <213> Homo sapiens
<400> 625
   gatgaggacc cccacatagg tttccaaacc aggatggcca 40
<210> 626
   <211> 40
   <212> DNA
   <213> Homo sapiens
<400> 626
   gatgaggacc cccacatagg gatggccata gcagccacaa 40
<210> 627
   <211> 40
   <212> DNA
   <213> Homo sapiens
<400> 627
   tgttttaaat tccatagcag ctatttctac agtaaaccat 40
<210> 628
   <211> 40
   <212> DNA
   <213> Homo sapiens
<400> 628
   tgttttaaat tccatagcag cattttcatc aatagctatt 40
<210> 629
   <211> 40
   <212> DNA
   <213> Homo sapiens
<400> 629
   gctgggatgt tagggctcag cctgtcgttc caggacccag 40
<210> 630
   <211> 40
   <212> DNA
   <213> Homo sapiens
<400> 630
   gctgggatgt tagggctcag ggaagaaaag tcagaagacc 40
<210> 631
   <211> 40
   <212> DNA
   <213> Homo sapiens
<400> 631
   ctggttattg caaattaaag ctctttgccg tcccctccta 40
<210> 632
   <211> 40
   <212> DNA
   <213> Homo sapiens
<400> 632
   ctggttattg caaattaaag gtcttcaacc ccaggattgg 40
<210> 633
   <211> 40
   <212> DNA
   <213> Homo sapiens
<400> 633
   ggtcatgcta atgagacagg tctgttgttt ttttagattt 40
<210> 634
   <211> 40
   <212> DNA
   <213> Homo sapiens
<400> 634
   ggtcatgcta atgagacagg atttgatgag gcgccaagaa 40
<210> 635
   <211> 40
   <212> DNA
   <213> Homo sapiens
<400> 635
   gtcagcattt gcagactttg tttcttttgg cagatggaga 40
<210> 636
   <211> 40
   <212> DNA
   <213> Homo sapiens
<400> 636
   gtcagcattt gcagactttg atggagatgg acacatggat 40
<210> 637
   <211> 40
   <212> DNA
   <213> Homo sapiens
<400> 637
   gcagagctgt ggcttaccag acttctccct ttccaggccc 40
<210> 638
   <211> 40
   <212> DNA
   <213> Homo sapiens
<400> 638
   tgcagctggc ccccgcccag gtcttttctc tcccacaggc 40
<210> 639
   <211> 40
   <212> DNA
   <213> Homo sapiens
<400> 639
   tgcagctggc ccccgcccag gcccctgtct cccagcctga 40
<210> 640
   <211> 40
   <212> DNA
   <213> Homo sapiens
<400> 640
   cacagcaagc accttctgag ttcttttctt atttcaggct 40
<210> 641
   <211> 40
   <212> DNA
   <213> Homo sapiens
<400> 641
   cacagcaagc accttctgag gctgatttgg agcaatataa 40
<210> 642
   <211> 40
   <212> DNA
   <213> Homo sapiens
<400> 642
   tcacacctgt aggaactgag tgtattatga tacaggaaga 40
<210> 643
   <211> 40
   <212> DNA
   <213> Homo sapiens
<400> 643
   tcacacctgt aggaactgag gaagaagtta tggcagaaga 40
<210> 644
   <211> 40
   <212> DNA
   <213> Homo sapiens
<400> 644
   aaaattgact atggcaacaa tttttgcttt acagaatcct 40
<210> 645
   <211> 40
   <212> DNA
   <213> Homo sapiens
<400> 645
   aaaattgact atggcaacaa aatccttgag cttgatttga 40
<210> 646
   <211> 40
   <212> DNA
   <213> Homo sapiens
<400> 646
   acacggagct caagaaacag aactaccagc agatctagaa 40
<210> 647
   <211> 40
   <212> DNA
   <213> Homo sapiens
<400> 647
   gggaggaaaa gtaattaatg tttttgtttt tcttttttag 40
<210> 648
   <211> 40
   <212> DNA
   <213> Homo sapiens
<400> 648
   gggaggaaaa gtaattaatg gaagttatag aactaaccaa 40
<210> 649
   <211> 40
   <212> DNA
   <213> Homo sapiens
<400> 649
   agaaggagct gcagggccag tgtttccttc acagaatgtg 40
<210> 650
   <211> 40
   <212> DNA
   <213> Homo sapiens
<400> 650
   agaaggagct gcagggccag aatgtggagg ctgtggaccc 40
<210> 651
   <211> 40
   <212> DNA
   <213> Homo sapiens
<400> 651
   gctctggaga atctcaataa ggtttttctt cctttagggc 40
<210> 652
   <211> 40
   <212> DNA
   <213> Homo sapiens
<400> 652
   gctctggaga atctcaataa ggctctccta gcagacattg 40
<210> 653
   <211> 40
   <212> DNA
   <213> Homo sapiens
<400> 653
   catgcaatga acccaaaagg ttgattccag tgctaaaagg 40
<210> 654
   <211> 40
   <212> DNA
   <213> Homo sapiens
<400> 654
   catgcaatga acccaaaagg tcactctgag aggagtgata 40
<210> 655
   <211> 40
   <212> DNA
   <213> Homo sapiens
<400> 655
   tgcttccgga acagtgacag ccccatctct gcccctgcta 40
<210> 656
   <211> 40
   <212> DNA
   <213> Homo sapiens
<400> 656
   tgcttccgga acagtgacag ggacttcgct tttgtggcaa 40
<210> 657
   <211> 40
   <212> DNA
   <213> Homo sapiens
<400> 657
   tgggtttcag caagagaaca ttgtttttct gattttctag 40
<210> 658
   <211> 40
   <212> DNA
   <213> Homo sapiens
<400> 658
   tgggtttcag caagagaaca ctggcagcct caggaaacaa 40
<210> 659
   <211> 40
   <212> DNA
   <213> Homo sapiens
<400> 659
   aggacatgga tttggtagag tgctctaatt tttgttttaa 40
<210> 660
   <211> 40
   <212> DNA
   <213> Homo sapiens
<400> 660
   aggacatgga tttggtagag gtgaatgaag cttttgctcc 40
<210> 661
   <211> 40
   <212> DNA
   <213> Homo sapiens
<400> 661
   atcacaaccg gaaccgcagg ctccttctgc cctgcccgca 40
<210> 662
   <211> 40
   <212> DNA
   <213> Homo sapiens
<400> 662
   atcacaaccg gaaccgcagg ctcatgatgg agcagtccaa 40
<210> 663
   <211> 40
   <212> DNA
   <213> Homo sapiens
<400> 663
   caggaagcag ctagtctttt atgtttattc tctttgtaga 40
<210> 664
   <211> 40
   <212> DNA
   <213> Homo sapiens
<400> 664
   caggaagcag ctagtctttt aggtaagaag tatggagaga 40
<210> 665
   <211> 40
   <212> DNA
   <213> Homo sapiens
<400> 665
   gaaccaatgg aatggagaag gcacaggcgt tttgcaaagg 40
<210> 666
   <211> 40
   <212> DNA
   <213> Homo sapiens
<400> 666
   gaaccaatgg aatggagaag gtcctatggc cgggctccga 40
<210> 667
   <211> 40
   <212> DNA
   <213> Homo sapiens
<400> 667
   tgcttgtaaa attgaaatgg tgcttttaat tattatagtt 40
<210> 668
   <211> 40
   <212> DNA
   <213> Homo sapiens
<400> 668
   tgcttgtaaa attgaaatgg ttgactacaa agaagaatat 40
<210> 669
   <211> 40
   <212> DNA
   <213> Homo sapiens
<400> 669
   ccacctcacc atcacccagg cccctccaca gggcccctct 40
<210> 670
   <211> 40
   <212> DNA
   <213> Homo sapiens
<400> 670
   cgtctccatg accatgcaag gtgtagacgc agtgctcccc 40
<210> 671
   <211> 40
   <212> DNA
   <213> Homo sapiens
<400> 671
   cgtctccatg accatgcaag gcttcctgaa ctactacgat 40
<210> 672
   <211> 40
   <212> DNA
   <213> Homo sapiens
<400> 672
   aggaggcaat taaggcaaag gccctttccc tgctacaggt 40
<210> 673
   <211> 40
   <212> DNA
   <213> Homo sapiens
<400> 673
   aggaggcaat taaggcaaag gtggggcagt acgtgtcccg 40
<210> 674
   <211> 40
   <212> DNA
   <213> Homo sapiens
<400> 674
   ttacctccga aggatcgtgg ttctctttgt agggtctgcc 40
<210> 675
   <211> 40
   <212> DNA
   <213> Homo sapiens
<400> 675
   ttacctccga aggatcgtgg ggtctgccac aaggtacctc 40
<210> 676
   <211> 40
   <212> DNA
   <213> Homo sapiens
<400> 676
   aataagccct cagatggcag cctgtctgac ctgtgggccc 40
<210> 677
   <211> 40
   <212> DNA
   <213> Homo sapiens
<400> 677
   aataagccct cagatggcag gcccaagtat ctggtggtga 40
<210> 678
   <211> 40
   <212> DNA
   <213> Homo sapiens
<400> 678
   gcctggacct gtacttggag gtgcagatcc aggcgtacct 40
<210> 679
   <211> 40
   <212> DNA
   <213> Homo sapiens
<400> 679
   gcctggacct gtacttggag aggcttcggc tcaccgagag 40
<210> 680
   <211> 40
   <212> DNA
   <213> Homo sapiens
<400> 680
   ctgtaactac tagcccacag tttctttttt attcaaatag 40
<210> 681
   <211> 40
   <212> DNA
   <213> Homo sapiens
<400> 681
   ctgtaactac tagcccacag agtgacatga tgagggagca 40
<210> 682
   <211> 40
   <212> DNA
   <213> Homo sapiens
<400> 682
   ctctcaatgc agctttacag ttttcctgca gattgttcaa 40
<210> 683
   <211> 40
   <212> DNA
   <213> Homo sapiens
<400> 683
   ctctcaatgc agctttacag gcttcaatgg ctgagaatag 40
<210> 684
   <211> 40
   <212> DNA
   <213> Homo sapiens
<400> 684
   ggagcagttc cagaagactg ctgcttctcc atagggacca 40
<210> 685
   <211> 40
   <212> DNA
   <213> Homo sapiens
<400> 685
   ggagcagttc cagaagactg ggaccattgt tgtggaaggc 40
<210> 686
   <211> 40
   <212> DNA
   <213> Homo sapiens
<400> 686
   cctgctggac cattcttacg ttgtctcccc ctgttcctaa 40
<210> 687
   <211> 40
   <212> DNA
   <213> Homo sapiens
<400> 687
   cctgctggac cattcttacg atttcaacca gctggatggt 40
<210> 688
   <211> 40
   <212> DNA
   <213> Homo sapiens
<400> 688
   ggattttgat aatgaagaag ttgtgctctt tttccagagg 40
<210> 689
   <211> 40
   <212> DNA
   <213> Homo sapiens
<400> 689
   ggattttgat aatgaagaag aggaacagtc agtccctccc 40
<210> 690
   <211> 40
   <212> DNA
   <213> Homo sapiens
<400> 690
   cgtctccatg accatgcaag ggcaggtgta gacgcagtgc 40
<210> 691
   <211> 40
   <212> DNA
   <213> Homo sapiens
<400> 691
   agtgccagct gcgggcccgg ctctcaccag tgacgccctc 40
<210> 692
   <211> 40
   <212> DNA
   <213> Homo sapiens
<400> 692
   agtgccagct gcgggcccgg gaatcgtaca agtacttccc 40
<210> 693
   <211> 40
   <212> DNA
   <213> Homo sapiens
<400> 693
   aacttacttt gtttatgatg cttttatttt agattcagag 40
<210> 694
   <211> 40
   <212> DNA
   <213> Homo sapiens
<400> 694
   aacttacttt gtttatgatg agtatgaaga tggtgatctg 40
<210> 695
   <211> 40
   <212> DNA
   <213> Homo sapiens
<400> 695
   atcatagccc acatgtccag tttttctttc taggtaaaag 40
<210> 696
   <211> 40
   <212> DNA
   <213> Homo sapiens
<400> 696
   atcatagccc acatgtccag gtaaaagcag cgtttaatga 40
<210> 697
   <211> 40
   <212> DNA
   <213> Homo sapiens
<400> 697
   tgactccgct gctcgccatg actttcagga ttaagcgatt 40
<210> 698
   <211> 40
   <212> DNA
   <213> Homo sapiens
<400> 698
   tgactccgct gctcgccatg tcttctcaca agactttcag 40
<210> 699
   <211> 40
   <212> DNA
   <213> Homo sapiens
<400> 699
   ccaagcacct gaaacagcag tttgcaggct tctattttag 40
<210> 700
   <211> 40
   <212> DNA
   <213> Homo sapiens
<400> 700
   ccaagcacct gaaacagcag atgctgaaaa agttcacttc 40
<210> 701
   <211> 40
   <212> DNA
   <213> Homo sapiens
<400> 701
   cgagctgttg gcatccttgg tttcttgtcc acaggagaag 40
<210> 702
   <211> 40
   <212> DNA
   <213> Homo sapiens
<400> 702
   cgagctgttg gcatccttgg gacctgccgc tgccaagcca 40
<210> 703
   <211> 40
   <212> DNA
   <213> Homo sapiens
<400> 703
   gctttctacg gaacatcaat gagcttctgt ctgcacacag 40
<210> 704
   <211> 40
   <212> DNA
   <213> Homo sapiens
<400> 704
   gctttctacg gaacatcaat gagtacctgg ccgtagtcga 40
<210> 705
   <211> 40
   <212> DNA
   <213> Homo sapiens
<400> 705
   caggaatacc tgcagataag atttcacaga atattcgcta 40
<210> 706
   <211> 40
   <212> DNA
   <213> Homo sapiens
<400> 706
   caggaatacc tgcagataag atgatagtta ctgatatata 40
<210> 707
   <211> 40
   <212> DNA
   <213> Homo sapiens
<400> 707
   ctacaccaag aagagaggac ctcttccctc gcgcagaatc 40
<210> 708
   <211> 40
   <212> DNA
   <213> Homo sapiens
<400> 708
   ctacaccaag aagagaggac agaggccaga cttcacagac 40
<210> 709
   <211> 40
   <212> DNA
   <213> Homo sapiens
<400> 709
   cggaggctgt ctcctctcag acttcctctc tcccaccagg 40
<210> 710
   <211> 40
   <212> DNA
   <213> Homo sapiens
<400> 710
   cggaggctgt ctcctctcag gaaatgctgc gctgcatttg 40
<210> 711
   <211> 40
   <212> DNA
   <213> Homo sapiens
<400> 711
   tcctgctgga gccacccaag ctttttcttc ttcagaaaag 40
<210> 712
   <211> 40
   <212> DNA
   <213> Homo sapiens
<400> 712
   tcctgctgga gccacccaag aaaagtgtga tgaagaccac 40
<210> 713
   <211> 40
   <212> DNA
   <213> Homo sapiens
<400> 713
   accaagcata cttccagatg ttctctctat ttaagggtca 40
<210> 714
   <211> 40
   <212> DNA
   <213> Homo sapiens
<400> 714
   accaagcata cttccagatg ggtcaatatt ctctcgagtt 40
<210> 715
   <211> 40
   <212> DNA
   <213> Homo sapiens
<400> 715
   cgggccgccc ccctgcccgg tgttcttctg ggcagtgcaa 40
<210> 716
   <211> 40
   <212> DNA
   <213> Homo sapiens
<400> 716
   cgggccgccc ccctgcccgg aggccggtcc ctgccaaggg 40
<210> 717
   <211> 40
   <212> DNA
   <213> Homo sapiens
<400> 717
   ggaggactgg ggtctgcaga catttcttgc agacagcacc 40
<210> 718
   <211> 40
   <212> DNA
   <213> Homo sapiens
<400> 718
   ggaggactgg ggtctgcaga acagcacctt gtattctggc 40
<210> 719
   <211> 40
   <212> DNA
   <213> Homo sapiens
<400> 719
   ctgccccctg cgccacacgg cctctttccc tgcagtgatg 40
<210> 720
   <211> 40
   <212> DNA
   <213> Homo sapiens
<400> 720
   ctgccccctg cgccacacgg tgatggttca ttcgcatatg 40
<210> 721
   <211> 40
   <212> DNA
   <213> Homo sapiens
<400> 721
   tgtaaatggg gaagcgctgt actgccattg ctatgcacgg 40
<210> 722
   <211> 40
   <212> DNA
   <213> Homo sapiens
<400> 722
   ctgatgaaaa ctactacaag cagacacctt acaggccagg 40
<210> 723
   <211> 40
   <212> DNA
   <213> Homo sapiens
<400> 723
   ctgatgaaaa ctactacaag gcccagaccc atggaaagtg 40
<210> 724
   <211> 40
   <212> DNA
   <213> Homo sapiens
<400> 724
   ttcagctgcc cctgaagaag aaacatgttc tccttccttc 40
<210> 725
   <211> 40
   <212> DNA
   <213> Homo sapiens
<400> 725
   ttcagctgcc cctgaagaag gaatgagtag cgacagtgac 40
<210> 726
   <211> 40
   <212> DNA
   <213> Homo sapiens
<400> 726
   tcccgaagcc acctcatgag cctctgcctt cccccaggtc 40
<210> 727
   <211> 40
   <212> DNA
   <213> Homo sapiens
<400> 727
   tcccgaagcc acctcatgag gtcgggcagt gtgatggagc 40
<210> 728
   <211> 40
   <212> DNA
   <213> Homo sapiens
<400> 728
   ccccggtgcg taaggaggag cctgcccccc tttggccctg 40
<210> 729
   <211> 40
   <212> DNA
   <213> Homo sapiens
<400> 729
   ccccggtgcg taaggaggag gaggacaatc ccaaggggga 40
<210> 730
   <211> 40
   <212> DNA
   <213> Homo sapiens
<400> 730
   agctggagaa aaaccttctt tttcttccag aactaccagc 40
<210> 731
   <211> 40
   <212> DNA
   <213> Homo sapiens
<400> 731
   agctggagaa aaaccttctt atggcaaacc aaaaagattt 40
<210> 732
   <211> 40
   <212> DNA
   <213> Homo sapiens
<400> 732
   ttcgttggca gcttctgctg agaccctgac ccccaccccc 40
<210> 733
   <211> 40
   <212> DNA
   <213> Homo sapiens
<400> 733
   ttcgttggca gcttctgctg cgtccacaga gaccctgacc 40
<210> 734
   <211> 40
   <212> DNA
   <213> Homo sapiens
<400> 734
   gtgccaacga ggaccaggag ttctttattt cagatggaac 40
<210> 735
   <211> 40
   <212> DNA
   <213> Homo sapiens
<400> 735
   gtgccaacga ggaccaggag atggaactag aagcattacg 40
<210> 736
   <211> 40
   <212> DNA
   <213> Homo sapiens
<400> 736
   cctcacgatg caaggccacg agttcatgtc ccacagggag 40
<210> 737
   <211> 40
   <212> DNA
   <213> Homo sapiens
<400> 737
   cctcacgatg caaggccacg ggagaagctg tgtacactgt 40
<210> 738
   <211> 40
   <212> DNA
   <213> Homo sapiens
<400> 738
   aaaaataaag cctttcccag gcccagaccc atggaaagtg 40
<210> 739
   <211> 40
   <212> DNA
   <213> Homo sapiens
<400> 739
   cgaggatgaa gacagagcag gtgaccaaga aaaaaaagaa 40
<210> 740
   <211> 40
   <212> DNA
   <213> Homo sapiens
<400> 740
   cgaggatgaa gacagagcag tacaggtgac caagaaaaaa 40
<210> 741
   <211> 40
   <212> DNA
   <213> Homo sapiens
<400> 741
   aaaatgggct cagcagttag ggttttttgt tgtttgtttg 40
<210> 742
   <211> 40
   <212> DNA
   <213> Homo sapiens
<400> 742
   aaaatgggct cagcagttag accttttcac agatgctgct 40
<210> 743
   <211> 40
   <212> DNA
   <213> Homo sapiens
<400> 743
   aagcactggc ccagtgtcag gagccagatt ctgtgcgaga 40
<210> 744
   <211> 40
   <212> DNA
   <213> Homo sapiens
<400> 744
   aagcactggc ccagtgtcag aaggagccag attctgtgcg 40
<210> 745
   <211> 40
   <212> DNA
   <213> Homo sapiens
<400> 745
   ccactctcac aatgacccag gaggaccccc ggcggcgctt 40
<210> 746
   <211> 40
   <212> DNA
   <213> Homo sapiens
<400> 746
   ccactctcac aatgacccag gctggatcaa gacctttgac 40
<210> 747
   <211> 40
   <212> DNA
   <213> Homo sapiens
<400> 747
   cctttacttg gggctctcag caactgatgt tgccatgcag 40
<210> 748
   <211> 40
   <212> DNA
   <213> Homo sapiens
<400> 748
   actcagatgc cgaaaactcg ccctcagtct gaggttctgt 40
<210> 749
   <211> 40
   <212> DNA
   <213> Homo sapiens
<400> 749
   actcagatgc cgaaaactcg tgcatggagc ccatggagac 40
<210> 750
   <211> 40
   <212> DNA
   <213> Homo sapiens
<400> 750
   gcctactctt aaccattagg gtggataggc atgtagacct 40
<210> 751
   <211> 40
   <212> DNA
   <213> Homo sapiens
<400> 751
   tggagtgcgg atttgcaaca cttgcttcct tctcccacat 40
<210> 752
   <211> 40
   <212> DNA
   <213> Homo sapiens
<400> 752
   tggagtgcgg atttgcaaca atcaaagatc tgcgagacca 40
<210> 753
   <211> 40
   <212> DNA
   <213> Homo sapiens
<400> 753
   caactggagt tcattttcag gttttttgac agactatgta 40
<210> 754
   <211> 40
   <212> DNA
   <213> Homo sapiens
<400> 754
   caactggagt tcattttcag actatgtatg agcacttggg 40
<210> 755
   <211> 40
   <212> DNA
   <213> Homo sapiens
<400> 755
   caatgtgttg accatcgcag tccccctaca gccctgttca 40
<210> 756
   <211> 40
   <212> DNA
   <213> Homo sapiens
<400> 756
   caatgtgttg accatcgcag cctctcctgc caacttacag 40
<210> 757
   <211> 40
   <212> DNA
   <213> Homo sapiens
<400> 757
   cagctgctct caggagagag tggactggct ctgtaggtac 40
<210> 758
   <211> 40
   <212> DNA
   <213> Homo sapiens
<400> 758
   cagctgctct caggagagag gtacaaagaa gacccctggc 40
<210> 759
   <211> 40
   <212> DNA
   <213> Homo sapiens
<400> 759
   tctctagtgg gcccttctag ttctacaagg taaaactcta 40
<210> 760
   <211> 40
   <212> DNA
   <213> Homo sapiens
<400> 760
   tctctagtgg gcccttctag gaatgaccaa aagaagacaa 40
<210> 761
   <211> 40
   <212> DNA
   <213> Homo sapiens
<400> 761
   agctccgaga gggcaaggag ctccctccct cctagaaatg 40
<210> 762
   <211> 40
   <212> DNA
   <213> Homo sapiens
<400> 762
   agctccgaga gggcaaggag aaatgtgtcc actactggcc 40
<210> 763
   <211> 40
   <212> DNA
   <213> Homo sapiens
<400> 763
   gacgtggcag ctcatgtgag cattgtgtcg ttacaggctt 40
<210> 764
   <211> 40
   <212> DNA
   <213> Homo sapiens
<400> 764
   gacgtggcag ctcatgtgag gcttcagtgt catttgagga 40
<210> 765
   <211> 40
   <212> DNA
   <213> Homo sapiens
<400> 765
   aaggaagaac aagactttgt ttagtgtgac tctggatcca 40
<210> 766
   <211> 40
   <212> DNA
   <213> Homo sapiens
<400> 766
   aatgttaagg agtcatcaag ttagtgtgac tctggatcca 40
<210> 767
   <211> 40
   <212> DNA
   <213> Homo sapiens
<400> 767
   aggagaacac cttatttcag cttttatttt tatgtgataa 40
<210> 768
   <211> 40
   <212> DNA
   <213> Homo sapiens
<400> 768
   aggagaacac cttatttcag aaaaggtgta ccatacctga 40
<210> 769
   <211> 40
   <212> DNA
   <213> Homo sapiens
<400> 769
   ctgatgaaaa ctactacaag acaccttaca ggccaggaga 40
<210> 770
   <211> 40
   <212> DNA
   <213> Homo sapiens
<400> 770
   ggggccacca ggttggccag cggccccctt tcccagggcc 40
<210> 771
   <211> 40
   <212> DNA
   <213> Homo sapiens
<400> 771
   ggggccacca ggttggccag ggccatggct gagcacgcag 40
<210> 772
   <211> 40
   <212> DNA
   <213> Homo sapiens
<400> 772
   tgcacacgcc tctcctacag agtctcttat gctggtccca 40
<210> 773
   <211> 40
   <212> DNA
   <213> Homo sapiens
<400> 773
   tgcacacgcc tctcctacag gcagcccagc aaatcatcga 40
<210> 774
   <211> 40
   <212> DNA
   <213> Homo sapiens
<400> 774
   gagctggaga ggaaggcgag aggcagctcg tcgggagcag 40
<210> 775
   <211> 40
   <212> DNA
   <213> Homo sapiens
<400> 775
   gagctggaga ggaaggcgag gcaggcactg gtcgaccact 40
<210> 776
   <211> 40
   <212> DNA
   <213> Homo sapiens
<400> 776
   ccgcctctgc cttcggatag gtctggcccc accctggagt 40
<210> 777
   <211> 40
   <212> DNA
   <213> Homo sapiens
<400> 777
   ccgcctctgc cttcggatag gaaaggttga aagagccaac 40
<210> 778
   <211> 40
   <212> DNA
   <213> Homo sapiens
<400> 778
   tttctcatat tgctcaacag ttctttttta ggtatcatct 40
<210> 779
   <211> 40
   <212> DNA
   <213> Homo sapiens
<400> 779
   tttctcatat tgctcaacag gtatcatctt tatcagaaag 40
<210> 780
   <211> 40
   <212> DNA
   <213> Homo sapiens
<400> 780
   agtggctttg gcgtcttatg gaggcttgct tgcagagggg 40
<210> 781
   <211> 40
   <212> DNA
   <213> Homo sapiens
<400> 781
   agtggctttg gcgtcttatg ggatggagga cgaaggttgg 40
<210> 782
   <211> 40
   <212> DNA
   <213> Homo sapiens
<400> 782
   ggtgacactc aacttcacag gtctctccct ctagtgccta 40
<210> 783
   <211> 40
   <212> DNA
   <213> Homo sapiens
<400> 783
   ggtgacactc aacttcacag tgcctactgg ggccagaagc 40
<210> 784
   <211> 40
   <212> DNA
   <213> Homo sapiens
<400> 784
   tttccattgg gccaatcaag atgcctggaa tgatgtcgtc 40
<210> 785
   <211> 40
   <212> DNA
   <213> Homo sapiens
<400> 785
   gagggccacc aatgggacaa atgcctggaa tgatgtcgtc 40
<210> 786
   <211> 40
   <212> DNA
   <213> Homo sapiens
<400> 786
   agagacaaag agaagaaaaa ctcttactgt tttacagtta 40
<210> 787
   <211> 40
   <212> DNA
   <213> Homo sapiens
<400> 787
   agagacaaag agaagaaaaa ttaactctgc tgtttgctgc 40
<210> 788
   <211> 40
   <212> DNA
   <213> Homo sapiens
<400> 788
   ctcaccagcg ccatcgtcag ctctaggagt tccagagcct 40
<210> 789
   <211> 40
   <212> DNA
   <213> Homo sapiens
<400> 789
   ctcaccagcg ccatcgtcag atggcaaggt cagccccggc 40
<210> 790
   <211> 40
   <212> DNA
   <213> Homo sapiens
<400> 790
   atcaggtgct catcctgagg tgtctgtctt taatacaggt 40
<210> 791
   <211> 40
   <212> DNA
   <213> Homo sapiens
<400> 791
   atcaggtgct catcctgagg gtaatgcaga gctctcagaa 40
<210> 792
   <211> 40
   <212> DNA
   <213> Homo sapiens
<400> 792
   tctggcagcc cacgatgctg caagatggca tcgagcagca 40
<210> 793
   <211> 40
   <212> DNA
   <213> Homo sapiens
<400> 793
   tctggcagcc cacgatgctg ggagtcgggc tcacgtcctt 40
<210> 794
   <211> 40
   <212> DNA
   <213> Homo sapiens
<400> 794
   agaattttaa gatacttcag attttgtctt gtaggtttta 40
<210> 795
   <211> 40
   <212> DNA
   <213> Homo sapiens
<400> 795
   agaattttaa gatacttcag gttttatggg agaattgtag 40
<210> 796
   <211> 40
   <212> DNA
   <213> Homo sapiens
<400> 796
   ccgcctctgc cttcggatag gctttattta ggtctggccc 40
<210> 797
   <211> 40
   <212> DNA
   <213> Homo sapiens
<400> 797
   gagctagtca gactttagag gaaacagtac tgctggagca 40
<210> 798
   <211> 40
   <212> DNA
   <213> Homo sapiens
<400> 798
   aaattcttga ccaatctagg gaaacagtac tgctggagca 40
<210> 799
   <211> 40
   <212> DNA
   <213> Homo sapiens
<400> 799
   cttcatctgt ggataagcag gtcatgtcct ccaggtttct 40
<210> 800
   <211> 40
   <212> DNA
   <213> Homo sapiens
<400> 800
   cttcatctgt ggataagcag tgcaggccaa ggccccctgc 40
<210> 801
   <211> 40
   <212> DNA
   <213> Homo sapiens
<400> 801
   agtatgggat attttaaaag attgttggac cttcagatgg 40
<210> 802
   <211> 40
   <212> DNA
   <213> Homo sapiens
<400> 802
   tcattcttat ttcaatgcag attgttggac cttcagatgg 40
<210> 803
   <211> 40
   <212> DNA
   <213> Homo sapiens
<400> 803
   ctttatctgt gcatgaacag tgcaggccaa ggccccctgc 40
<210> 804
   <211> 40
   <212> DNA
   <213> Homo sapiens
<400> 804
   aagtcgtcct cttcagaaag gccggagcct caacagaaag 40
<210> 805
   <211> 40
   <212> DNA
   <213> Homo sapiens
<400> 805
   agagagaaac atccgaaaaa gccggagcct caacagaaag 40
<210> 806
   <211> 40
   <212> DNA
   <213> Homo sapiens
<400> 806
   tgggctacct taaccctggg gtatttacac agagtcggcg 40
<210> 807
   <211> 40
   <212> DNA
   <213> Homo sapiens
<400> 807
   tgggctacct taaccctggg gatttttgac cctcgtgtgg 40
<210> 808
   <211> 40
   <212> DNA
   <213> Homo sapiens
<400> 808
   gactgcccta aaaggaaaag tttactgttt agactaaaga 40
<210> 809
   <211> 40
   <212> DNA
   <213> Homo sapiens
<400> 809
   gactgcccta aaaggaaaag actaaagaag aaagacagtg 40
<210> 810
   <211> 40
   <212> DNA
   <213> Homo sapiens
<400> 810
   tgatagttgg agcggagact cataatggca gaacctgttt 40
<210> 811
   <211> 40
   <212> DNA
   <213> Homo sapiens
<400> 811
   tgatagttgg agcggagact tagcataatg gcagaacctg 40
<210> 812
   <211> 40
   <212> DNA
   <213> Homo sapiens
<400> 812
   tcattcttat ttcaatgcag agacagggtc ttgctctgtt 40
<210> 813
   <211> 40
   <212> DNA
   <213> Homo sapiens
<400> 813
   tgacggtgcc accgcggcgc ttttctccct tagatgcctt 40
<210> 814
   <211> 40
   <212> DNA
   <213> Homo sapiens
<400> 814
   tgacggtgcc accgcggcgc agaggagtct gcaatgccga 40
<210> 815
   <211> 40
   <212> DNA
   <213> Homo sapiens
<400> 815
   gcagtggctg gagatcaaag tttcaccccc agagggagcc 40
<210> 816
   <211> 40
   <212> DNA
   <213> Homo sapiens
<400> 816
   gcagtggctg gagatcaaag agagagtgtg cctattgact 40
<210> 817
   <211> 40
   <212> DNA
   <213> Homo sapiens
<400> 817
   ggacgatggg gatgagaaag atgacgagga ggataaagat 40
<210> 818
   <211> 40
   <212> DNA
   <213> Homo sapiens
<400> 818
   ggacgatggg gatgagaaag aagatgacga ggaggataaa 40
<210> 819
   <211> 40
   <212> DNA
   <213> Homo sapiens
<400> 819
   actcttatgc agtccccatg gactgaacca tcaagacacc 40
<210> 820
   <211> 40
   <212> DNA
   <213> Homo sapiens
<400> 820
   gacccatgca tcctcctgtg ctcctcccac tgcagtgggc 40
<210> 821
   <211> 40
   <212> DNA
   <213> Homo sapiens
<400> 821
   gacccatgca tcctcctgtg tgggcacagt ggctcaggga 40
<210> 822
   <211> 40
   <212> DNA
   <213> Homo sapiens
<400> 822
   catcaagcag ctgttgcaat gtttagtccc aggaagcacc 40
<210> 823
   <211> 40
   <212> DNA
   <213> Homo sapiens
<400> 823
   catcaagcag ctgttgcaat ctgcccacaa agaatccagc 40
<210> 824
   <211> 40
   <212> DNA
   <213> Homo sapiens
<400> 824
   caatcattga caatattatg accctgcatg tgatggatca 40
<210> 825
   <211> 40
   <212> DNA
   <213> Homo sapiens
<400> 825
   caatcattga caatattatg gaactgactc agcgcaagaa 40
<210> 826
   <211> 40
   <212> DNA
   <213> Homo sapiens
<400> 826
   gggacactgt gccgaatgaa cttgcttgcc ttttgtttta 40
<210> 827
   <211> 40
   <212> DNA
   <213> Homo sapiens
<400> 827
   gggacactgt gccgaatgaa cagctgcagt atctcggaag 40
<210> 828
   <211> 40
   <212> DNA
   <213> Homo sapiens
<400> 828
   tcagggggcg cgtgctgaag gagctgcctg agttcgaggg 40
<210> 829
   <211> 40
   <212> DNA
   <213> Homo sapiens
<400> 829
   tcgagccagg ctgcaaaaag gagctgcctg agttcgaggg 40
<210> 830
   <211> 40
   <212> DNA
   <213> Homo sapiens
<400> 830
   ctacaaccag agcgaggctg ggtctcacac cctccaggga 40
<210> 831
   <211> 40
   <212> DNA
   <213> Homo sapiens
<400> 831
   ctacaaccag agcgaggctg ggaatgaatg gctgcgacat 40
<210> 832
   <211> 40
   <212> DNA
   <213> Homo sapiens
<400> 832
   tccaacaagc acctctgaag tcttctcatt cacaggttaa 40
<210> 833
   <211> 40
   <212> DNA
   <213> Homo sapiens
<400> 833
   tccaacaagc acctctgaag gttaaggcta cctttccaga 40
<210> 834
   <211> 40
   <212> DNA
   <213> Homo sapiens
<400> 834
   gttcccgagg ctgtcaccag ggtgttccct caggtcaatg 40
<210> 835
   <211> 40
   <212> DNA
   <213> Homo sapiens
<400> 835
   gttcccgagg ctgtcaccag tggatactga ggctgtgtgg 40
<210> 836
   <211> 40
   <212> DNA
   <213> Homo sapiens
<400> 836
   ccagatcaac acaattgata gtcgtactct ttcagatgtc 40
<210> 837
   <211> 40
   <212> DNA
   <213> Homo sapiens
<400> 837
   ccagatcaac acaattgata atgtcagcaa tatttccaac 40
<210> 838
   <211> 40
   <212> DNA
   <213> Homo sapiens
<400> 838
   cgtcctgccc ccaactgccg ctctgtcttc cctgttccca 40
<210> 839
   <211> 40
   <212> DNA
   <213> Homo sapiens
<400> 839
   cgtcctgccc ccaactgccg cctctcagcg agaaggacac 40
<210> 840
   <211> 40
   <212> DNA
   <213> Homo sapiens
<400> 840
   tgcaggggga gcagcccaag gaggccccac cgccactgtc 40
<210> 841
   <211> 40
   <212> DNA
   <213> Homo sapiens
<400> 841
   tgcaggggga gcagcccaag ccggccagcc ctgctgagga 40
<210> 842
   <211> 40
   <212> DNA
   <213> Homo sapiens
<400> 842
   gactgcccta aaaggaaaag cagtttactg tttagactaa 40
<210> 843
   <211> 40
   <212> DNA
   <213> Homo sapiens
<400> 843
   ctatgaggcc atgactgcag tggatactga ggctgtgtgg 40
<210> 844
   <211> 40
   <212> DNA
   <213> Homo sapiens
<400> 844
   cttctcaaga tcagtctcag gtgccacgtg tgccaacgca 40
<210> 845
   <211> 40
   <212> DNA
   <213> Homo sapiens
<400> 845
   cttctcaaga tcagtctcag gaacctgaca gaacttcaca 40
<210> 846
   <211> 40
   <212> DNA
   <213> Homo sapiens
<400> 846
   accttaacaa gatttatgag acttccttta ataagtgttg 40
<210> 847
   <211> 40
   <212> DNA
   <213> Homo sapiens
<400> 847
   aatcactagg aactccagag acttccttta ataagtgttg 40
<210> 848
   <211> 40
   <212> DNA
   <213> Homo sapiens
<400> 848
   actgggcttc caccgagcag aaacagcact tcttctcagt 40
<210> 849
   <211> 40
   <212> DNA
   <213> Homo sapiens
<400> 849
   actgggcttc caccgagcag gagattacct ggggcaattg 40
<210> 850
   <211> 40
   <212> DNA
   <213> Homo sapiens
<400> 850
   ctgaagacgg gattctttag ctctccccac ctggtgcagg 40
<210> 851
   <211> 40
   <212> DNA
   <213> Homo sapiens
<400> 851
   ctgaagacgg gattctttag gttcgggagc ggatccgcat 40
<210> 852
   <211> 40
   <212> DNA
   <213> Homo sapiens
<400> 852
   atctcaggag cacctgaatg gtcccctgcc tgtgcccttc 40
<210> 853
   <211> 40
   <212> DNA
   <213> Homo sapiens
<400> 853
   cccacccctt caccctgcag gtcccctgcc tgtgcccttc 40
<210> 854
   <211> 40
   <212> DNA
   <213> Homo sapiens
<400> 854
   ggccacacgc ctctgccaag cccctctccc ctggcacaga 40
<210> 855
   <211> 40
   <212> DNA
   <213> Homo sapiens
<400> 855
   ggccacacgc ctctgccaag acattgatga gtgtgagtct 40
<210> 856
   <211> 40
   <212> DNA
   <213> Homo sapiens
<400> 856
   gagtacgagg tctccagcag cctgccctgt gcctacagcc 40
<210> 857
   <211> 40
   <212> DNA
   <213> Homo sapiens
<400> 857
   gagtacgagg tctccagcag cctcgtgtgc atcaccgggg 40
<210> 858
   <211> 40
   <212> DNA
   <213> Homo sapiens
<400> 858
   agcgcatcgc agcttccaag tacttcttca cagctcccct 40
<210> 859
   <211> 40
   <212> DNA
   <213> Homo sapiens
<400> 859
   agcgcatcgc agcttccaag gctctcctcc atcagtactt 40
<210> 860
   <211> 40
   <212> DNA
   <213> Homo sapiens
<400> 860
   tgggcagccc cccgcagacg ttggtttttc agcagacctg 40
<210> 861
   <211> 40
   <212> DNA
   <213> Homo sapiens
<400> 861
   tgggcagccc cccgcagacg ctcaacatcc tggtggatac 40
<210> 862
   <211> 40
   <212> DNA
   <213> Homo sapiens
<400> 862
   aaccaagagg acccacacag gatggtcttc acaggttctc 40
<210> 863
   <211> 40
   <212> DNA
   <213> Homo sapiens
<400> 863
   aaccaagagg acccacacag gttctcaaag ctggcccaga 40
<210> 864
   <211> 40
   <212> DNA
   <213> Homo sapiens
<400> 864
   atattccttt tatttctaag tcttttgtct taggagttaa 40
<210> 865
   <211> 40
   <212> DNA
   <213> Homo sapiens
<400> 865
   atattccttt tatttctaag gagttaaaca tagatgtagc 40
<210> 866
   <211> 40
   <212> DNA
   <213> Homo sapiens
<400> 866
   ggtcctgaac gctgtgaaat aacttcgccc ccagcttcaa 40
<210> 867
   <211> 40
   <212> DNA
   <213> Homo sapiens
<400> 867
   ggtcctgaac gctgtgaaat tgtactgtca gaacttcgcc 40
<210> 868
   <211> 40
   <212> DNA
   <213> Homo sapiens
<400> 868
   tggagcagta tgccagcaag acttttcccc caggttcttc 40
<210> 869
   <211> 40
   <212> DNA
   <213> Homo sapiens
<400> 869
   tggagcagta tgccagcaag gttcttcatg acagccagat 40
<210> 870
   <211> 40
   <212> DNA
   <213> Homo sapiens
<400> 870
   tcgtgcagac cctggagaag atctcacaga tgtgcagtct 40
<210> 871
   <211> 40
   <212> DNA
   <213> Homo sapiens
<400> 871
   tcgtgcagac cctggagaag catggcttca gtgatattaa 40
<210> 872
   <211> 40
   <212> DNA
   <213> Homo sapiens
<400> 872
   ttgaagctca gtgagaaaag ttcttctgtt tatgtcttcc 40
<210> 873
   <211> 40
   <212> DNA
   <213> Homo sapiens
<400> 873
   ttgaagctca gtgagaaaag gatgatggag atagccaaag 40
<210> 874
   <211> 40
   <212> DNA
   <213> Homo sapiens
<400> 874
   cgccctgaca cacaatcagg acttctctat ctacaggctc 40
<210> 875
   <211> 40
   <212> DNA
   <213> Homo sapiens
<400> 875
   cgccctgaca cacaatcagg gctctgttgc aagagggggt 40
<210> 876
   <211> 40
   <212> DNA
   <213> Homo sapiens
<400> 876
   ttgctggcca tcggattggg cccttcgttt caggatggat 40
<210> 877
   <211> 40
   <212> DNA
   <213> Homo sapiens
<400> 877
   ttgctggcca tcggattggg gatctatatt ggaaggcgtc 40
<210> 878
   <211> 40
   <212> DNA
   <213> Homo sapiens
<400> 878
   ccattcgaga gcatcagaag attgggagga aggaccggct 40
<210> 879
   <211> 40
   <212> DNA
   <213> Homo sapiens
<400> 879
   ccattcgaga gcatcagaag ctaaaccatt tcccaggctc 40
<210> 880
   <211> 40
   <212> DNA
   <213> Homo sapiens
<400> 880
   tcaggagcag agaggaaaag tgcatttgcc cagtataaca 40
<210> 881
   <211> 40
   <212> DNA
   <213> Homo sapiens
<400> 881
   ctcagggaag gggcagcaca tgcatttgcc cagtataaca 40
<210> 882
   <211> 40
   <212> DNA
   <213> Homo sapiens
<400> 882
   gtgtggcaag tactttcaag tatctgccct tctattacag 40
<210> 883
   <211> 40
   <212> DNA
   <213> Homo sapiens
<400> 883
   gtgtggcaag tactttcaag gccggggttt gaagtctcac 40
<210> 884
   <211> 40
   <212> DNA
   <213> Homo sapiens
<400> 884
   aaaatcattg attcccttga aattctcttt actctacctt 40
<210> 885
   <211> 40
   <212> DNA
   <213> Homo sapiens
<400> 885
   aaaatcattg attcccttga gtggttagac gatgctatta 40
<210> 886
   <211> 40
   <212> DNA
   <213> Homo sapiens
<400> 886
   tgctcagagg tgctttgaag cccatccaca acctgctcat 40
<210> 887
   <211> 40
   <212> DNA
   <213> Homo sapiens
<400> 887
   tgctcagagg tgctttgaag atgccggagg ccccgcctct 40
<210> 888
   <211> 40
   <212> DNA
   <213> Homo sapiens
<400> 888
   ccacggccac ggccgcatag ctttgtattc ctgcaggcaa 40
<210> 889
   <211> 40
   <212> DNA
   <213> Homo sapiens
<400> 889
   ccacggccac ggccgcatag gcaagcaccg gaagcacccc 40
<210> 890
   <211> 40
   <212> DNA
   <213> Homo sapiens
<400> 890
   catgccgggg ccagaggatg gctctttcca cctgtctgca 40
<210> 891
   <211> 40
   <212> DNA
   <213> Homo sapiens
<400> 891
   catgccgggg ccagaggatg ctctgcaccc gggacagtga 40
<210> 892
   <211> 40
   <212> DNA
   <213> Homo sapiens
<400> 892
   ggccaagcaa gaacaaaaag tattttcttc taggatggaa 40
<210> 893
   <211> 40
   <212> DNA
   <213> Homo sapiens
<400> 893
   ggccaagcaa gaacaaaaag tgaaatgcaa aatggaggac 40
<210> 894
   <211> 40
   <212> DNA
   <213> Homo sapiens
<400> 894
   gaagaacagg atattaatag tatgtttttg tttttaggag 40
<210> 895
   <211> 40
   <212> DNA
   <213> Homo sapiens
<400> 895
   gaagaacagg atattaatag gaggattctc tatgggagga 40
<210> 896
   <211> 40
   <212> DNA
   <213> Homo sapiens
<400> 896
   caattcagta gattcaccct caacatctga atgaattgat 40
<210> 897
   <211> 40
   <212> DNA
   <213> Homo sapiens
<400> 897
   aggtcttcct ggacctggag caacatctga atgaattgat 40
<210> 898
   <211> 40
   <212> DNA
   <213> Homo sapiens
<400> 898
   agagggcacg ggacatccag cccctctgcc cctgcaggag 40
<210> 899
   <211> 40
   <212> DNA
   <213> Homo sapiens
<400> 899
   agagggcacg ggacatccag gaggccgtgg agtcctgcct 40
<210> 900
   <211> 40
   <212> DNA
   <213> Homo sapiens
<400> 900
   agatgattga ggcagccaag ctcttttctg tcttcttggt 40
<210> 901
   <211> 40
   <212> DNA
   <213> Homo sapiens
<400> 901
   agatgattga ggcagccaag gccgtctata cccaggattg 40
<210> 902
   <211> 40
   <212> DNA
   <213> Homo sapiens
<400> 902
   gctgctctct tcaatacaag tgcttctgct tccaggatac 40
<210> 903
   <211> 40
   <212> DNA
   <213> Homo sapiens
<400> 903
   gctgctctct tcaatacaag gataccaagg gttcgagttt 40
<210> 904
   <211> 40
   <212> DNA
   <213> Homo sapiens
<400> 904
   aatagaactt ccaactactg gccctttttc agtaaagtca 40
<210> 905
   <211> 40
   <212> DNA
   <213> Homo sapiens
<400> 905
   aatagaactt ccaactactg taaagtcatc acctggcctt 40
<210> 906
   <211> 40
   <212> DNA
   <213> Homo sapiens
<400> 906
   tgttactgca gtggctacag gtctctctct tgcaggtggt 40
<210> 907
   <211> 40
   <212> DNA
   <213> Homo sapiens
<400> 907
   tgttactgca gtggctacag gtggtcctga caaccaagtc 40
<210> 908
   <211> 40
   <212> DNA
   <213> Homo sapiens
<400> 908
   acatcacaaa gcaacctgtg gggttttgtt tttgttttag 40
<210> 909
   <211> 40
   <212> DNA
   <213> Homo sapiens
<400> 909
   acatcacaaa gcaacctgtg gtgtacctga aggaaatctt 40
<210> 910
   <211> 40
   <212> DNA
   <213> Homo sapiens
<400> 910
   ggtgctggct gcctgcgaaa ccctggctgc ccctgcaggc 40
<210> 911
   <211> 40
   <212> DNA
   <213> Homo sapiens
<400> 911
   ggtgctggct gcctgcgaaa gcctgctcac cagccgccag 40
<210> 912
   <211> 40
   <212> DNA
   <213> Homo sapiens
<400> 912
   cctctctgct cgagaaggag tgtgtgtctt tttgccaaca 40
<210> 913
   <211> 40
   <212> DNA
   <213> Homo sapiens
<400> 913
   cctctctgct cgagaaggag ctggagcaga gccagaagga 40
<210> 914
   <211> 40
   <212> DNA
   <213> Homo sapiens
<400> 914
   ctggaagctc aaggtactag atttttcctc tctctgtctt 40
<210> 915
   <211> 40
   <212> DNA
   <213> Homo sapiens
<400> 915
   ctggaagctc aaggtactag tttgccaaag aaactagagt 40
<210> 916
   <211> 40
   <212> DNA
   <213> Homo sapiens
<400> 916
   cggcgatgac tcggacccag cttctctcca cagggctcct 40
<210> 917
   <211> 40
   <212> DNA
   <213> Homo sapiens
<400> 917
   cggcgatgac tcggacccag ggctccttca gtggtagatg 40
<210> 918
   <211> 40
   <212> DNA
   <213> Homo sapiens
<400> 918
   ccgccaggag aacaagccca tcccctcaca ggcagagata 40
<210> 919
   <211> 40
   <212> DNA
   <213> Homo sapiens
<400> 919
   ccgccaggag aacaagccca agttagtccc ctcacaggca 40
<210> 920
   <211> 40
   <212> DNA
   <213> Homo sapiens
<400> 920
   cagcagcagc tctgcttgag ctactgccaa caccactgct 40
<210> 921
   <211> 40
   <212> DNA
   <213> Homo sapiens
<400> 921
   cagcagcagc tctgcttgag gtgttggatc ctgaacaaaa 40
<210> 922
   <211> 40
   <212> DNA
   <213> Homo sapiens
<400> 922
   gcacttatgg tggtggcgtg agtttccaga ccttcagcat 40
<210> 923
   <211> 40
   <212> DNA
   <213> Homo sapiens
<400> 923
   gcacttatgg tggtggcgtg cacctgtcca gcccactggc 40
<210> 924
   <211> 40
   <212> DNA
   <213> Homo sapiens
<400> 924
   gtggctccag tatcagaaag agaccacaga gctgggcagc 40
<210> 925
   <211> 40
   <212> DNA
   <213> Homo sapiens
<400> 925
   gacctgctca agttcactca agaccacaga gctgggcagc 40
<210> 926
   <211> 40
   <212> DNA
   <213> Homo sapiens
<400> 926
   tgtgggcatg gagcgaaaag tgctgccctg ctttctctgt 40
<210> 927
   <211> 40
   <212> DNA
   <213> Homo sapiens
<400> 927
   tgtgggcatg gagcgaaaag ggtgtgctgt ccgacctcac 40
<210> 928
   <211> 40
   <212> DNA
   <213> Homo sapiens
<400> 928
   ttcctcttcc cctcatcaag tcctctcttt ctcctttgtc 40
<210> 929
   <211> 40
   <212> DNA
   <213> Homo sapiens
<400> 929
   ttcctcttcc cctcatcaag agctatctgt tccagctgct 40
<210> 930
   <211> 40
   <212> DNA
   <213> Homo sapiens
<400> 930
   ggggcactga cacggctact agcctctctg gcctcttcca 40
<210> 931
   <211> 40
   <212> DNA
   <213> Homo sapiens
<400> 931
   ggggcactga cacggctact gtgttggaca tggccacgga 40
<210> 932
   <211> 40
   <212> DNA
   <213> Homo sapiens
<400> 932
   aggctgtagc aggactccag ggttgggaag aacatggaaa 40
<210> 933
   <211> 40
   <212> DNA
   <213> Homo sapiens
<400> 933
   aggctgtagc aggactccag gaagatgtta ccgagtactt 40
<210> 934
   <211> 40
   <212> DNA
   <213> Homo sapiens
<400> 934
   ccgaggatgc taaggggcag tttctgttcc aggtgaaatc 40
<210> 935
   <211> 40
   <212> DNA
   <213> Homo sapiens
<400> 935
   ccgaggatgc taaggggcag gattggatag ctttagtcaa 40
<210> 936
   <211> 40
   <212> DNA
   <213> Homo sapiens
<400> 936
   gcctcccggt ccgcaagcag aatgaagaac tgcatgtggc 40
<210> 937
   <211> 40
   <212> DNA
   <213> Homo sapiens
<400> 937
   gcctcccggt ccgcaagcag ttccagttat actccgtgta 40
<210> 938
   <211> 40
   <212> DNA
   <213> Homo sapiens
<400> 938
   tcgtaacagg ggttgcacag gtgaagatca tgacggagaa 40
<210> 939
   <211> 40
   <212> DNA
   <213> Homo sapiens
<400> 939
   ctgtgacggg tgtcgcccag gtgaagatca tgacggagaa 40
<210> 940
   <211> 40
   <212> DNA
   <213> Homo sapiens
<400> 940
   gtttggggaa gtatggatgg agaaagctga tggtttgtgt 40
<210> 941
   <211> 40
   <212> DNA
   <213> Homo sapiens
<400> 941
   gtttggggaa gtatggatgg gtacctggaa tggaaacaca 40
<210> 942
   <211> 40
   <212> DNA
   <213> Homo sapiens
<400> 942
   taactaatcc ttctcagcag aaagagctgg gctccactga 40
<210> 943
   <211> 40
   <212> DNA
   <213> Homo sapiens
<400> 943
   cagcctacca gaggcaccag aaagagctgg gctccactga 40
<210> 944
   <211> 40
   <212> DNA
   <213> Homo sapiens
<400> 944
   cctgcgcaac tggtaccgag gcgcagccag tgtctttgga 40
<210> 945
   <211> 40
   <212> DNA
   <213> Homo sapiens
<400> 945
   cctgcgcaac tggtaccgag gggacaaccc caacaagccc 40
<210> 946
   <211> 40
   <212> DNA
   <213> Homo sapiens
<400> 946
   ataaaaattg cttagtaaag atttttgcct tctctcaggt 40
<210> 947
   <211> 40
   <212> DNA
   <213> Homo sapiens
<400> 947
   ataaaaattg cttagtaaag gtcaaagatt ctaaactgcc 40
<210> 948
   <211> 40
   <212> DNA
   <213> Homo sapiens
<400> 948
   tgagttcatg gatgatgcca aaattctttt taatctttcg 40
<210> 949
   <211> 40
   <212> DNA
   <213> Homo sapiens
<400> 949
   tgagttcatg gatgatgcca acatgtgcat tgccattgcg 40
<210> 950
   <211> 40
   <212> DNA
   <213> Homo sapiens
<400> 950
   agagttgaaa aacactggcg tctccttttc aggaatcaca 40
<210> 951
   <211> 40
   <212> DNA
   <213> Homo sapiens
<400> 951
   agagttgaaa aacactggcg tttaattggt tggggtcaga 40
<210> 952
   <211> 40
   <212> DNA
   <213> Homo sapiens
<400> 952
   tggggccaca aagacagatg ctggatacac agtatcgtcg 40
<210> 953
   <211> 40
   <212> DNA
   <213> Homo sapiens
<400> 953
   tggggccaca aagacagatg aaaccccatg gcgactctag 40
<210> 954
   <211> 40
   <212> DNA
   <213> Homo sapiens
<400> 954
   aacatggaat catcaggaag ttctccattt ctatttagcc 40
<210> 955
   <211> 40
   <212> DNA
   <213> Homo sapiens
<400> 955
   aacatggaat catcaggaag ccaaggtgga agagcacctt 40
<210> 956
   <211> 40
   <212> DNA
   <213> Homo sapiens
<400> 956
   cgtgcgtgtg tgtgctcttg ctatacacag aatgggattt 40
<210> 957
   <211> 40
   <212> DNA
   <213> Homo sapiens
<400> 957
   cttaggaaag acaaagaact ctatacacag aatgggattt 40
<210> 958
   <211> 40
   <212> DNA
   <213> Homo sapiens
<400> 958
   cccagcctgc tgtccagcag cctcttgcac tgtaccccca 40
<210> 959
   <211> 40
   <212> DNA
   <213> Homo sapiens
<400> 959
   cccagcctgc tgtccagcag gcccccaccc ccgctgcccc 40
<210> 960
   <211> 40
   <212> DNA
   <213> Homo sapiens
<400> 960
   acccaaggct cgtcctgaag tttctctgtt tccttctgca 40
<210> 961
   <211> 40
   <212> DNA
   <213> Homo sapiens
<400> 961
   acccaaggct cgtcctgaag acgtggttaa cttggacctc 40
<210> 962
   <211> 40
   <212> DNA
   <213> Homo sapiens
<400> 962
   gaacccggtg gtacccatag ttgctttgtc ccctcctcag 40
<210> 963
   <211> 40
   <212> DNA
   <213> Homo sapiens
<400> 963
   gaacccggtg gtacccatag gttgcctggc cacggcggcc 40
<210> 964
   <211> 40
   <212> DNA
   <213> Homo sapiens
<400> 964
   aatggaagta ccagcagaag aattttattt ttttcaagat 40
<210> 965
   <211> 40
   <212> DNA
   <213> Homo sapiens
<400> 965
   aatggaagta ccagcagaag attctactca acatgtccct 40
<210> 966
   <211> 40
   <212> DNA
   <213> Homo sapiens
<400> 966
   ggcagctgtt agccgagcaa gagctggacg aggtattgtg 40
<210> 967
   <211> 40
   <212> DNA
   <213> Homo sapiens
<400> 967
   ggcagctgtt agccgagcaa cttgctgatg accgtatggc 40
<210> 968
   <211> 40
   <212> DNA
   <213> Homo sapiens
<400> 968
   gtttagaaat ggaaaaatgt tttttgcttt tacagtaaca 40
<210> 969
   <211> 40
   <212> DNA
   <213> Homo sapiens
<400> 969
   gtttagaaat ggaaaaatgt taacaaatgt ggcaattatt 40
<210> 970
   <211> 40
   <212> DNA
   <213> Homo sapiens
<400> 970
   aaatctcgtg gacttctaag ttttctgttt gcccagaaag 40
<210> 971
   <211> 40
   <212> DNA
   <213> Homo sapiens
<400> 971
   aaatctcgtg gacttctaag aaagcgccat ggcctgtgct 40
<210> 972
   <211> 40
   <212> DNA
   <213> Homo sapiens
<400> 972
   agttccgggg ctacctgatg ccttcctctt tgcagaaatc 40
<210> 973
   <211> 40
   <212> DNA
   <213> Homo sapiens
<400> 973
   agttccgggg ctacctgatg aaatctctcc agacctcgct 40
<210> 974
   <211> 40
   <212> DNA
   <213> Homo sapiens
<400> 974
   aagaaggaat ccacgttcta gtcatttctt ttcaggattg 40
<210> 975
   <211> 40
   <212> DNA
   <213> Homo sapiens
<400> 975
   aagaaggaat ccacgttcta gattggccat ttgatgatgg 40
<210> 976
   <211> 40
   <212> DNA
   <213> Homo sapiens
<400> 976
   cgctggcacc atgaacccag gaccaagtga gcagagagaa 40
<210> 977
   <211> 40
   <212> DNA
   <213> Homo sapiens
<400> 977
   agcccctgct tgacaaccag tttcatgtcc caccaggttg 40
<210> 978
   <211> 40
   <212> DNA
   <213> Homo sapiens
<400> 978
   agcccctgct tgacaaccag gttggtttta agaacatgca 40
<210> 979
   <211> 40
   <212> DNA
   <213> Homo sapiens
<400> 979
   ttggctgtag gaaactcagg gtccagctgt agttcctctg 40
<210> 980
   <211> 40
   <212> DNA
   <213> Homo sapiens
<400> 980
   ttggctgtag gaaactcagg cggcgttgac attccccagg 40
<210> 981
   <211> 40
   <212> DNA
   <213> Homo sapiens
<400> 981
   tgtatctccg acactcagag actgtctctg gaggttatga 40
<210> 982
   <211> 40
   <212> DNA
   <213> Homo sapiens
<400> 982
   tgtatctccg acactcagag gatttcccta gagattatga 40
<210> 983
   <211> 40
   <212> DNA
   <213> Homo sapiens
<400> 983
   aggctgatct actgcaggag ccacgtcatg aatattttaa 40
<210> 984
   <211> 40
   <212> DNA
   <213> Homo sapiens
<400> 984
   aggctgatct actgcaggag gaagctgaaa ccccacgtag 40
<210> 985
   <211> 40
   <212> DNA
   <213> Homo sapiens
<400> 985
   gctcagcccc ctccccacag ggcccctaga agcctgtttc 40
<210> 986
   <211> 40
   <212> DNA
   <213> Homo sapiens
<400> 986
   tgaccctgca gctcctcaaa ggcccctaga agcctgtttc 40
<210> 987
   <211> 40
   <212> DNA
   <213> Homo sapiens
<400> 987
   gccgacccgc ctgcgacgct cttttcttgc ctggagaaga 40
<210> 988
   <211> 40
   <212> DNA
   <213> Homo sapiens
<400> 988
   gccgacccgc ctgcgacgct gggaccgtga tgcccggccc 40
<210> 989
   <211> 40
   <212> DNA
   <213> Homo sapiens
<400> 989
   tgcggagacc ccttcgggag gtgacagttc gtgatgctat 40
<210> 990
   <211> 40
   <212> DNA
   <213> Homo sapiens
<400> 990
   tgcggagacc ccttcgggag gtctccgggc tgctgaagag 40
<210> 991
   <211> 40
   <212> DNA
   <213> Homo sapiens
<400> 991
   ctatcagtag gtttttagag atgaacatca ctcgaaaact 40
<210> 992
   <211> 40
   <212> DNA
   <213> Homo sapiens
<400> 992
   gcattgatgt ggaagatgca atgaacatca ctcgaaaact 40
<210> 993
   <211> 40
   <212> DNA
   <213> Homo sapiens
<400> 993
   gcattgatgt ggaagatgca gtttttttcc tggcagaaga 40
<210> 994
   <211> 40
   <212> DNA
   <213> Homo sapiens
<400> 994
   cagtgggcgg atgacatttg gtacagcctc ggaactggct 40
<210> 995
   <211> 40
   <212> DNA
   <213> Homo sapiens
<400> 995
   cagtgggcgg atgacatttg ccctctgttg ctattctttg 40
<210> 996
   <211> 40
   <212> DNA
   <213> Homo sapiens
<400> 996
   ggtgtccatg gcctgcactc ctataccttt ctgccgtgta 40
<210> 997
   <211> 40
   <212> DNA
   <213> Homo sapiens
<400> 997
   ggtgtccatg gcctgcactc ttacgaaaag cggctgtact 40
<210> 998
   <211> 40
   <212> DNA
   <213> Homo sapiens
<400> 998
   actgggaagt tcttaaaaag tccccctcta cacaagaatc 40
<210> 999
   <211> 40
   <212> DNA
   <213> Homo sapiens
<400> 999
   actgggaagt tcttaaaaag gttcacagat gaagagtcta 40
<210> 1000
   <211> 40
   <212> DNA
   <213> Homo sapiens
<400> 1000
   gctgagcggg gcgacccgag tcttctcatt cacaggttaa 40
<210> 1001
   <211> 40
   <212> DNA
   <213> Homo sapiens
<400> 1001
   tgttccacct cctcctgcag ctcccccttt tcttccagtg 40
<210> 1002
   <211> 40
   <212> DNA
   <213> Homo sapiens
<400> 1002
   tgttccacct cctcctgcag tgggccggat gtatcccccg 40
<210> 1003
   <211> 40
   <212> DNA
   <213> Homo sapiens
<400> 1003
   acccatgaga atgctcagag ctatgaagac cccgcggccc 40
<210> 1004
   <211> 40
   <212> DNA
   <213> Homo sapiens
<400> 1004
   ctggcccctg agatccgcag ctatgaagac cccgcggccc 40
<210> 1005
   <211> 40
   <212> DNA
   <213> Homo sapiens
<400> 1005
   caagctcgag tccatcgatg aacccatctg cgccgtcggc 40
<210> 1006
   <211> 40
   <212> DNA
   <213> Homo sapiens
<400> 1006
   caagctcgag tccatcgatg gtgcccggta ccatgccctc 40
<210> 1007
   <211> 40
   <212> DNA
   <213> Homo sapiens
<400> 1007
   cttcactgtc accgtcacag aacccccagt gcggatcata 40
<210> 1008
   <211> 40
   <212> DNA
   <213> Homo sapiens
<400> 1008
   cttcactgtc accgtcacag agtcttacca aagtcaggac 40
<210> 1009
   <211> 40
   <212> DNA
   <213> Homo sapiens
<400> 1009
   agaaacagaa accagcacag gatgtacctg gcaaagattc 40
<210> 1010
   <211> 40
   <212> DNA
   <213> Homo sapiens
<400> 1010
   agaaacagaa accagcacag aattatgatg acaatttcaa 40
<210> 1011
   <211> 40
   <212> DNA
   <213> Homo sapiens
<400> 1011
   ttctgcatct gtgggccgag tgatcctgcc atgaagcagt 40
<210> 1012
   <211> 40
   <212> DNA
   <213> Homo sapiens
<400> 1012
   aaaggagtgc ttatagaatg tgatcctgcc atgaagcagt 40
<210> 1013
   <211> 40
   <212> DNA
   <213> Homo sapiens
<400> 1013
   aggatcggca acatggcaag gcctctacta cgtggacagt 40
<210> 1014
   <211> 40
   <212> DNA
   <213> Homo sapiens
<400> 1014
   ctgggataag agaggccctg gcctctacta cgtggacagt 40
<210> 1015
   <211> 40
   <212> DNA
   <213> Homo sapiens
<400> 1015
   gttcaggaca caataagcag gttgcagagc ctgaggcctg 40
<210> 1016
   <211> 40
   <212> DNA
   <213> Homo sapiens
<400> 1016
   gggagggaga gaatacccag gttgcagagc ctgaggcctg 40
<210> 1017
   <211> 40
   <212> DNA
   <213> Homo sapiens
<400> 1017
   tacccggatg atggcatggg aagttcttgc tgtctttcag 40
<210> 1018
   <211> 40
   <212> DNA
   <213> Homo sapiens
<400> 1018
   tacccggatg atggcatggg gtatggcgac tacccgaagc 40
<210> 1019
   <211> 40
   <212> DNA
   <213> Homo sapiens
<400> 1019
   gacatatgag tcaaaggaag cccggtggcg cctgtccgtc 40
<210> 1020
   <211> 40
   <212> DNA
   <213> Homo sapiens
<400> 1020
   gacatatgag tcaaaggaag aagcccggtg gcgcctgtcc 40
<210> 1021
   <211> 40
   <212> DNA
   <213> Homo sapiens
<400> 1021
   cggatcaact tcgacaaata gtggttgtta cctcttccta 40
<210> 1022
   <211> 40
   <212> DNA
   <213> Homo sapiens
<400> 1022
   cggatcaact tcgacaaata ccacccaggc tactttggga 40
<210> 1023
   <211> 40
   <212> DNA
   <213> Homo sapiens
<400> 1023
   ttataggcgt gatgatagag tttcatttaa cttaggtccc 40
<210> 1024
   <211> 40
   <212> DNA
   <213> Homo sapiens
<400> 1024
   ttataggcgt gatgatagag gtccccccca aagacccaaa 40
<210> 1025
   <211> 40
   <212> DNA
   <213> Homo sapiens
<400> 1025
   tggaaatatt tctagacttg gtgtcagttg tacccgaggc 40
<210> 1026
   <211> 40
   <212> DNA
   <213> Homo sapiens
<400> 1026
   tcggcccaga agaaccccgc ctatacacag aatgggattt 40
<210> 1027
   <211> 40
   <212> DNA
   <213> Homo sapiens
<400> 1027
   gctgaaggga aaagacacca aaacacaaac agcagaatgg 40
<210> 1028
   <211> 40
   <212> DNA
   <213> Homo sapiens
<400> 1028
   gctgaaggga aaagacacca gttgcctggc agagcagtgg 40
<210> 1029
   <211> 40
   <212> DNA
   <213> Homo sapiens
<400> 1029
   catcatcaag tttttcaatg acgagctggt ccagccatcc 40
<210> 1030
   <211> 40
   <212> DNA
   <213> Homo sapiens
<400> 1030
   catcatcaag tttttcaatg aacgtgctga gcatcacgat 40
<210> 1031
   <211> 40
   <212> DNA
   <213> Homo sapiens
<400> 1031
   gagggcctgc tcattcaaag atgttctcag tgcagctgag 40
<210> 1032
   <211> 40
   <212> DNA
   <213> Homo sapiens
<400> 1032
   acatgcttca aataaatcag atgttctcag tgcagctgag 40
<210> 1033
   <211> 40
   <212> DNA
   <213> Homo sapiens
<400> 1033
   ctgaggctaa tgaaaaacag ggaagctgcc aaagaatgtc 40
<210> 1034
   <211> 40
   <212> DNA
   <213> Homo sapiens
<400> 1034
   ctgaggctaa tgaaaaacag ggaagctgcc cgggagtgtc 40
<210> 1035
   <211> 40
   <212> DNA
   <213> Homo sapiens
<400> 1035
   cggctgggac tcttccatgc gtggcactgg aagcagactg 40
<210> 1036
   <211> 40
   <212> DNA
   <213> Homo sapiens
<400> 1036
   cggctgggac tcttccatgc agttgaaact ggttgacaac 40
<210> 1037
   <211> 40
   <212> DNA
   <213> Homo sapiens
<400> 1037
   agtgaatgta gttgcaccag tgacaatact tgtatggagt 40
<210> 1038
   <211> 40
   <212> DNA
   <213> Homo sapiens
<400> 1038
   agtgaatgta gttgcaccag gatttgtaca cacagatatg 40
<210> 1039
   <211> 40
   <212> DNA
   <213> Homo sapiens
<400> 1039
   attcacacag agccacctag gccaggctac caacgtcttt 40
<210> 1040
   <211> 40
   <212> DNA
   <213> Homo sapiens
<400> 1040
   tgaggatcaa tcctggggag gccaggctac caacgtcttt 40
<210> 1041
   <211> 40
   <212> DNA
   <213> Homo sapiens
<400> 1041
   tggaaaagta taaaggcaaa attcttcaaa gaaggaacca 40
<210> 1042
   <211> 40
   <212> DNA
   <213> Homo sapiens
<400> 1042
   tggaaaagta taaaggcaaa gtttcactag ttgtaaacgt 40
<210> 1043
   <211> 40
   <212> DNA
   <213> Homo sapiens
<400> 1043
   atactaagaa caacaatttg aatgggacaa cagaagaagt 40
<210> 1044
   <211> 40
   <212> DNA
   <213> Homo sapiens
<400> 1044
   atactaagaa caacaatttg cttcgtcagc aattgaagtg 40
<210> 1045
   <211> 40
   <212> DNA
   <213> Homo sapiens
<400> 1045
   tggccttgac ctccaaccag gtcctgcacc cagacctcac 40
<210> 1046
   <211> 40
   <212> DNA
   <213> Homo sapiens
<400> 1046
   tggccttgac ctccaaccag gagtacctgg acctgtccat 40
<210> 1047
   <211> 40
   <212> DNA
   <213> Homo sapiens
<400> 1047
   tccttgaaca ctacaattag acctcttctt gggtgaattt 40
<210> 1048
   <211> 40
   <212> DNA
   <213> Homo sapiens
<400> 1048
   tccttgaaca ctacaattag ctgttctgaa gcccagaaaa 40
<210> 1049
   <211> 40
   <212> DNA
   <213> Homo sapiens
<400> 1049
   acaccattga ggagatccag gtgcggcagc tggtgcctcg 40
<210> 1050
   <211> 40
   <212> DNA
   <213> Homo sapiens
<400> 1050
   acaccattga ggagatccag ggactgacca cagcccatga 40
<210> 1051
   <211> 40
   <212> DNA
   <213> Homo sapiens
<400> 1051
   cccatgtata aggctttccg gatgtgctct ttgtcctcca 40
<210> 1052
   <211> 40
   <212> DNA
   <213> Homo sapiens
<400> 1052
   cccatgtata aggctttccg gagtgacagt tcattcaatt 40
<210> 1053
   <211> 40
   <212> DNA
   <213> Homo sapiens
<400> 1053
   ctcccagtgc tgtatatccc ggaattcctg gggaagtcgg 40
<210> 1054
   <211> 40
   <212> DNA
   <213> Homo sapiens
<400> 1054
   gagctgccac ggatactgag ggaattcctg gggaagtcgg 40
<210> 1055
   <211> 40
   <212> DNA
   <213> Homo sapiens
<400> 1055
   ggatgcgcgt ctggtcaagg gctgcagaga aggctggtat 40
<210> 1056
   <211> 40
   <212> DNA
   <213> Homo sapiens
<400> 1056
   agccgcagag catcctggcg gctgcagaga aggctggtat 40
<210> 1057
   <211> 40
   <212> DNA
   <213> Homo sapiens
<400> 1057
   acatgcttca aataaatcag cttctctcca agataaaatg 40
<210> 1058
   <211> 40
   <212> DNA
   <213> Homo sapiens
<400> 1058
   aacaaagaaa taattcacag gatgaagatg ggtttcaaga 40
<210> 1059
   <211> 40
   <212> DNA
   <213> Homo sapiens
<400> 1059
   ctgagtcttt atattttgag gatgaagatg ggtttcaaga 40
<210> 1060
   <211> 40
   <212> DNA
   <213> Homo sapiens
<400> 1060
   tagccaccac tgtgtgccag ggatatcttc taaccatacc 40
<210> 1061
   <211> 40
   <212> DNA
   <213> Homo sapiens
<400> 1061
   gggaaaagtc tttcaccctg ggatatcttc taaccatacc 40
<210> 1062
   <211> 40
   <212> DNA
   <213> Homo sapiens
<400> 1062
   cctaccagcc acttcgggag gtatcagagt gctccatctc 40
<210> 1063
   <211> 40
   <212> DNA
   <213> Homo sapiens
<400> 1063
   cctaccagcc acttcgggag gtattgccag ggaacagacg 40
<210> 1064
   <211> 40
   <212> DNA
   <213> Homo sapiens
<400> 1064
   gtcccggctt ccccctactc gcctggctca gaatctaacc 40
<210> 1065
   <211> 40
   <212> DNA
   <213> Homo sapiens
<400> 1065
   gtcccggctt ccccctactc agtgaagaag ccaccctcag 40
<210> 1066
   <211> 40
   <212> DNA
   <213> Homo sapiens
<400> 1066
   cttaagcata tatttaaagg gtgaagatgc ttttgatgcc 40
<210> 1067
   <211> 40
   <212> DNA
   <213> Homo sapiens
<400> 1067
   cttaagcata tatttaaagg gagatgtttt tgattcagcc 40
<210> 1068
   <211> 40
   <212> DNA
   <213> Homo sapiens
<400> 1068
   caggaacaag tatctgacag aaaatatctt tcaggcctgg 40
<210> 1069
   <211> 40
   <212> DNA
   <213> Homo sapiens
<400> 1069
   caggaacaag tatctgacag tcaagtccta attcgaagca 40
<210> 1070
   <211> 40
   <212> DNA
   <213> Homo sapiens
<400> 1070
   gaagttctga ggaaaagcag aatgctgtgt cctctgaaga 40
<210> 1071
   <211> 40
   <212> DNA
   <213> Homo sapiens
<400> 1071
   gaagttctga ggaaaagcag ctttacaaca aatacccaga 40
<210> 1072
   <211> 40
   <212> DNA
   <213> Homo sapiens
<400> 1072
   atctccctct tggtgtacaa attgttttca gaaaacacaa 40
<210> 1073
   <211> 40
   <212> DNA
   <213> Homo sapiens
<400> 1073
   atctccctct tggtgtacaa aaaacacaag gaatacaacc 40
<210> 1074
   <211> 40
   <212> DNA
   <213> Homo sapiens
<400> 1074
   tgcgagtact gcttcaccag aaagaagatt ggcccatgca 40
<210> 1075
   <211> 40
   <212> DNA
   <213> Homo sapiens
<400> 1075
   tgcgagtact gcttcaccag gaaagaagga ttgtccaaat 40
<210> 1076
   <211> 40
   <212> DNA
   <213> Homo sapiens
<400> 1076
   tccagaaagt gaaactaaaa ttttaatcca ggtgctggtt 40
<210> 1077
   <211> 40
   <212> DNA
   <213> Homo sapiens
<400> 1077
   tccagaaagt gaaactaaaa gagcgtcagg aagcagagaa 40
<210> 1078
   <211> 40
   <212> DNA
   <213> Homo sapiens
<400> 1078
   agattctaca gataaatcag atttcggaaa cttctggcag 40
<210> 1079
   <211> 40
   <212> DNA
   <213> Homo sapiens
<400> 1079
   agattctaca gataaatcag ctgcacttag tgcattggaa 40
<210> 1080
   <211> 40
   <212> DNA
   <213> Homo sapiens
<400> 1080
   tggctggctt cagtggacca aattttcagg atggctgtat 40
<210> 1081
   <211> 40
   <212> DNA
   <213> Homo sapiens
<400> 1081
   tggctggctt cagtggacca gccttcatgg tgaaacacct 40
<210> 1082
   <211> 40
   <212> DNA
   <213> Homo sapiens
<400> 1082
   ccctgctcat cacctacggg gaacccagaa tgggggcttc 40
<210> 1083
   <211> 40
   <212> DNA
   <213> Homo sapiens
<400> 1083
   ggcagccacc acgggctcgg acaatttatg aaaaccgaat 40
<210> 1084
   <211> 40
   <212> DNA
   <213> Homo sapiens
<400> 1084
   tgataattgg gcctccaaga acaatttatg aaaaccgaat 40
<210> 1085
   <211> 40
   <212> DNA
   <213> Homo sapiens
<400> 1085
   accgccctgc actgctacag gagtcctccg ctctgccaca 40
<210> 1086
   <211> 40
   <212> DNA
   <213> Homo sapiens
<400> 1086
   accgccctgc actgctacag gaagggcctg accttcgtct 40
<210> 1087
   <211> 40
   <212> DNA
   <213> Homo sapiens
<400> 1087
   tcacaattat aggggaagag ctcgtggtct gggttgatcc 40
<210> 1088
   <211> 40
   <212> DNA
   <213> Homo sapiens
<400> 1088
   gtgctattaa agaagaagat ctcgtggtct gggttgatcc 40
<210> 1089
   <211> 40
   <212> DNA
   <213> Homo sapiens
<400> 1089
   ctcgtctatg atatcaccag atgcccgaat gctagcgagc 40
<210> 1090
   <211> 40
   <212> DNA
   <213> Homo sapiens
<400> 1090
   ctcgtctatg atatcaccag ccgagaaacc tacaatgcgc 40
<210> 1091
   <211> 40
   <212> DNA
   <213> Homo sapiens
<400> 1091
   caatgccaca gggcaggctg gaaggctggg atgcatggga 40
<210> 1092
   <211> 40
   <212> DNA
   <213> Homo sapiens
<400> 1092
   caatgccaca gggcaggctg actgcaaagc ccaggatgag 40
<210> 1093
   <211> 40
   <212> DNA
   <213> Homo sapiens
<400> 1093
   tcagaagaga aaatcggatg acaggcggac ccacaggccc 40
<210> 1094
   <211> 40
   <212> DNA
   <213> Homo sapiens
<400> 1094
   tcagaagaga aaatcggatg gaccttgacc ctgctgttca 40
<210> 1095
   <211> 40
   <212> DNA
   <213> Homo sapiens
<400> 1095
   tgactgccgc tttctctcag gcccggaaac aaaactcatg 40
<210> 1096
   <211> 40
   <212> DNA
   <213> Homo sapiens
<400> 1096
   ctaaagcctt ctataaaact gcccggaaac aaaactcatg 40
<210> 1097
   <211> 40
   <212> DNA
   <213> Homo sapiens
<400> 1097
   atgcagatac acaaagcaag ccatgcagtt tggtcagctc 40
<210> 1098
   <211> 40
   <212> DNA
   <213> Homo sapiens
<400> 1098
   atgcagatac acaaagcaag gtgcaccagc tatatgaaac 40
<210> 1099
   <211> 40
   <212> DNA
   <213> Homo sapiens
<400> 1099
   tactgatcat attgtccaag tcaaagtaaa caagtatgga 40
<210> 1100
   <211> 40
   <212> DNA
   <213> Homo sapiens
<400> 1100
   aagagtgcca aaaaaagaag tcaaagtaaa caagtatgga 40
<210> 1101
   <211> 40
   <212> DNA
   <213> Homo sapiens
<400> 1101
   tgtcatccat tgtggaagag ccccgaaaca cagcagagct 40
<210> 1102
   <211> 40
   <212> DNA
   <213> Homo sapiens
<400> 1102
   tgtcatccat tgtggaagag ctgctggatc agtgcctggc 40
<210> 1103
   <211> 40
   <212> DNA
   <213> Homo sapiens
<400> 1103
   taccggaaac ctaggaaaag gcgccaagcc catctttgtg 40
<210> 1104
   <211> 40
   <212> DNA
   <213> Homo sapiens
<400> 1104
   gctgccaaag ccttagacaa gcgccaagcc catctttgtg 40
<210> 1105
   <211> 40
   <212> DNA
   <213> Homo sapiens
<400> 1105
   aggatatcgg tttcattaag aaagacctga gctgtcttcc 40
<210> 1106
   <211> 40
   <212> DNA
   <213> Homo sapiens
<400> 1106
   aggatatcgg tttcattaag ttggactaaa tgctcttcct 40
<210> 1107
   <211> 40
   <212> DNA
   <213> Homo sapiens
<400> 1107
   gcggcgggca gtggcggcag gtgtacattt ttatctttca 40
<210> 1108
   <211> 40
   <212> DNA
   <213> Homo sapiens
<400> 1108
   gcggcgggca gtggcggcag aatgttggct accagggtat 40
<210> 1109
   <211> 40
   <212> DNA
   <213> Homo sapiens
<400> 1109
   tatccagcac tgaccacatg gacagacgtt gaaagatacc 40
<210> 1110
   <211> 40
   <212> DNA
   <213> Homo sapiens
<400> 1110
   cctgattctc cccaccagag gacagacgtt gaaagatacc 40
<210> 1111
   <211> 40
   <212> DNA
   <213> Homo sapiens
<400> 1111
   ttcatcatgg tgtggtggag ctctcctctt gtttttcagg 40
<210> 1112
   <211> 40
   <212> DNA
   <213> Homo sapiens
<400> 1112
   ttcatcatgg tgtggtggag gttgacgccg ctgtcacccc 40
<210> 1113
   <211> 40
   <212> DNA
   <213> Homo sapiens
<400> 1113
   tgaaatcaga aaaaaatatg tttattttgt ttcaggcctg 40
<210> 1114
   <211> 40
   <212> DNA
   <213> Homo sapiens
<400> 1114
   tgaaatcaga aaaaaatatg gcctgtttaa agaagaaaac 40
<210> 1115
   <211> 40
   <212> DNA
   <213> Homo sapiens
<400> 1115
   gcaaggatat ataataactg ctgctttatt tttccacaga 40
<210> 1116
   <211> 40
   <212> DNA
   <213> Homo sapiens
<400> 1116
   gcaaggatat ataataactg attggtgtgc ccgtttaata 40
<210> 1117
   <211> 40
   <212> DNA
   <213> Homo sapiens
<400> 1117
   gaactgcaaa ggcttcagag gatttgtaca cacagatatg 40
<210> 1118
   <211> 40
   <212> DNA
   <213> Homo sapiens
<400> 1118
   ttggagatca ggacgcaaag gtcaccatca gaaaagctaa 40
<210> 1119
   <211> 40
   <212> DNA
   <213> Homo sapiens
<400> 1119
   gatctggatt ctcgtttcag gtcaccatca gaaaagctaa 40
<210> 1120
   <211> 40
   <212> DNA
   <213> Homo sapiens
<400> 1120
   tggaagaggc tacctctggg gtcaatgaga gtgaaatggc 40
<210> 1121
   <211> 40
   <212> DNA
   <213> Homo sapiens
<400> 1121
   tggaagaggc tacctctggg gtaacccccg ggactttgcc 40
<210> 1122
   <211> 40
   <212> DNA
   <213> Homo sapiens
<400> 1122
   tctggagcca tacgtgacag tgacctgacc aacggtgcag 40
<210> 1123
   <211> 40
   <212> DNA
   <213> Homo sapiens
<400> 1123
   tctggagcca tacgtgacag aaatggctca gggaactgtt 40
<210> 1124
   <211> 40
   <212> DNA
   <213> Homo sapiens
<400> 1124
   ctgcagtatc tgtaaccgag gtctccaggc accaggagcc 40
<210> 1125
   <211> 40
   <212> DNA
   <213> Homo sapiens
<400> 1125
   ctgcagtatc tgtaaccgag gtttctcctc tgcctcctac 40
<210> 1126
   <211> 40
   <212> DNA
   <213> Homo sapiens
<400> 1126
   tgatttcaag tttgaacaag gggttggcat ctgcacatcc 40
<210> 1127
   <211> 40
   <212> DNA
   <213> Homo sapiens
<400> 1127
   tgatgagact ccagacagag gggttggcat ctgcacatcc 40
<210> 1128
   <211> 40
   <212> DNA
   <213> Homo sapiens
<400> 1128
   agcgagctcc tcagcctcag gcatctgcat ctgggaccga 40
<210> 1129
   <211> 40
   <212> DNA
   <213> Homo sapiens
<400> 1129
   ccggggattg ccggcgccag gcatctgcat ctgggaccga 40
<210> 1130
   <211> 40
   <212> DNA
   <213> Homo sapiens
<400> 1130
   agtttctact agtccagttg gtgactctcc tattccatct 40
<210> 1131
   <211> 40
   <212> DNA
   <213> Homo sapiens
<400> 1131
   agtttctact agtccagttg ggttaccatc cattgaccca 40
<210> 1132
   <211> 40
   <212> DNA
   <213> Homo sapiens
<400> 1132
   ggaaaggaca gcaagcacag gtgagactgt ggagatgaga 40
<210> 1133
   <211> 40
   <212> DNA
   <213> Homo sapiens
<400> 1133
   tgaggtgccc taagcacaag gtgagactgt ggagatgaga 40
<210> 1134
   <211> 40
   <212> DNA
   <213> Homo sapiens
<400> 1134
   agttgcatgt tgactttagg gagtctgtgt gaagcagcac 40
<210> 1135
   <211> 40
   <212> DNA
   <213> Homo sapiens
<400> 1135
   agttgcatgt tgactttagg aacgtgaagc tcttggagca 40
<210> 1136
   <211> 40
   <212> DNA
   <213> Homo sapiens
<400> 1136
   ccgcccccgt tccatccacg ggggagctca gtgtgaacac 40
<210> 1137
   <211> 40
   <212> DNA
   <213> Homo sapiens
<400> 1137
   ccgcccccgt tccatccacg gacgagtgtg aggacgccaa 40
<210> 1138
   <211> 40
   <212> DNA
   <213> Homo sapiens
<400> 1138
   tggagccgaa caacatcgtg ctcagcgatg cctgccgctt 40
<210> 1139
   <211> 40
   <212> DNA
   <213> Homo sapiens
<400> 1139
   tggagccgaa caacatcgtg gttctgctcc agacgagccc 40
<210> 1140
   <211> 40
   <212> DNA
   <213> Homo sapiens
<400> 1140
   gcctggaaag ctaccaaaag gagctgtcca gacagctggt 40
<210> 1141
   <211> 40
   <212> DNA
   <213> Homo sapiens
<400> 1141
   gcctggaaag ctaccaaaag ggatctctgc aggagctgtc 40
<210> 1142
   <211> 40
   <212> DNA
   <213> Homo sapiens
<400> 1142
   tgttattgta gattctgggg gtggacttct caaaccaaca 40
<210> 1143
   <211> 40
   <212> DNA
   <213> Homo sapiens
<400> 1143
   tgttattgta gattctgggg gctttgatga actaggtgga 40
<210> 1144
   <211> 40
   <212> DNA
   <213> Homo sapiens
<400> 1144
   ggggaccaag aaaagcagca tggttgcact gaaaagactg 40
<210> 1145
   <211> 40
   <212> DNA
   <213> Homo sapiens
<400> 1145
   ggggaccaag aaaagcagca ccatgaatga cctggtgcag 40
<210> 1146
   <211> 40
   <212> DNA
   <213> Homo sapiens
<400> 1146
   caaaaaagac caaaactgag gaactccctc ggccacagtc 40
<210> 1147
   <211> 40
   <212> DNA
   <213> Homo sapiens
<400> 1147
   caaaaaagac caaaactgag caggaactcc ctcggccaca 40
<210> 1148
   <211> 40
   <212> DNA
   <213> Homo sapiens
<400> 1148
   ccaaagcaga gacccaggag gtgtacatgg acatcaagat 40
<210> 1149
   <211> 40
   <212> DNA
   <213> Homo sapiens
<400> 1149
   ccaaagcaga gacccaggag ggagagccca ttgctaaaaa 40
<210> 1150
   <211> 40
   <212> DNA
   <213> Homo sapiens
<400> 1150
   gccgaatcac ctgatctaag gagattacct ggggcaattg 40
<210> 1151
   <211> 40
   <212> DNA
   <213> Homo sapiens
<400> 1151
   acgccgcaag tcctccagag gaacagcagc acaatggacc 40
<210> 1152
   <211> 40
   <212> DNA
   <213> Homo sapiens
<400> 1152
   agcacccatg ggtgcagggg gaacagcagc acaatggacc 40
<210> 1153
   <211> 40
   <212> DNA
   <213> Homo sapiens
<400> 1153
   aaccagtaac aacggaacct cagagtccag atctgaacga 40
<210> 1154
   <211> 40
   <212> DNA
   <213> Homo sapiens
<400> 1154
   aaccagtaac aacggaacct agtccagatc tgaacgatgc 40
<210> 1155
   <211> 40
   <212> DNA
   <213> Homo sapiens
<400> 1155
   gagaccgcgt gcgaggaccg cagcaatgca gagtccctgg 40
<210> 1156
   <211> 40
   <212> DNA
   <213> Homo sapiens
<400> 1156
   gagaccgcgt gcgaggaccg caatgcagag tccctggaca 40
<210> 1157
   <211> 40
   <212> DNA
   <213> Homo sapiens
<400> 1157
   gtctctggca agtaatccag aacttcttaa tcttccatcc 40
<210> 1158
   <211> 40
   <212> DNA
   <213> Homo sapiens
<400> 1158
   gtctctggca agtaatccag taattaagaa gaaagttcat 40
<210> 1159
   <211> 40
   <212> DNA
   <213> Homo sapiens
<400> 1159
   aagcatgtag aaagccggaa caggtactta aaatgaatgc 40
<210> 1160
   <211> 40
   <212> DNA
   <213> Homo sapiens
<400> 1160
   aagcatgtag aaagccggaa ggataaagaa atggagaaga 40
<210> 1161
   <211> 40
   <212> DNA
   <213> Homo sapiens
<400> 1161
   agcacccatg ggtgcagggg caagctccag aaaagggact 40
<210> 1162
   <211> 40
   <212> DNA
   <213> Homo sapiens
<400> 1162
   tccacaagag cgaggaggcg aagcgggtgc tgcggtatta 40
<210> 1163
   <211> 40
   <212> DNA
   <213> Homo sapiens
<400> 1163
   aggcggtgag tgtcggacag aagcgggtgc tgcggtatta 40
<210> 1164
   <211> 40
   <212> DNA
   <213> Homo sapiens
<400> 1164
   tccgccccac agtccacgag actttaccag aatgcaggac 40
<210> 1165
   <211> 40
   <212> DNA
   <213> Homo sapiens
<400> 1165
   ggcggagaca tggaccagag actttaccag aatgcaggac 40
<210> 1166
   <211> 40
   <212> DNA
   <213> Homo sapiens
<400> 1166
   ttgatcttcg gccccacacg aacagcagag aggggcagca 40
<210> 1167
   <211> 40
   <212> DNA
   <213> Homo sapiens
<400> 1167
   ttgatcttcg gccccacacg cagagagggg cagcaggatg 40
<210> 1168
   <211> 40
   <212> DNA
   <213> Homo sapiens
<400> 1168
   gaaaaacttt ccagccattg gggggacagg ccccacctcg 40
<210> 1169
   <211> 40
   <212> DNA
   <213> Homo sapiens
<400> 1169
   gaaaaacttt ccagccattg gaggttgtcg ggacatttca 40
<210> 1170
   <211> 40
   <212> DNA
   <213> Homo sapiens
<400> 1170
   gcgctcgccc gggcggcaga ctgtgaggtg gagcagtggg 40
<210> 1171
   <211> 40
   <212> DNA
   <213> Homo sapiens
<400> 1171
   ccgcaggata cccgccgagg ctgtgaggtg gagcagtggg 40
<210> 1172
   <211> 40
   <212> DNA
   <213> Homo sapiens
<400> 1172
   cgggacgact tctacgacag gctcttcgac taccggggcc 40
<210> 1173
   <211> 40
   <212> DNA
   <213> Homo sapiens
<400> 1173
   gcagcatctg ccatatacag gctcttcgac taccggggcc 40
<210> 1174
   <211> 40
   <212> DNA
   <213> Homo sapiens
<400> 1174
   actgaagcag caacacgcct ctctgcgtac gtgtcctatg 40
<210> 1175
   <211> 40
   <212> DNA
   <213> Homo sapiens
<400> 1175
   actgaagcag caacacgcct gctgagattg agagctgctg 40
<210> 1176
   <211> 40
   <212> DNA
   <213> Homo sapiens
<400> 1176
   ctgccggcgg agaatataag gagatggaca aaccgtgtgg 40
<210> 1177
   <211> 40
   <212> DNA
   <213> Homo sapiens
<400> 1177
   ctgccggcgg agaatataag gtgtgtgtga ccatggaacg 40
<210> 1178
   <211> 40
   <212> DNA
   <213> Homo sapiens
<400> 1178
   caacctctaa gactggagcg gttcttcttc cgcagtggga 40
<210> 1179
   <211> 40
   <212> DNA
   <213> Homo sapiens
<400> 1179
   caacctctaa gactggagcg tgggaacatc gagcacccgg 40
<210> 1180
   <211> 21
   <212> DNA
   <213> Artificial Sequence
<220>
   <223> Description of Artificial Sequence: Synthetic oligonucleotide
<400> 1180
   gcgagacaca ctggtattaa g 21
<210> 1181
   <211> 21
   <212> DNA
   <213> Artificial Sequence
<220>
   <223> Description of Artificial Sequence: Synthetic oligonucleotide
<400> 1181
   tgtggatgag cagcagaaag t 21
<210> 1182
   <211> 21
   <212> DNA
   <213> Artificial Sequence
<220>
   <223> Description of Artificial Sequence: Synthetic oligonucleotide
<400> 1182
   gatgagcagc atgaagttcg g 21
<210> 1183
   <211> 23
   <212> DNA
   <213> Artificial Sequence
<220>
   <223> Description of Artificial Sequence: Synthetic primer
<400> 1183
   gacttccttc tttattgccc ttc 23
<210> 1184
   <211> 22
   <212> DNA
   <213> Artificial Sequence
<220>
   <223> Description of Artificial Sequence: Synthetic primer
<400> 1184
   agcactgatg gtccgaactt tc 22
<210> 1185
   <211> 21
   <212> DNA
   <213> Artificial Sequence
<220>
   <223> Description of Artificial Sequence: Synthetic primer
<400> 1185
   gtgtgcaaaa gcaagaagtc c 21
<210> 1186
   <211> 21
   <212> DNA
   <213> Artificial Sequence
<220>
   <223> Description of Artificial Sequence: Synthetic primer
<400> 1186
   gcactgatgg tccgaacttc a 21
<210> 1187
   <211> 25
   <212> DNA
   <213> Artificial Sequence
<220>
   <223> Description of Artificial Sequence: Synthetic primer
<400> 1187
   gcttggcggt gggaaagaga aattg 25
<210> 1188
   <211> 27
   <212> DNA
   <213> Artificial Sequence
<220>
   <223> Description of Artificial Sequence: Synthetic primer
<400> 1188
   aaccagtcat accacccaaa ggtgttg 27
<210> 1189
   <211> 25
   <212> DNA
   <213> Artificial Sequence
<220>
   <223> Description of Artificial Sequence: Synthetic primer
<400> 1189
   ggcacccagc acaatgaaga tcaag 25
<210> 1190
   <211> 26
   <212> DNA
   <213> Artificial Sequence
<220>
   <223> Description of Artificial Sequence: Synthetic primer
<400> 1190
   actcgtcata ctcctgcttg ctgatc 26
<210> 1191
   <211> 20
   <212> DNA
   <213> Artificial Sequence
<220>
   <223> Description of Artificial Sequence: Synthetic primer
<400> 1191
   actctcttcc gcatcgctgt 20
<210> 1192
   <211> 20
   <212> DNA
   <213> Artificial Sequence
<220>
   <223> Description of Artificial Sequence: Synthetic primer
<400> 1192
   ccgacgggtt tccgatccaa 20
<210> 1193
   <211> 18
   <212> DNA
   <213> Artificial Sequence
<220>
   <223> Description of Artificial Sequence: Synthetic probe
<400> 1193
   ctgttgggct cgcggttg 18
<210> 1194
   <211> 21
   <212> DNA
   <213> Artificial Sequence
<220>
   <223> Description of Artificial Sequence: Synthetic primer
<400> 1194
   tcttgtctac ctctggttcc t 21
<210> 1195
   <211> 22
   <212> DNA
   <213> Artificial Sequence
<220>
   <223> Description of Artificial Sequence: Synthetic primer
<400> 1195
   ccttcttgtt gatgtgcctt tc 22
<210> 1196
   <211> 24
   <212> DNA
   <213> Artificial Sequence
<220>
   <223> Description of Artificial Sequence: Synthetic probe
<400> 1196
   cagtcttcgc ccctcttttc ttag 24
<210> 1197
   <211> 17
   <212> DNA
   <213> Artificial Sequence
<220>
   <223> Description of Artificial Sequence: Synthetic primer
<400> 1197
   cggtggtgca agtggaa 17
<210> 1198
   <211> 20
   <212> DNA
   <213> Artificial Sequence
<220>
   <223> Description of Artificial Sequence: Synthetic primer
<400> 1198
   gccttggtgt cctcatttct 20
<210> 1199
   <211> 24
   <212> DNA
   <213> Artificial Sequence
<220>
   <223> Description of Artificial Sequence: Synthetic probe
<400> 1199
   cttgaggttt catttccccc tccc 24
<210> 1200
   <211> 1304
   <212> PRT
   <213> Homo sapiens
<400> 1200
   Met Ala Lys Ile Ala Lys Thr His Glu Asp Ile Glu Ala Gln Ile Arg
   1               5                   10                  15
   Glu Ile Gln Gly Lys Lys Ala Ala Leu Asp Glu Ala Gln Gly Val Gly
               20                  25                  30
   Leu Asp Ser Thr Gly Tyr Tyr Asp Gln Glu Ile Tyr Gly Gly Ser Asp
           35                  40                  45
   Ser Arg Phe Ala Gly Tyr Val Thr Ser Ile Ala Ala Thr Glu Leu Glu
       50                  55                  60
   Asp Asp Asp Asp Asp Tyr Ser Ser Ser Thr Ser Leu Leu Gly Gln Lys
   65                  70                  75                  80
   Lys Pro Gly Tyr His Ala Pro Val Ala Leu Leu Asn Asp Ile Pro Gln
                   85                  90                  95
   Ser Thr Glu Gln Tyr Asp Pro Phe Ala Glu His Arg Pro Pro Lys Ile
               100                 105                 110
   Ala Asp Arg Glu Asp Glu Tyr Lys Lys His Arg Arg Thr Met Ile Ile
           115                 120                 125
   Ser Pro Glu Arg Leu Asp Pro Phe Ala Asp Gly Gly Lys Thr Pro Asp
       130                 135                 140
   Pro Lys Met Asn Ala Arg Thr Tyr Met Asp Val Met Arg Glu Gln His
   145                 150                 155                 160
   Leu Thr Lys Glu Glu Arg Glu Ile Arg Gln Gln Leu Ala Glu Lys Ala
                   165                 170                 175
   Lys Ala Gly Glu Leu Lys Val Val Asn Gly Ala Ala Ala Ser Gln Pro
               180                 185                 190
   Pro Ser Lys Arg Lys Arg Arg Trp Asp Gln Thr Ala Asp Gln Thr Pro
           195                 200                 205
   Gly Ala Thr Pro Lys Lys Leu Ser Ser Trp Asp Gln Ala Glu Thr Pro
       210                 215                 220
   Gly His Thr Pro Ser Leu Arg Trp Asp Glu Thr Pro Gly Arg Ala Lys
   225                 230                 235                 240
   Gly Ser Glu Thr Pro Gly Ala Thr Pro Gly Ser Lys Ile Trp Asp Pro
                   245                 250                 255
   Thr Pro Ser His Thr Pro Ala Gly Ala Ala Thr Pro Gly Arg Gly Asp
               260                 265                 270
   Thr Pro Gly His Ala Thr Pro Gly His Gly Gly Ala Thr Ser Ser Ala
           275                 280                 285
   Arg Lys Asn Arg Trp Asp Glu Thr Pro Lys Thr Glu Arg Asp Thr Pro
       290                 295                 300
   Gly His Gly Ser Gly Trp Ala Glu Thr Pro Arg Thr Asp Arg Gly Gly
   305                 310                 315                 320
   Asp Ser Ile Gly Glu Thr Pro Thr Pro Gly Ala Ser Lys Arg Lys Ser
                   325                 330                 335
   Arg Trp Asp Glu Thr Pro Ala Ser Gln Met Gly Gly Ser Thr Pro Val
               340                 345                 350
   Leu Thr Pro Gly Lys Thr Pro Ile Gly Thr Pro Ala Met Asn Met Ala
           355                 360                 365
   Thr Pro Thr Pro Gly His Ile Met Ser Met Thr Pro Glu Gln Leu Gln
       370                 375                 380
   Ala Trp Arg Trp Glu Arg Glu Ile Asp Glu Arg Asn Arg Pro Leu Ser
   385                 390                 395                 400
   Asp Glu Glu Leu Asp Ala Met Phe Pro Glu Gly Tyr Lys Val Leu Pro
                   405                 410                 415
   Pro Pro Ala Gly Tyr Val Pro Ile Arg Thr Pro Ala Arg Lys Leu Thr
               420                 425                 430
   Ala Thr Pro Thr Pro Leu Gly Gly Met Thr Gly Phe His Met Gln Thr
           435                 440                 445
   Glu Asp Arg Thr Met Lys Ser Val Asn Asp Gln Pro Ser Gly Asn Leu
       450                 455                 460
   Pro Phe Leu Lys Pro Asp Asp Ile Gln Tyr Phe Asp Lys Leu Leu Val
   465                 470                 475                 480
   Asp Val Asp Glu Ser Thr Leu Ser Pro Glu Glu Gln Lys Glu Arg Lys
                   485                 490                 495
   Ile Met Lys Leu Leu Leu Lys Ile Lys Asn Gly Thr Pro Pro Met Arg
               500                 505                 510
   Lys Ala Ala Leu Arg Gln Ile Thr Asp Lys Ala Arg Glu Phe Gly Ala
           515                 520                 525
   Gly Pro Leu Phe Asn Gln Ile Leu Pro Leu Leu Met Ser Pro Thr Leu
       530                 535                 540
   Glu Asp Gln Glu Arg His Leu Leu Val Lys Val Ile Asp Arg Ile Leu
   545                 550                 555                 560
   Tyr Lys Leu Asp Asp Leu Val Arg Pro Tyr Val His Lys Ile Leu Val
                   565                 570                 575
   Val Ile Glu Pro Leu Leu Ile Asp Glu Asp Tyr Tyr Ala Arg Val Glu
               580                 585                 590
   Gly Arg Glu Ile Ile Ser Asn Leu Ala Lys Ala Ala Gly Leu Ala Thr
           595                 600                 605
   Met Ile Ser Thr Met Arg Pro Asp Ile Asp Asn Met Asp Glu Tyr Val
       610                 615                 620
   Arg Asn Thr Thr Ala Arg Ala Phe Ala Val Val Ala Ser Ala Leu Gly
   625                 630                 635                 640
   Ile Pro Ser Leu Leu Pro Phe Leu Lys Ala Val Cys Lys Ser Lys Lys
                   645                 650                 655
   Ser Trp Gln Ala Arg His Thr Gly Ile Lys Ile Val Gln Gln Ile Ala
               660                 665                 670
   Ile Leu Met Gly Cys Ala Ile Leu Pro His Leu Arg Ser Leu Val Glu
           675                 680                 685
   Ile Ile Glu His Gly Leu Val Asp Glu Gln Gln Lys Val Arg Thr Ile
       690                 695                 700
   Ser Ala Leu Ala Ile Ala Ala Leu Ala Glu Ala Ala Thr Pro Tyr Gly
   705                 710                 715                 720
   Ile Glu Ser Phe Asp Ser Val Leu Lys Pro Leu Trp Lys Gly Ile Arg
                   725                 730                 735
   Gln His Arg Gly Lys Gly Leu Ala Ala Phe Leu Lys Ala Ile Gly Tyr
               740                 745                 750
   Leu Ile Pro Leu Met Asp Ala Glu Tyr Ala Asn Tyr Tyr Thr Arg Glu
           755                 760                 765
   Val Met Leu Ile Leu Ile Arg Glu Phe Gln Ser Pro Asp Glu Glu Met
       770                 775                 780
   Lys Lys Ile Val Leu Lys Val Val Lys Gln Cys Cys Gly Thr Asp Gly
   785                 790                 795                 800
   Val Glu Ala Asn Tyr Ile Lys Thr Glu Ile Leu Pro Pro Phe Phe Lys
                   805                 810                 815
   His Phe Trp Gln His Arg Met Ala Leu Asp Arg Arg Asn Tyr Arg Gln
               820                 825                 830
   Leu Val Asp Thr Thr Val Glu Leu Ala Asn Lys Val Gly Ala Ala Glu
           835                 840                 845
   Ile Ile Ser Arg Ile Val Asp Asp Leu Lys Asp Glu Ala Glu Gln Tyr
       850                 855                 860
   Arg Lys Met Val Met Glu Thr Ile Glu Lys Ile Met Gly Asn Leu Gly
   865                 870                 875                 880
   Ala Ala Asp Ile Asp His Lys Leu Glu Glu Gln Leu Ile Asp Gly Ile
                   885                 890                 895
   Leu Tyr Ala Phe Gln Glu Gln Thr Thr Glu Asp Ser Val Met Leu Asn
               900                 905                 910
   Gly Phe Gly Thr Val Val Asn Ala Leu Gly Lys Arg Val Lys Pro Tyr
           915                 920                 925
   Leu Pro Gln Ile Cys Gly Thr Val Leu Trp Arg Leu Asn Asn Lys Ser
       930                 935                 940
   Ala Lys Val Arg Gln Gln Ala Ala Asp Leu Ile Ser Arg Thr Ala Val
   945                 950                 955                 960
   Val Met Lys Thr Cys Gln Glu Glu Lys Leu Met Gly His Leu Gly Val
                   965                 970                 975
   Val Leu Tyr Glu Tyr Leu Gly Glu Glu Tyr Pro Glu Val Leu Gly Ser
               980                 985                 990
   Ile Leu Gly Ala Leu Lys Ala Ile  Val Asn Val Ile Gly  Met His Lys
   995 1000 1005
   Met Thr Pro Pro Ile Lys Asp Leu Leu Pro Arg Leu Thr Pro Ile
   1010 1015 1020
   Leu Lys Asn Arg His Glu Lys Val Gln Glu Asn Cys Ile Asp Leu
   1025 1030 1035
   Val Gly Arg Ile Ala Asp Arg Gly Ala Glu Tyr Val Ser Ala Arg
   1040 1045 1050
   Glu Trp Met Arg Ile Cys Phe Glu Leu Leu Glu Leu Leu Lys Ala
   1055 1060 1065
   His Lys Lys Ala Ile Arg Arg Ala Thr Val Asn Thr Phe Gly Tyr
   1070 1075 1080
   Ile Ala Lys Ala Ile Gly Pro His Asp Val Leu Ala Thr Leu Leu
   1085 1090 1095
   Asn Asn Leu Lys Val Gln Glu Arg Gln Asn Arg Val Cys Thr Thr
   1100 1105 1110
   Val Ala Ile Ala Ile Val Ala Glu Thr Cys Ser Pro Phe Thr Val
   1115 1120 1125
   Leu Pro Ala Leu Met Asn Glu Tyr Arg Val Pro Glu Leu Asn Val
   1130 1135 1140
   Gln Asn Gly Val Leu Lys Ser Leu Ser Phe Leu Phe Glu Tyr Ile
   1145 1150 1155
   Gly Glu Met Gly Lys Asp Tyr Ile Tyr Ala Val Thr Pro Leu Leu
   1160 1165 1170
   Glu Asp Ala Leu Met Asp Arg Asp Leu Val His Arg Gln Thr Ala
   1175 1180 1185
   Ser Ala Val Val Gln His Met Ser Leu Gly Val Tyr Gly Phe Gly
   1190 1195 1200
   Cys Glu Asp Ser Leu Asn His Leu Leu Asn Tyr Val Trp Pro Asn
   1205 1210 1215
   Val Phe Glu Thr Ser Pro His Val Ile Gln Ala Val Met Gly Ala
   1220 1225 1230
   Leu Glu  Gly Leu Arg Val Ala  Ile Gly Pro Cys Arg  Met Leu Gln
       1235                 1240                 1245
   Tyr Cys  Leu Gln Gly Leu Phe  His Pro Ala Arg Lys  Val Arg Asp
       1250                 1255                 1260
   Val Tyr  Trp Lys Ile Tyr Asn  Ser Ile Tyr Ile Gly  Ser Gln Asp
       1265                 1270                 1275
   Ala Leu  Ile Ala His Tyr Pro  Arg Ile Tyr Asn Asp  Asp Lys Asn
       1280                 1285                 1290
   Thr Tyr  Ile Arg Tyr Glu Leu  Asp Tyr Ile Leu
       1295                 1300